# EUROPEAN PATENT SPECIFICATION

(11) **EP 4 370 183 B1**
(45) Date of publication and mention of the grant of the patent: **02.09.2026**
(21) Application number: 22840868.8
(22) Date of filing: 14.07.2022
(51) Int. Cl.: A61M 16/06, A61M 16/08

(54) **FLOW CONNECTOR FOR A PRESSURIZED FACE MASK**
STRÖMUNGSVERBINDER FÜR EINE UNTER DRUCK STEHENDE GESICHTSMASKE
RACCORD D'ÉCOULEMENT POUR UN MASQUE FACIAL SOUS PRESSION

(30) Priority: 14.07.2021 AU 2021902143
(43) Date of publication of application: 22.05.2024
(73) Proprietor: ResMed Pty Ltd, Bella Vista, New South Wales 2153 (AU)
(72) Inventor: CHENGALVARAYAN, Kishore Markapuram, Bella Vista, New South Wales 2153 (AU); GUNEY, Memduh, Bella Vista, New South Wales 2153 (AU); EVES, Matthew, Bella Vista, New South Wales 2153 (AU); BATE, Andrew James, Bella Vista, New South Wales 2153 (AU); MCMANUS, Jeremy David, Bella Vista, New South Wales 2153 (AU)
(74) Representative: Vossius & Partner Patentanwälte Rechtsanwälte mbB
(86) International application number: PCT/AU2022/050739
(87) International publication number: WO 2023/283690

(56) References cited:
- WO-A1-2018/126295
- WO-A1-2020/079617
- WO-A1-2020/191463
- WO-A1-2021/072495
- US-A1- 2016 271 351
- US-A1- 2019 076 611
- US-B2- 8 636 007

## Description

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

### 1 CROSS-REFERENCE TO RELATED APPLICATIONS

This application claims priority to Australian Provisional Application No. 2021902143, filed July 14, 2021.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube or endotracheal tube. In some forms, the comfort and effectiveness of these therapies may be improved.

### 2.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004310; WO 2006/074513; WO 2010/135785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO 2004/073778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063328 and WO 2006/130903 (describing amongst other things aspects of the ResMed Limited MIRAGE LIBERTYTM full-face mask); International Patent Application WO 2009/052560 (describing amongst other things aspects of the ResMed Limited SWIFTTM FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 2.2.3.5 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034665; International Patent Application Publication No. WO 2000/078381; US Patent No. 6,581,594; US Patent Application Publication No. US 2009/0050156; US Patent Application Publication No. 2009/0044808.

**Table of noise of prior masks (ISO 17510-2:2007, 10 cmH₂O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed MirageTM (*) | nasal | 29.5 | 21.5 | 1998 |
| ResMed UltraMirageT M | nasal | 36 (3) | 28 (3) | 2000 |
| ResMed Mirage ActivaTM | nasal | 32 (3) | 24 (3) | 2002 |
| ResMed Mirage MicroTM | nasal | 30 (3) | 22 (3) | 2008 |
| ResMed MirageTM SoftGel | nasal | 29 (3) | 22 (3) | 2008 |
| ResMed MirageTM FX | nasal | 26 (3) | 18 (3) | 2010 |
| ResMed Mirage SwiftTM (*) | nasal pillows | 37 | 29 | 2004 |
| ResMed Mirage SwiftTM II | nasal pillows | 28 (3) | 20 (3) | 2005 |
| ResMed Mirage SwiftTM LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH2O) | | | | |

Sound pressure values of a variety of objects are listed below

| Object | A-weighted sound pressure dB(A) | Notes |
|---|---|---|
| Vacuum cleaner: Nilfisk Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

WO 2020/191463 A1 relates to a patient interface which includes a plenum chamber and a positioning and stabilising structure. The plenum chamber may include a seal-forming structure and a fascia portion. At least a medial portion of the fascia portion is flexible. The patient interface may include a rigidiser to control flexing of the fascia portion.

WO 2021/072495 A1 relates to a patient interface including a seal-forming structure with a textile membrane that has at least one hole such that the flow of air at a therapeutic pressure is delivered to at least an entrance to the patient's nares and/or an entrance to the patient's mouth. The seal-forming structure is constructed and arranged to maintain the therapeutic pressure in a cavity of a plenum chamber throughout the patient's respiratory cycle, in use. The textile membrane includes a first portion that is held in a relaxed state and a second portion that is held in a taut state. The taut state of the second portion is configured to allow the seal-forming structure to include a three-dimensional shape that has multiple curvatures.

WO 2020/079617 A1 relates to a patient interface which comprises a support structure and a seal-forming structure. The support structure is arranged to support the sealing portion and is configured to connect to the frame. The sealing portion comprises textile and is attached to the support structure along an outer perimeter of the sealing portion such that in use the sealing portion may be in tension due to reactive stress of the support structure and/or a resilient stretch characteristic of the textile such that the sealing portion exerts a force against the patient's face.

### 3 BRIEF SUMMARY OF THE TECHNOLOGY

The invention is defined in the appended claims. Aspects, embodiments and examples disclosed herein which do not fall within the scope of the appended claims do not form part of the invention and are merely provided for illustrative purposes.

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a positioning and stabilizing structure for providing a force to hold a seal-forming structure in a therapeutically effective position on the patient's head, the positioning and stabilizing structure comprising a frame configured to connect to a plenum chamber.

One form of the present technology comprises a patient interface comprising:
a plenum chamber pressurisable to a therapeutic pressure above ambient air pressure; and
a seal-forming structure constructed and arranged to form a seal with a region of the patient's face surrounding an entrance to the patient's airways, the seal-forming structure constructed and arranged to maintain said therapeutic pressure in the plenum chamber throughout the patient's respiratory cycle in use.

One form of the present technology comprises a patient interface comprising:
a plenum chamber pressurisable to a therapeutic pressure above ambient air pressure;
a seal-forming structure constructed and arranged to form a seal with a region of the patient's face surrounding an entrance to the patient's airways, the seal-forming structure constructed and arranged to maintain said therapeutic pressure in the plenum chamber throughout the patient's respiratory cycle in use; and
a positioning and stabilising structure configured to provide a force to hold the seal-forming structure in a therapeutically effective position on the patient's head.

One form of the present technology comprises a patient interface comprising:
a plenum chamber pressurisable to a therapeutic pressure above ambient air pressure;
an oral seal-forming structure constructed and arranged to form a seal with a region of the patient's face at least partially surrounding an entrance to the patient's mouth such that the flow of air at said therapeutic pressure is delivered to the patient's mouth, the oral seal-forming structure constructed and arranged to maintain said therapeutic pressure in the plenum chamber throughout the patient's respiratory cycle in use;
a nasal seal-forming structure constructed and arranged to form a seal with a region of the patient's face surrounding an entrance to the patient's nose such that the flow of air at said therapeutic pressure is delivered to the patient's nose, the nasal seal-forming structure constructed and arranged to maintain said therapeutic pressure in the plenum chamber throughout the patient's respiratory cycle in use; and
a positioning and stabilising structure configured to provide a force to hold the seal-forming structure in a therapeutically effective position on the patient's head.

One form of the present technology comprises a positioning and stabilizing structure comprising a frame and conduit headgear connected to the frame.

Another aspect of one form of the present technology is a positioning and stabilizing structure comprising a frame with a central portion and a pair of arms connected to the central portion; the frame also comprising conduit headgear connected to the pair of arms of the frame.

Another aspect of one form of the present technology is a positioning and stabilizing structure comprising a frame and conduit headgear, the frame including a central portion having a central portion with a cavity and a pair of arms connected to the central portion and including a flow path into the cavity, the conduit headgear connected to the pair of arms and configured to convey pressurized airflow to the pair of arms.

Another aspect of one form of the present technology is a positioning and stabilizing structure comprising a frame and conduit headgear, the frame includes a central portion, a left annular portion coupled to the central portion, and a right annular portion coupled to the central portion, the conduit headgear configured to connect to a plenum chamber through the left annular portion and through the right annular portion.

One form of the present technology comprises a patient interface comprising:
a positioning and stabilizing structure comprising,
a frame configured to connect to a plenum chamber, the frame including,
   a central portion having a cavity,
   a pair of arms connected to the central portion, each arm of the pair of arms including a flow path; and
conduit headgear connected to each arm configured to convey a flow of pressurized air to each arm.

One form of the present technology comprises a patient interface comprising:
a plenum chamber pressurisable to a therapeutic pressure of at least 6 cmH₂O above ambient air pressure, said plenum chamber including an inlet port sized and structured to receive a flow of air at the therapeutic pressure for breathing by a patient,
a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use;
a vent structure to allow a continuous flow of gases exhaled by the patient from an interior of the plenum chamber to vent to ambient, said vent structure being sized and shaped to maintain the therapeutic pressure in the plenum chamber in use; and
a positioning and stabilising structure configured to provide a force to hold the seal-forming structure in a therapeutically effective position on the patient's head, the positioning and stabilising structure comprising:
   a frame coupled to the plenum chamber, the frame including,
   a central portion having a cavity connected to the inlet port,
   a pair of arms connected to the central portion, each arm of the pair of arms including a flow path configured to convey the flow of pressurized air toward the cavity; and
conduit headgear connected to each arm and configured to provide at least a portion of the force, the conduit headgear configured to convey the flow of pressurized air to each arm, and the conduit headgear being constructed and arranged so that at least a portion overlies a region of the patient's head superior to an otobasion superior of the patient's head in use.

In some examples: a) the frame is constructed from a rigid material; b) the pair of arms are integrally formed with the central portion; c) the pair of arms extend laterally from the central portion; d) the pair of arms extend posterior to a front surface of the central portion, in use; e) the seal-forming structure is a nasal seal-forming structure, the patient interface further comprises a oral seal-forming structure constructed and arranged to form a seal with a region of the patient's face at least partially surrounding an entrance to the patient's mouth such that the flow of air at said therapeutic pressure is delivered to the mouth, the oral seal-forming structure constructed and arranged to maintain said therapeutic pressure in the plenum chamber throughout the patient's respiratory cycle in use; f) the nasal seal-forming structure comprises a nasal bridge region; g) nasal bridge region is constructed to have a nasal bridge saddle-shaped region; h) a connection port is connected to each arm of the pair of arms; and/or i) the conduit headgear is directly connected to the connection ports.

In some examples: a) the plenum chamber includes a groove; b) the central portion is positioned within the groove; c) the plenum chamber is removably positionable within the groove; d) the central portion of the frame includes the vent; e) headgear straps are connected to the plenum chamber and configured to provide a portion of the force; f) the headgear straps are connected directly to the frame; g) the headgear straps are connected directly to the plenum chamber; and/or h) the headgear straps are constructed from a breathable material to allow moisture vapor to escape and/or be transmitted therethrough.

One form of the present technology comprises a patient interface comprising:
a plenum chamber including a cavity that is pressurisable to a therapeutic pressure of at least 6 cmH₂O above ambient air pressure, said plenum chamber including a plenum chamber inlet port sized and structured to receive a flow of air at the therapeutic pressure for breathing by a patient;
an oral seal-forming structure constructed and arranged to form a seal with a region of the patient's face at least partially surrounding an entrance to the patient's mouth such that the flow of air at said therapeutic pressure is delivered to the patient's mouth, the oral seal-forming structure constructed and arranged to maintain said therapeutic pressure in the plenum chamber throughout the patient's respiratory cycle in use;
a nasal seal-forming structure constructed and arranged to form a seal with a region of the patient's face surrounding an entrance to the patient's nose such that the flow of air at said therapeutic pressure is delivered to the patient's nose, the nasal seal-forming structure constructed and arranged to maintain said therapeutic pressure in the plenum chamber throughout the patient's respiratory cycle in use;
a vent structure to allow a continuous flow of gases exhaled by the patient from an interior of the plenum chamber to vent to ambient, said vent structure being sized and shaped to maintain the therapeutic pressure in the plenum chamber in use; and
a positioning and stabilizing structure configured to maintain the nasal seal-forming structure in a therapeutically effective position, the positioning and stabilizing structure comprising:
   a frame coupled to the plenum chamber, the frame including,
   a central portion having a hollow interior space in fluid communication with the plenum chamber inlet port,
   a pair of arms connected to the central portion, each arm of the pair of arms including a flow path into the hollow interior space; and
conduit headgear connected to each arm and configured to provide at least a portion of the force, the conduit headgear configured to convey the flow of pressurized air into the hollow interior space through each arm, and the conduit headgear being constructed and arranged so that at least a portion overlies a region of the patient's head superior to an otobasion superior of the patient's head in use.

In some examples: a) a wall extends from the central portion; b) the wall forms an opening into the hollow interior portion; and/or c) the wall is configured to be positioned within the plenum chamber inlet port in order to convey the flow of pressurized air from the hollow interior space to the plenum chamber.

In some examples: a) the central portion includes an aperture that provides an opening into the hollow interior space; b) the aperture is configured to face in an anterior direction in use; c) the aperture has a substantially elliptical shape; d) the central portion includes a posterior lip that extends radially inward from an outer perimeter of the aperture; and/or e) the posterior lip is recessed from an anterior surface of the frame.

In some examples: a) a vent module is positioned within an aperture of the central portion; b) the vent module includes a vent body with vent holes; c) the vent module includes a vent arm extending from the vent body; d) the vent module includes an opening formed between the vent body and the vent arm; e) the opening is configured to allow the flow of pressurized air to enter the hollow interior space; f) the vent arm is disposed within the hollow interior space; g) the vent body includes a recessed surface with the vent holes; h) the recessed surface is recessed in a posterior direction, in use; i) a dampening member is positioned proximate to the vent holes on an anterior side of the recessed surface; j) the dampening member is configured to dampen noise from the vent holes; k) vent body includes an anterior lip; 1) vent body includes a posterior lip; m) vent body includes a groove disposed between the anterior lip and the posterior lip; n) the posterior lip is configured to contact the groove; and/or o) the vent module is removably coupled to the frame.

In some examples: a) the pair of arms are integrally formed with the central portion; b) the pair of arms extend laterally from the central portion; and/or c) wherein the pair of arms extend posterior to a front surface of the central portion, in use.

In some examples: a) each arm includes a vent opening; b) each vent opening is configured to face in the anterior direction, in use; c) a connection port is connected to each arm; d) each connection port includes an emergency vent aligned with the respective vent opening; e) each connection port further comprises an anti-asphyxia valve configured to selectively seal against the emergency vent and limit airflow through the vent opening; f) the anti-asphyxia valves are movable between a relaxed position where airflow in permitted to flow through the vent openings, and a closed position where airflow is limited from flowing through the vent openings; g) the anti-asphyxia valves are configured to move into the closed position because of the flow of air; and/or h) the conduit headgear is directly connected to each connection port.

In some examples: a) the frame further includes a pair of wings integrally formed with the central portion; b) the pair of wings extends laterally from the central portion; c) the pair of wings are inferior to a pair of arms of the frame, in use; d) an engagement mechanism is coupled to each wing of the pair of wings; e) the engagement mechanisms are magnets; f) headgear straps are removably connected to the engagement mechanisms and configured to provide a portion of the force; and/or g) the headgear straps are constructed from a breathable material to allow moisture vapor to escape and/or be transmitted therethrough.

In some examples: a) the plenum chamber includes a groove with a shape configured to receive a shape of the frame; b) the frame is constructed from a rigid material; and/or c) the plenum chamber is constructed from a flexible or semi-rigid material.

One form of the present technology comprises a patient interface comprising:
a plenum chamber including a cavity that is pressurisable to a therapeutic pressure of at least 6 cmH₂O above ambient air pressure, said plenum chamber sized and structured to receive a flow of air at the therapeutic pressure for breathing by a patient;
a nasal seal-forming structure constructed and arranged to form a seal with a region of the patient's face surrounding an entrance to the patient's nose such that the flow of air at said therapeutic pressure is delivered to the patient's nose, the nasal seal-forming structure constructed and arranged to maintain said therapeutic pressure in the plenum chamber throughout the patient's respiratory cycle in use;
a vent structure to allow a continuous flow of gases exhaled by the patient from an interior of the plenum chamber to vent to ambient, said vent structure being sized and shaped to maintain the therapeutic pressure in the plenum chamber in use; and
a positioning and stabilizing structure configured to maintain the nasal seal-forming structure in a therapeutically effective position, the positioning and stabilizing structure comprising:
   a frame coupled to the plenum chamber, the frame including,
      a central portion having a cavity, the central portion being connected to the plenum chamber to form a pressurized volume,
      a pair of arms connected to the central portion, each arm of the pair of arms including a flow path into the pressurized volume; and
   conduit headgear connected to each arm and configured to provide at least a portion of the force, the conduit headgear configured to convey the flow of pressurized air into the pressurized volume through each arm, and the conduit headgear being constructed and arranged so that at least a portion overlies a region of the patient's head superior to an otobasion superior of the patient's head in use.

In some examples: a) the pair of arms are integrally formed with the central portion; b) the pair of arms extends laterally from the central portion; c) the pair of arms are posterior to a front surface of the central portion, in use; d) each arm of the pair of arms extends at least partially into the cavity; e) the frame is constructed from a rigid material; and/or f) the plenum chamber is constructed from a flexible or semi-rigid material.

In some examples: a) a sleeve at least partially covers the frame; b) the sleeve and the plenum chamber are constructed from the same material; c) the sleeve includes a pair of connection ports configured to directly connect to the conduit headgear; d) each connection port of the pair of connection ports is aligned with one arm of the pair of arms; e) the central portion includes the vent; and/or f) the sleeve includes a vent opening configured to allow for substantially unobstructed airflow through the vent.

In some examples: a) the central portion includes a dividing wall disposed within the cavity; b) the dividing wall at least partially separates the pair of arms from the vent; and/or c) the vent is disposed on the central portion inferior to the dividing wall.

In some examples: a) the central portion includes a deflection wall disposed between the pair of arms; b) the central portion includes a substantially triangular shape; and/or c) the deflection wall is configured to deflect the flow of air exiting the pair of arms toward a posterior direction, in use.

In some examples the nasal seal-forming structure does not cover the patient's mouth.

In some examples: a) an oral seal-forming structure is constructed and arranged to form a seal with a region of the patient's face at least partially surrounding an entrance to the patient's mouth such that the flow of air at said therapeutic pressure is delivered to the patient's mouth, the oral seal-forming structure is constructed and arranged to maintain said therapeutic pressure in the plenum chamber throughout the patient's respiratory cycle in use; b) headgear straps are removably connected to the plenum chamber and configured to provide a portion of the force; c) the headgear straps are constructed from a breathable material to allow moisture vapor to escape and/or be transmitted therethrough; d) the central portion includes a vent opening and an anti-asphyxia valve configured to selectively seal against the vent opening and limit airflow through the vent opening; e) the anti-asphyxia valve is movable between a relaxed position where airflow in permitted to flow through the vent opening, and a closed position where airflow is limited from flowing through the vent opening; f) the anti-asphyxia valves are configured to move into the closed position because of the flow of air; g) the vent opening includes at least one rib; and/or h) the anti-asphyxia valve is configured to contact the at least one rib in the closed position.

One form of the present technology comprises a patient interface comprising:
a plenum chamber including a cavity that is pressurisable to a therapeutic pressure of at least 6 cmH₂O above ambient air pressure, said plenum chamber including a left plenum chamber inlet port sized and structured to receive a flow of air at the therapeutic pressure for breathing by a patient, and a right plenum chamber inlet port sized and structured to receive a flow of air at the therapeutic pressure for breathing by a patient;
an oral seal-forming structure constructed and arranged to form a seal with a region of the patient's face at least partially surrounding an entrance to the patient's mouth such that the flow of air at said therapeutic pressure is delivered to the patient's mouth, the oral seal-forming structure constructed and arranged to maintain said therapeutic pressure in the plenum chamber throughout the patient's respiratory cycle in use;
a nasal seal-forming structure constructed and arranged to form a seal with a region of the patient's face surrounding an entrance to the patient's nose such that the flow of air at said therapeutic pressure is delivered to the patient's nose, the nasal seal-forming structure constructed and arranged to maintain said therapeutic pressure in the plenum chamber throughout the patient's respiratory cycle in use;
a vent structure to allow a continuous flow of gases exhaled by the patient from an interior of the plenum chamber to vent to ambient, said vent structure being sized and shaped to maintain the therapeutic pressure in the plenum chamber in use; and
a positioning and stabilizing structure configured to maintain the nasal seal-forming structure and the oral seal-forming structure in a therapeutically effective position, the positioning and stabilizing structure comprising:
   a frame coupled to the plenum chamber, the frame including,
      a central portion coupled to the plenum chamber outside of the cavity,
      a left annular portion coupled to the central portion and configured to be aligned with the left plenum chamber inlet port,
      a right annular portion coupled to the central portion and configured to be aligned with the right plenum chamber inlet port; and
   conduit headgear connected to the left plenum chamber inlet port and to the right plenum chamber inlet port, the conduit headgear configured to provide at least a portion of the force, the conduit headgear configured to convey the flow of pressurized air into the cavity through the left plenum chamber inlet port and through the right plenum chamber inlet port, and the conduit headgear being constructed and arranged so that at least a portion overlies a region of the patient's head superior to an otobasion superior of the patient's head in use.

In some examples: a) the frame includes an opening radially within the central portion; b) the opening is substantially triangular in shape; c) the central portion is formed as an integral piece with the left annular portion and with the right annular portion; d) the frame is constructed from a more rigid material than the plenum chamber; e) the plenum chamber includes a groove configured to receive the frame; and/or f) the left plenum chamber inlet port and the right plenum chamber inlet port protrude in an anterior direction.

In some examples: a) a left conduit connection structure includes a connection port configured to directly connect to the conduit headgear; b) a right conduit connection structure including a connection port configured to directly connect to the conduit headgear; c) each of the left conduit connection structure and the right conduit connection structure include an anti-asphyxia valve configured to selectively seal against an emergency vent and limit airflow through the emergency vent; d) each anti-asphyxia valve is movable between a relaxed position where airflow in permitted to flow through the emergency vent, and a closed position where airflow is limited from flowing through the emergency vent; e) each anti-asphyxia valve is configured to move into the closed position because of the flow of air; f) each emergency vent includes at least one rib; g) each anti-asphyxia valve is configured to contact the at least one rib in the closed position; h) the left conduit connection structure and the right conduit connection structure each include a wall inclined with respect to the connection port; i) each wall is inclined 45°; j) the left conduit connection structure and the right conduit connection structure are integrally formed with the frame; k) the left conduit connection structure and the right conduit connection structure are removably connected to the frame; 1) the left conduit connection structure and the right conduit connection structure each include vent holes of the vent; and/or m) the left conduit connection structure and the right conduit connection structure each include a dividing wall at least partially separating the connection port from the vent holes.

In some examples: a) an engagement mechanism is coupled to the central portion of the frame; b) the engagement mechanism is a magnet; c) the positioning and stabilizing structure further comprises headgear straps removably connected to the engagement mechanism and configured to provide a portion of the force; and/or d) the headgear straps are constructed from a breathable material to allow moisture vapor to escape and/or be transmitted therethrough.

One form of the present technology comprises a patient interface comprising:
a plenum chamber including a cavity that is pressurisable to a therapeutic pressure of at least 6 cmH₂O above ambient air pressure, said plenum chamber including a left plenum chamber inlet port sized and structured to receive a flow of air at the therapeutic pressure for breathing by a patient, and a right plenum chamber inlet port sized and structured to receive a flow of air at the therapeutic pressure for breathing by a patient, the plenum chamber constructed from a flexible material;
a seal-forming structure constructed and arranged to form a seal with a region of the patient's face surrounding an entrance to the patient's airways such that the flow of air at said therapeutic pressure is delivered to the patient's airways, the seal-forming structure constructed and arranged to maintain said therapeutic pressure in the plenum chamber throughout the patient's respiratory cycle in use;
a vent structure connected to a vent opening in the plenum chamber, the vent structure configured to allow a continuous flow of gases exhaled by the patient from an interior of the plenum chamber to vent to ambient, said vent structure being sized and shaped to maintain the therapeutic pressure in the plenum chamber in use, the vent structure comprising:
   a vent body formed from a rigid material and directly positioned within the vent opening, the vent body configured to direct bending movement in the plenum chamber away from central axis that intersects the vent opening, the vent body comprising a surface having a plurality of vent holes configured to allow air to exit the plenum chamber and at least one connecting feature, the at least one connecting feature being spaced apart from an outermost edge of the surface
   a dampening member positioned in the vent body and at least partially projecting over the plurality of vent holes, the dampening member configured to decrease a noise output of air through the vent holes, and
   a cover including at least one complementary connecting feature configured to engage the at least connecting feature of the vent body, the cover having an outer diameter less than an inner diameter of the vent body, the at least one complementary connection feature being radially inside of and spaced apart from the outer diameter of the cover,
   wherein a circumferential gap is formed between the outer diameter of the cover and the inner diameter of the vent body, the circumferential gap forming a first pathway that is configured to allow air to exit the vent structure after passing through the plurality of vent holes, and
   wherein the at least one connecting feature is configured to engage the at least one complementary connecting feature so that neither are visible in use; and
a positioning and stabilizing structure configured to maintain the nasal seal-forming structure and the oral seal-forming structure in a therapeutically effective position

One form of the present technology comprises a patient interface comprising:
a plenum chamber including a cavity that is pressurisable to a therapeutic pressure of at least 6 cmH₂O above ambient air pressure, said plenum chamber including a left plenum chamber inlet port sized and structured to receive a flow of air at the therapeutic pressure for breathing by a patient, and a right plenum chamber inlet port sized and structured to receive a flow of air at the therapeutic pressure for breathing by a patient, the plenum chamber constructed from a flexible material;
an oral seal-forming structure constructed and arranged to form a seal with a region of the patient's face at least partially surrounding an entrance to the patient's mouth such that the flow of air at said therapeutic pressure is delivered to the patient's mouth, the oral seal-forming structure constructed and arranged to maintain said therapeutic pressure in the plenum chamber throughout the patient's respiratory cycle in use;
a nasal seal-forming structure constructed and arranged to form a seal with a region of the patient's face surrounding an entrance to the patient's nose such that the flow of air at said therapeutic pressure is delivered to the patient's nose, the nasal seal-forming structure constructed and arranged to maintain said therapeutic pressure in the plenum chamber throughout the patient's respiratory cycle in use;
a vent structure connected to a vent opening in the plenum chamber, the vent structure configured to allow a continuous flow of gases exhaled by the patient from an interior of the plenum chamber to vent to ambient, said vent structure being sized and shaped to maintain the therapeutic pressure in the plenum chamber in use, the vent structure comprising:
   a vent body formed from a rigid material and directly positioned within the vent opening, the vent body configured to direct bending movement in the plenum chamber away from central axis that intersects the vent opening, the vent body comprising a surface having a plurality of vent holes configured to allow air to exit the plenum chamber and at least one connecting feature,
   a dampening member positioned in the vent body and at least partially projecting over the plurality of vent holes, the dampening member configured to decrease a noise output of air through the vent holes, and
   a cover including at least one complementary connecting feature configured to engage the at least connecting feature of the vent body, the cover having an outer diameter less than an inner diameter of the vent body,
   wherein a circumferential gap is formed between the outer diameter of the cover and the inner diameter of the vent body, the circumferential gap forming a first pathway that is configured to allow air to exit the vent structure after passing through the plurality of vent holes; and
a positioning and stabilizing structure configured to maintain the nasal seal-forming structure and the oral seal-forming structure in a therapeutically effective position.

In some forms: a) the cover is permanently connected to the vent body; b) the at least one connecting feature includes a finger forming an overhang; c) the at least one connecting feature is inclined relative to the surface; d) the at least one connecting feature is a plurality of connecting features arranged in a circular orientation; e) the at least one complementary connecting feature includes a lip; f) the at least one complementary connecting feature is a plurality of complementary connecting features arranged in a circular orientation; g) the at least one connecting feature engages the at least one complementary connecting feature with a snap-fit; h) the cover is circular in shape and includes a solid surface configured to prevent air from exiting the vent through a center of the cover; and/or i) the cover is ring-shaped and includes an open center forming a second pathway that is configured to allow air to exit the vent structure after passing through the plurality of vent holes.

In some forms: a) the plurality of vent holes are arranged in clusters; and/or b) the clusters include four, five, or six vent holes.

In some forms: a) the surface includes at least one rib, the dampening member contacting the at least one rib and being spaced apart from the vent holes; b) the at least one rib is integrally formed with the at least one connecting feature; c) the vent holes have a substantially trapezoidal shape; d) the left plenum chamber inlet port and the right plenum chamber inlet port are each formed from a rigid clip; e) the rigid clips are each removably positionable within a groove on the plenum chamber; f) the rigid clips each include a connection member spaced apart from the respective plenum chamber inlet port; g) the connection member is a magnet; and/or f) wherein the plenum chamber further includes a fold line intersecting the center line and spaced apart from the vent structure and from the rigid clips.

In some forms: a) a sound power output by the vent structure is about 1 dBA to about 50 dBA; b) the sound power output by the vent structure is about 5 dBA to about 25 dBA; c) the sound power output by the vent structure is about 19.3 dBA; d) a sound pressure output by the vent structure is about 1 dBA to about 50 dBA; e) the sound pressure output by the vent structure is about 5 dBA to about 25 dBA; and/or f) the sound pressure output by the vent structure is about 12.7 dBA.

One form of the present technology comprises a vent structure configured to connect to a vent opening in the plenum chamber, the vent structure configured to allow a continuous flow of gases exhaled by the patient from an interior of the plenum chamber to vent to ambient, said vent structure being sized and shaped to maintain the therapeutic pressure in the plenum chamber in use, the vent structure comprising:
a vent body formed from a rigid material and directly positioned within the vent opening, the vent body configured to direct bending movement in the plenum chamber away from central axis that intersects the vent opening, the vent body comprising a surface having a plurality of vent holes configured to allow air to exit the plenum chamber and at least one connecting feature,
a dampening member positioned in the vent body and at least partially projecting over the plurality of vent holes, the dampening member configured to decrease a noise output of air through the vent holes, and
a cover including at least one complementary connecting feature configured to engage the at least connecting feature of the vent body, the cover having an outer diameter less than an inner diameter of the vent body,
wherein a circumferential gap is formed between the outer diameter of the cover and the inner diameter of the vent body, the circumferential gap forming a first pathway that is configured to allow air to exit the vent structure after passing through the plurality of vent holes.

In some forms, the vent structure is configured to be used with the plenum chamber of a ultra-compact full-face mask, a full-face mask, a nasal mask (e.g., including an under the nose seal, nasal pillows, etc.), or any similar type of mask.

One form of the present technology comprises a patient interface comprising:
a plenum chamber including a cavity that is pressurisable to a therapeutic pressure of at least 6 cmH₂O above ambient air pressure, said plenum chamber including a left plenum chamber inlet port sized and structured to receive a flow of air at the therapeutic pressure for breathing by a patient, and a right plenum chamber inlet port sized and structured to receive a flow of air at the therapeutic pressure for breathing by a patient, the plenum chamber being constructed from a flexible material;
a seal-forming structure constructed and arranged to form a seal with a region of the patient's face surrounding an entrance to the patient's airways such that the flow of air at said therapeutic pressure is delivered to the patient's airways, the seal-forming structure constructed and arranged to maintain said therapeutic pressure in the plenum chamber throughout the patient's respiratory cycle in use;
a vent structure connected to a vent opening in the plenum chamber, the vent structure configured to allow a continuous flow of gases exhaled by the patient from an interior of the plenum chamber to vent to ambient, said vent structure being sized and shaped to maintain the therapeutic pressure in the plenum chamber in use, the vent structure comprising:
   a vent body formed from a rigid material and directly positioned within the vent opening, the vent body configured to direct bending movement in the plenum chamber away from central axis that intersects the vent opening, the vent body comprising a surface having a plurality of vent holes configured to allow air to exit the plenum chamber,
   a cover being permanently connected to the vent body and including a plurality of openings, each opening being in communication with one of the respective vent holes in order to provide a plurality of flow paths for air to exit the plenum chamber, and
wherein an inner surface of the cover configured to face the plenum chamber and be pressurised to the therapeutic pressure in use; and
a positioning and stabilizing structure configured to maintain the seal-forming structure in a therapeutically effective position.

In some forms, a) the plurality of openings on the cover are oriented radially outward; b) first axes through a center of each opening of the plurality of openings is including relative to second axes through a center of each vent hole of a plurality of vent holes; c) the first axes and the second axes are oriented approximately 45° with respect to one another; and/or d) the first axes and the second axes are oriented approximately 90° with respect to one another.

In some forms, a) the plurality of vent holes and/or the plurality of openings is a U-shape; b) the cover projects away from the remainder of the vent body in a direction away from the plenum chamber; c) the plurality of openings on the cover are oriented in the substantially same direction as the plurality of vent holes; and/or d) the diameter of the plurality of vent holes is greater than the diameter of the plurality of openings.

In some forms, a) the plurality of vent holes are arranged in clusters, wherein each cluster is arranged in a given pattern and is spaced apart from adjacent clusters; b) the clusters include four, five, or six vent holes; c) vent body includes an anterior surface and a posterior surface opposite the anterior surface and positioned within the plenum chamber in use; d) the plurality of vent holes are recessed relative to the posterior surface in the direction of the anterior surface; e) the cover projects beyond the anterior surface in a direction away from the posterior surface; f) the plurality of openings are disposed between the anterior surface and an outer surface of the cover; g) a groove is disposed between the anterior surface and the posterior surface; and/or h) the groove is configured to receive a portion of the plenum chamber forming the outer perimeter of the vent opening.

One form of the present technology comprises a patient interface comprising:
a plenum chamber including a cavity that is pressurisable to a therapeutic pressure of at least 6 cmH₂O above ambient air pressure, said plenum chamber including a left plenum chamber inlet port sized and structured to receive a flow of air at the therapeutic pressure for breathing by a patient, and a right plenum chamber inlet port sized and structured to receive a flow of air at the therapeutic pressure for breathing by a patient, the plenum chamber being constructed from a flexible material, the plenum chamber further including a left groove at least partially surrounding the left plenum chamber inlet port and a right groove at least partially surrounding the right plenum chamber inlet port;
a seal-forming structure constructed and arranged to form a seal with a region of the patient's face surrounding an entrance to the patient's airways such that the flow of air at said therapeutic pressure is delivered to the patient's airways, the seal-forming structure constructed and arranged to maintain said therapeutic pressure in the plenum chamber throughout the patient's respiratory cycle in use;
a vent structure connected to a vent opening in the plenum chamber, the vent structure configured to allow a continuous flow of gases exhaled by the patient from an interior of the plenum chamber to vent to ambient, said vent structure being sized and shaped to maintain the therapeutic pressure in the plenum chamber in use, the vent structure comprising:
   a vent body formed from a rigid material and directly positioned within the vent opening, the vent body configured to direct bending movement in the plenum chamber away from central axis that intersects the vent opening, the vent body comprising a surface having a plurality of vent holes configured to allow air to exit the plenum chamber; and
   a positioning and stabilizing structure configured to maintain the seal-forming structure in a therapeutically effective position, the positioning and stabilizing structure comprising:
      a first left clip received in the left plenum chamber inlet port,
      a second left clip received in the left groove,
      a first right left clip received in the right plenum chamber inlet port, and
      a second right clip received in the right groove.

In some forms, a) the first left clip and the second left clip are connected to the positioning and stabilizing structure independently of one another; b) the first right clip and the second right clip are connected to the plenum chamber independently of one another; and/or c) the second left clip and the second right clip are permanently connected to the plenum chamber.

In some forms, a) the first left clip and the second left clip are permanently connected to one another; b) the first right clip and the second right clip are permanently connected to one another; c) the second left clip and the second right clip each include a connection member; and/or d) the connection member is a magnet.

In some forms, a) the positioning and stabilizing structure includes a conduit headgear configured to convey the flow of air to the plenum chamber; b) the first left clip and the first right clip are connected to the conduit headgear; c) the positioning and stabilizing structure further comprises at least one strap; and/or d) the conduit headgear includes a tab configured to removably receive the at least one strap.

In some forms, a) the plurality of openings on the cover are oriented radially outward; b) first axes through a center of each opening of the plurality of openings is including relative to second axes through a center of each vent hole of a plurality of vent holes; c) the first axes and the second axes are oriented approximately 45° with respect to one another; and/or d) the first axes and the second axes are oriented approximately 90° with respect to one another.

In some forms, a) the plurality of vent holes and/or the plurality of openings is a U-shape; b) the cover projects away from the remainder of the vent body in a direction away from the plenum chamber; c) the plurality of openings on the cover are oriented in the substantially same direction as the plurality of vent holes; and/or d) the diameter of the plurality of vent holes is greater than the diameter of the plurality of openings.

In some forms, a) the plurality of vent holes are arranged in clusters, wherein each cluster is arranged in a given pattern and is spaced apart from adjacent clusters; b) the clusters include four, five, or six vent holes; c) vent body includes an anterior surface and a posterior surface opposite the anterior surface and positioned within the plenum chamber in use; d) the plurality of vent holes are recessed relative to the posterior surface in the direction of the anterior surface; e) the cover projects beyond the anterior surface in a direction away from the posterior surface; f) the plurality of openings are disposed between the anterior surface and an outer surface of the cover; g) a groove is disposed between the anterior surface and the posterior surface; and/or h) the groove is configured to receive a portion of the plenum chamber forming the outer perimeter of the vent opening.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.
Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

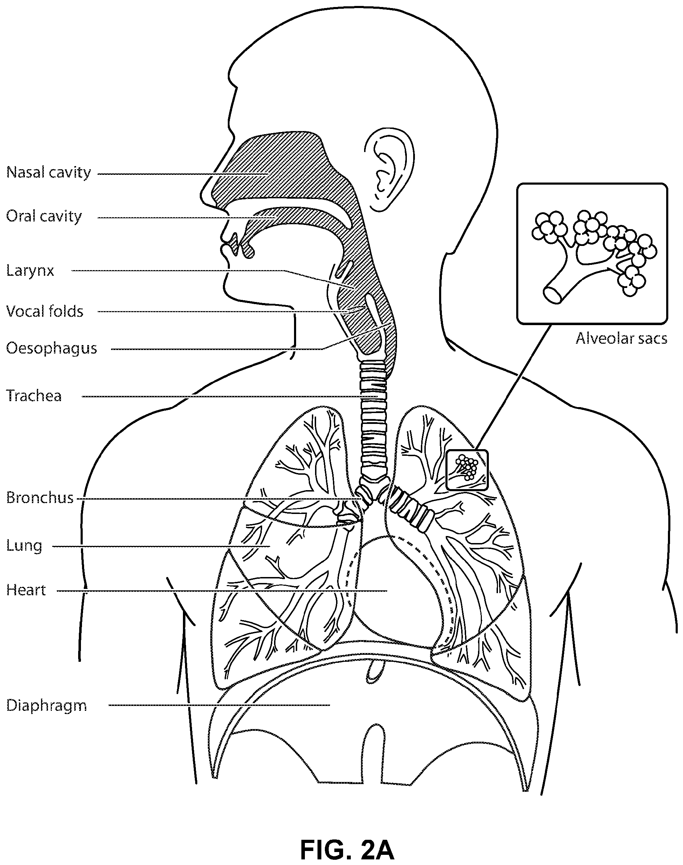

Fig. 2A shows an overview of a human respiratory system including the nasal and oral cavities, the larynx, vocal folds, oesophagus, trachea, bronchus, lung, alveolar sacs, heart and diaphragm.

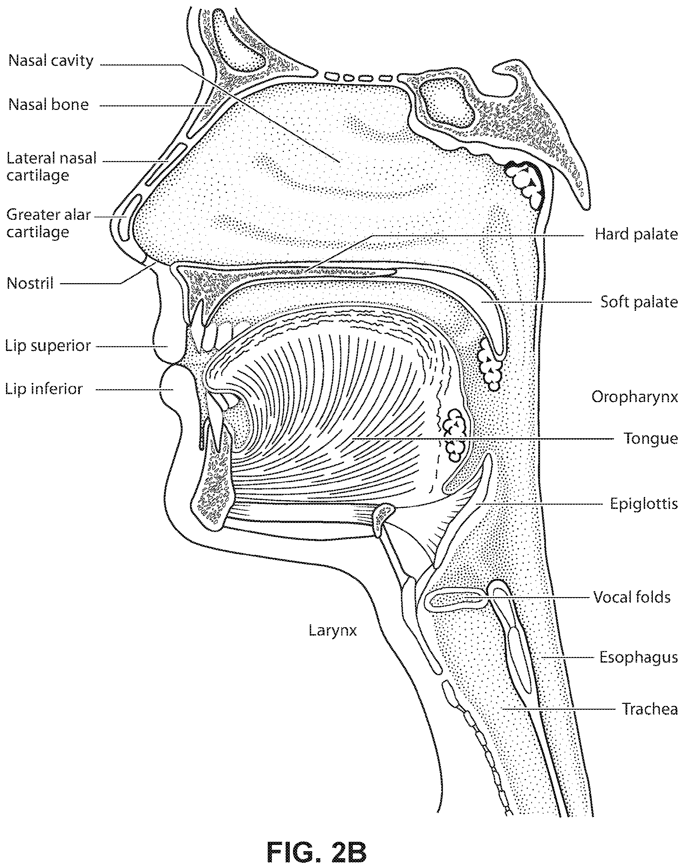

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

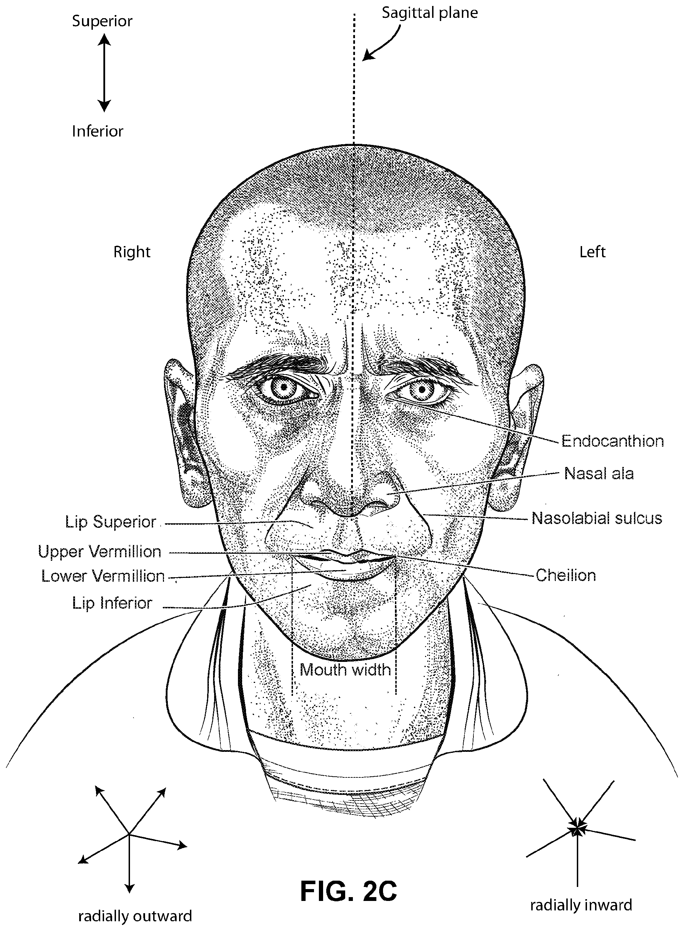

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

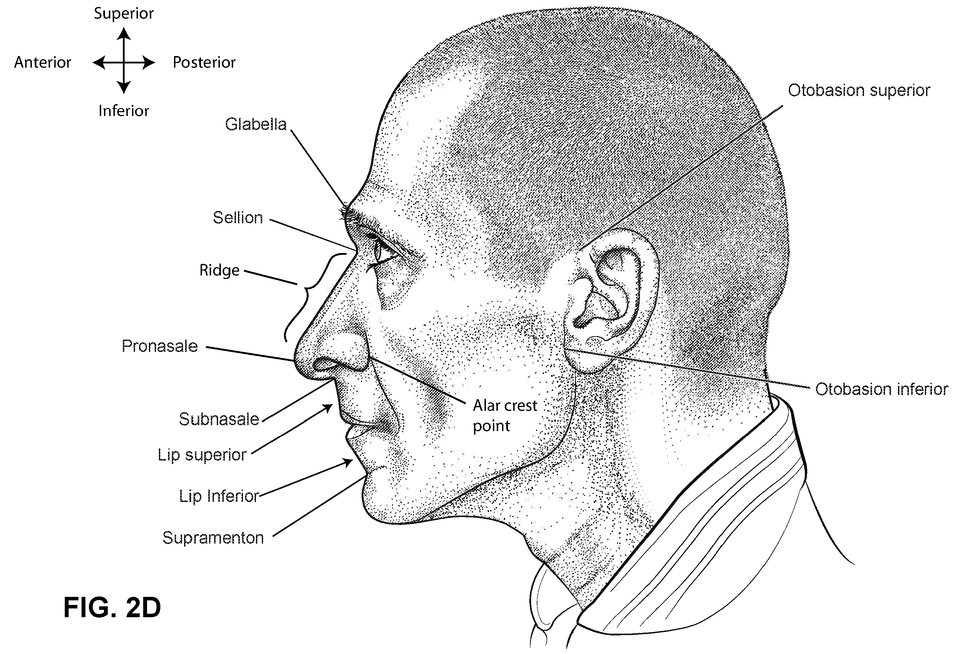

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

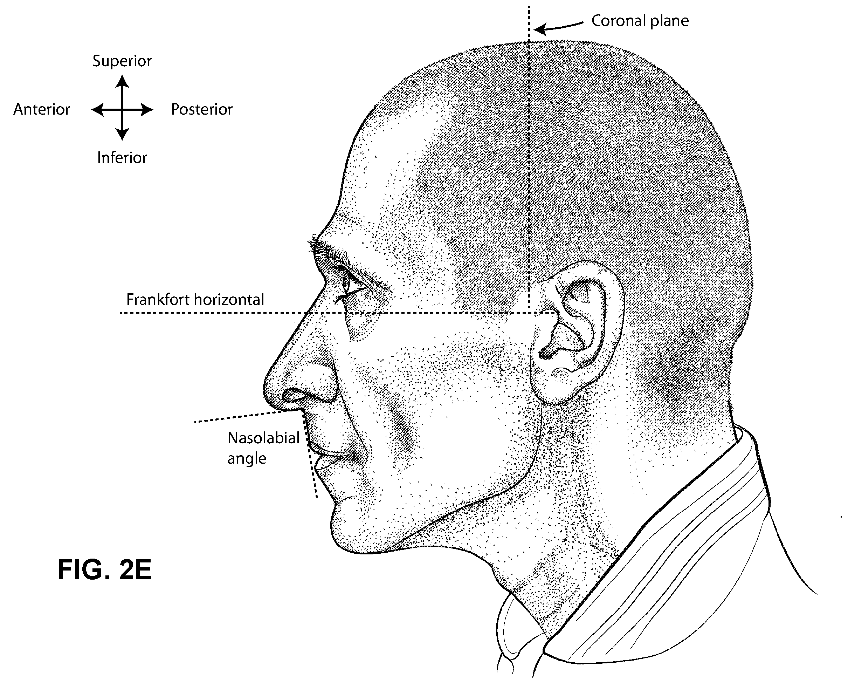

Fig. 2E is a further side view of a head. The approximate locations of the Frankfort horizontal and nasolabial angle are indicated. The coronal plane is also indicated.

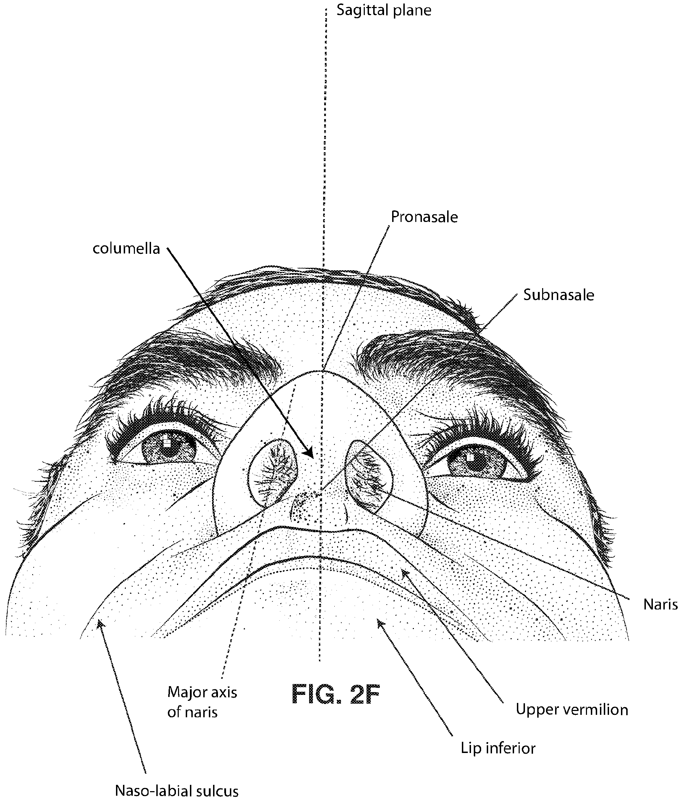

Fig. 2F shows a base view of a nose with several features identified including naso-labial sulcus, lip inferior, upper Vermilion, naris, subnasale, columella, pronasale, the major axis of a naris and the midsagittal plane.

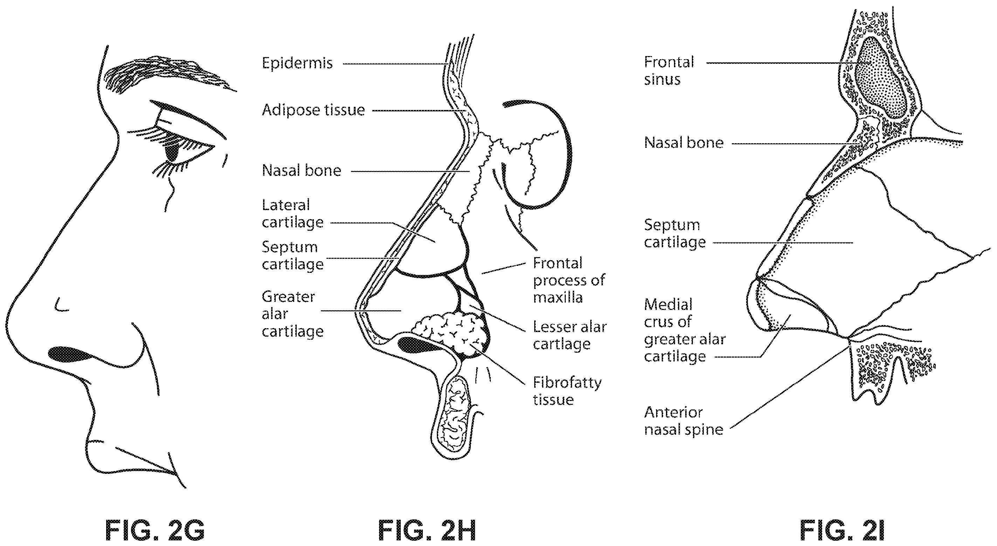

Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the midsagittal plane, amongst other things showing the septum cartilage and medial crus of greater alar cartilage.

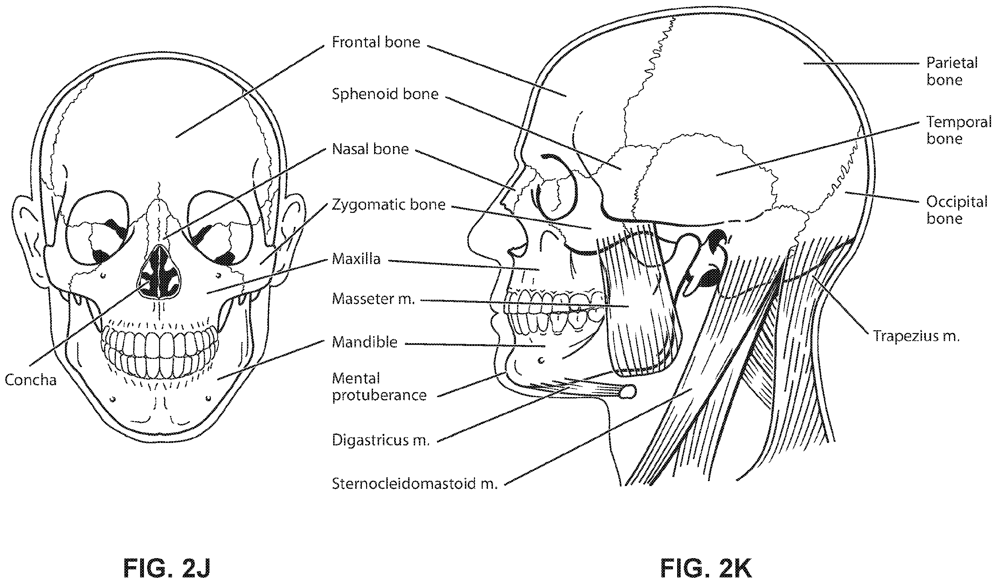

Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

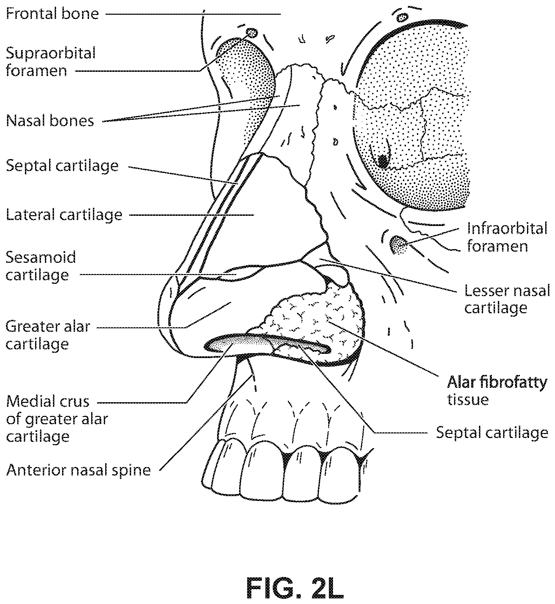

Fig. 2L shows an anterolateral view of a nose.

### 4.3 PATIENT INTERFACE

Fig. 3A shows a patient interface in the form of a nasal mask in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

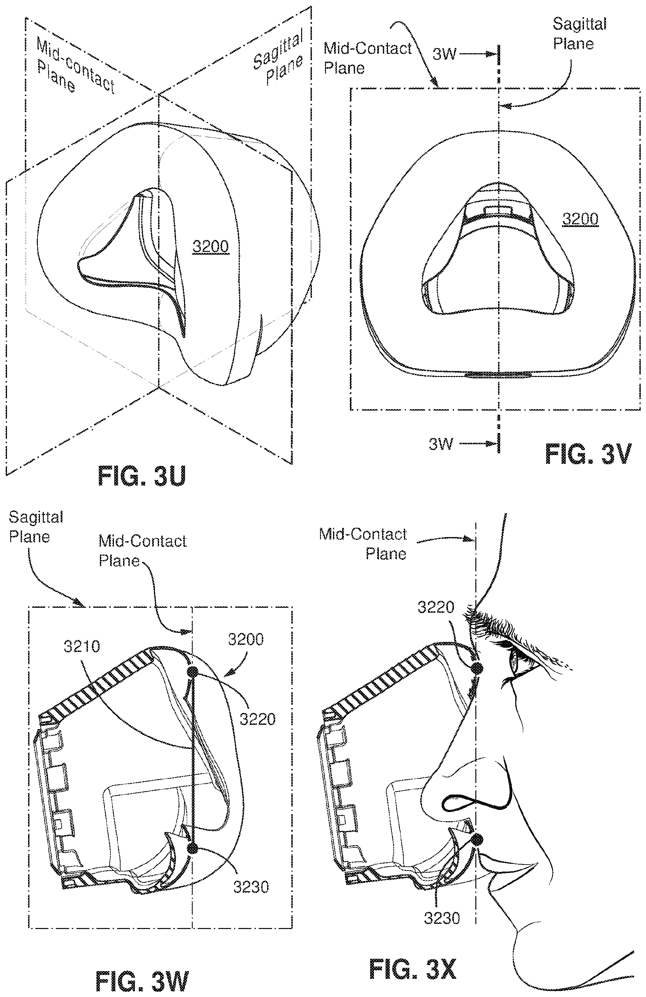

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

### 4.4 RPT DEVICE

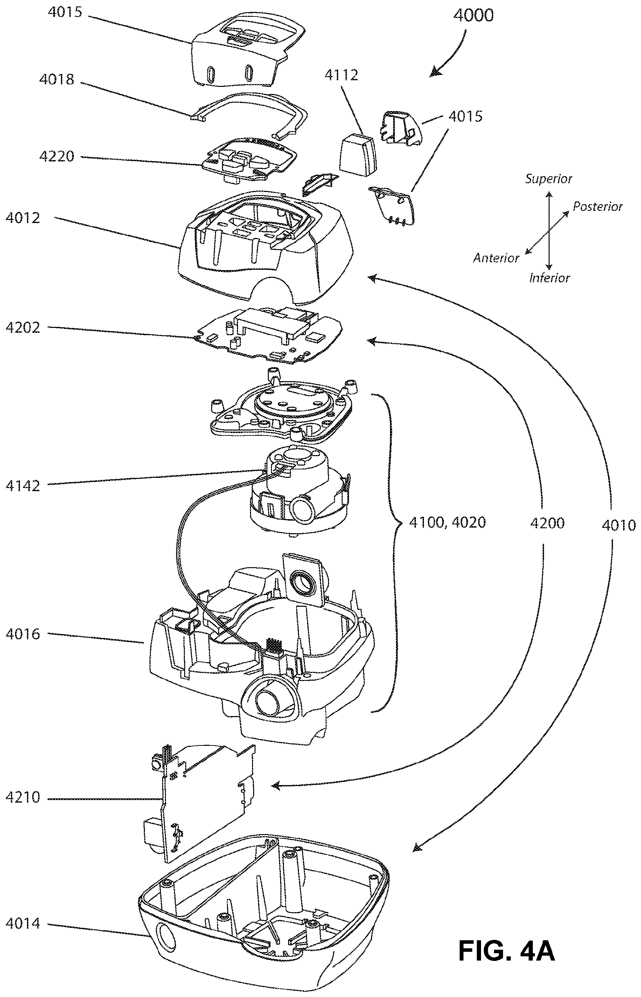

Fig. 4A shows an RPT device in accordance with one form of the present technology.

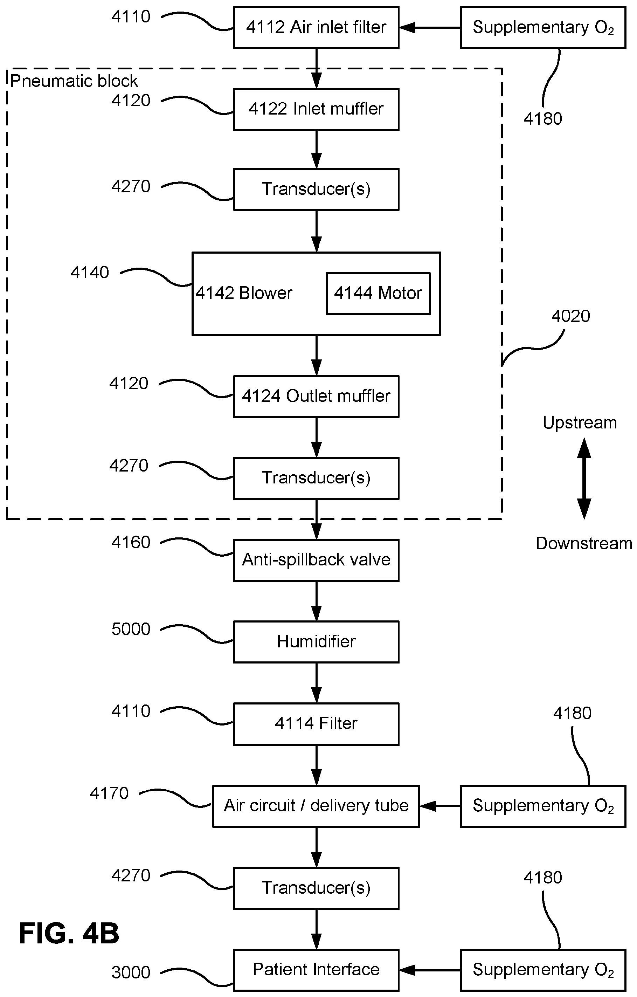

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 4.6 BREATHING WAVEFORMS

Fig. 6A shows a model typical breath waveform of a person while sleeping.

### 4.7 FRAME

Fig. 7 is a front view of a patient wearing a full-face patient interface including a frame of a first example.
Fig. 8 is front view the frame connected to a plenum chamber of the patient interface of Fig. 7.
Fig. 9 is top view the frame connected to the plenum chamber of the patient interface of Fig. 7.
Fig. 10 is side view the frame connected to the plenum chamber of the patient interface of Fig. 7.
Fig. 11 is rear perspective view the frame connected to the plenum chamber of the patient interface of Fig. 7, with a portion of the seal-forming structure removed.
Fig. 12 is a cross-sectional view of the frame and plenum chamber of Fig. 8 viewed along section 12--12, illustrating the frame engaging a groove of the plenum chamber.
Fig. 13 is a cross-sectional view of the frame and plenum chamber of Fig. 8 viewed along section 13--13, illustrating the frame engaging the groove of the plenum chamber.
Fig. 14 is a cross-sectional view of the frame and plenum chamber of Fig. 8 viewed along section 14--14, illustrating a first example of a vent connected to the frame.
Fig. 15 is a cross-sectional view of the frame and plenum chamber of Fig. 8 viewed along section 14--14, illustrating a second example of a vent connected to the frame.
Fig. 16 is a front perspective view of the frame of Fig. 7.
Fig. 17 is an exploded view of the frame of Fig. 7.
Fig. 18 is a front view of a plenum chamber of the patient interface of Fig. 7.
Fig. 19 is a front view of a patient wearing a nasal-only patient interface including a frame of a second example.
Fig. 20 is a front view of the frame of Fig. 19, connected to a sleeve.
Fig. 21 is a side view of the frame of Fig. 19, connected to the sleeve.
Fig. 22 is a rear view of the frame of Fig. 19, connected to the sleeve.
Fig. 23 is a front perspective view of the frame of Fig. 19, illustrating the sleeve partially uncoupled from the frame.
Fig. 24 is a front view of the frame of Fig. 19, disconnected from the sleeve.
Fig. 25 is a front view of the sleeve of Fig. 20.
Fig. 26 is a front view of a patient wearing a full-face patient interface including the frame of Fig. 20.
Fig. 27 is a front view of a patient wearing a full-face patient interface including a frame of a third example.
Fig. 28 is a front perspective view of the frame coupled to a plenum chamber of the patient interface of Fig. 27.
Fig. 29 is a front perspective view of the frame of Fig. 27, disconnected from the plenum chamber.
Fig. 30 is a cross-sectional view of Fig. 28 along section 30--30, illustrating an engagement between the frame and the plenum chamber.
Fig. 31 is a front perspective view of a fourth example of a frame, coupled to a plenum chamber.
Fig. 32 is a front perspective view of the frame of Fig. 31, disconnected from the plenum chamber.
Fig. 33 is a cross-sectional view of Fig. 31 along section 33--33, illustrating an engagement between the frame and the plenum chamber.
Fig. 34 is a front perspective view of the plenum chamber of the patient interface of Fig. 27.
Fig. 35 is a side view of the plenum chamber of Fig. 34.
Fig. 36 is a bottom perspective view of a conduit connection structure of the patient interface of Fig. 27.
Fig. 37 is a bottom perspective view of a conduit connection structure of the patient interface of Fig. 27, including a dividing wall at least partially separating vent openings from an emergency vent.
Fig. 38 is a perspective view of another form of a patient interface worn by a patient.
Fig. 39 is an exploded view of the patient interface of Fig. 38.
Fig. 40 is a front view of the patient interface of Fig. 38.
Fig. 41 is a rear view of the patient interface of Fig. 38.
Fig. 42 is a front view of the patient interface of Fig. 38 with the conduits disconnected from the plenum chamber.
Fig. 42-1 is a front view of the patient interface of Fig. 38 according to an alternate version with the connection members for the headgear formed separately from the conduits.
Fig. 43 is a rear view of the patient interface of Fig. 38 with the conduits disconnected from the plenum chamber.
Fig. 44 is a cross-sectional view of the plenum chamber of Fig. 42, illustrating an internal support structure.
Fig. 45 is a partial exploded cross-sectional view of the plenum chamber of Fig. 42, illustrating the plenum chamber constructed from multiple materials.
Fig. 46 is a front perspective view of another example of a vent assembly for use with an example of a patient interface.
Fig. 47 is a front view of the vent assembly of Fig. 46.
Fig. 48 is a rear view of the vent assembly of Fig. 46.
Fig. 49 is a top exploded view of the vent assembly of Fig. 46.
Fig. 49-1 is a bottom exploded view of the vent assembly of Fig. 46.
Fig. 50 is a cross-sectional view of Fig. 46 viewed along line 50--50.
Fig. 51 is a front perspective view of yet another example of a vent assembly for use with an example of a patient interface.
Fig. 52 is a front view of the vent assembly of Fig. 51.
Fig. 53 is a rear view of the vent assembly of Fig. 51.
Fig. 54 is an exploded view of the vent assembly of Fig. 51.
Fig. 54-1 is a bottom exploded view of the vent assembly of Fig. 51.
Fig. 55 is a cross-sectional view of Fig. 51 viewed along line 55--55.
Fig. 56 is an exploded view of yet another example of a vent assembly for use with an example of a patient interface.
Fig. 56-1 is a rear view of a vent body of the vent assembly of Fig. 56 having a first hole pattern.
Fig. 56-2 is a rear view of a vent body of the vent assembly of Fig. 56 having a second hole pattern.
Fig. 56-3 is a rear view of a vent body of the vent assembly of Fig. 56 having a third hole pattern.
Fig. 57 is a front perspective view of yet another example of a vent assembly for use with an example of a patient interface.
Fig. 58 is a rear perspective view of the vent assembly of Fig. 57.
Fig. 59 is a cross-sectional view of the vent of Fig. 57, viewed along line 59--59.
Fig. 60 is a front perspective view of yet another example of a vent assembly for use with an example of a patient interface.
Fig. 61 is an alternate front perspective view of the vent assembly of Fig. 60.
Fig. 62 is a rear perspective view of the vent assembly of Fig. 60.
Fig. 63 is a cross-sectional view of the vent assembly of Fig. 60, viewed along line 63--63.
Fig. 64 is a front perspective view of yet another example of a vent assembly for use with an example of a patient interface.
Fig. 65 is an alternate front perspective view of the vent assembly of Fig. 64.
Fig. 66 is a rear perspective view of the vent assembly of Fig. 64.
Fig. 67 is a cross-sectional view of the vent assembly of Fig. 64, viewed along line 67--67.
Fig. 68 is a front perspective view of yet another vent assembly for use with an example of a patient interface.
Fig. 69 is another front perspective view of the vent assembly of Fig. 68.
Fig. 70 is a rear perspective view of the vent assembly of Fig. 68.
Fig. 71 is a front exploded view of the of the vent assembly of Fig. 68.
Fig. 72 is a rear exploded view of the vent assembly of Fig. 68.
Fig. 73 is a cross-sectional view of the vent assembly of Fig. 68, viewed along line 73--73.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

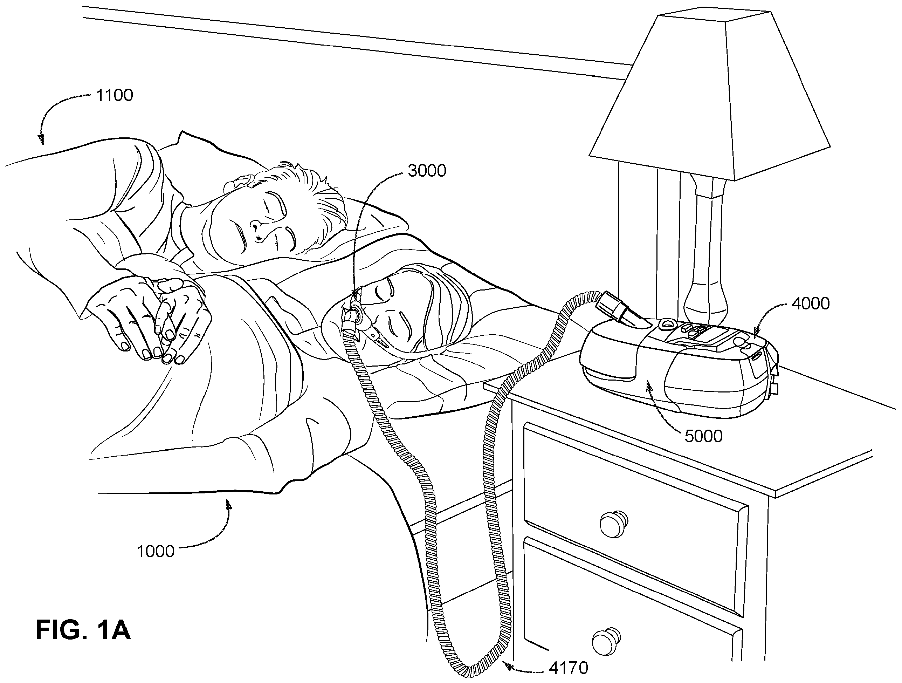

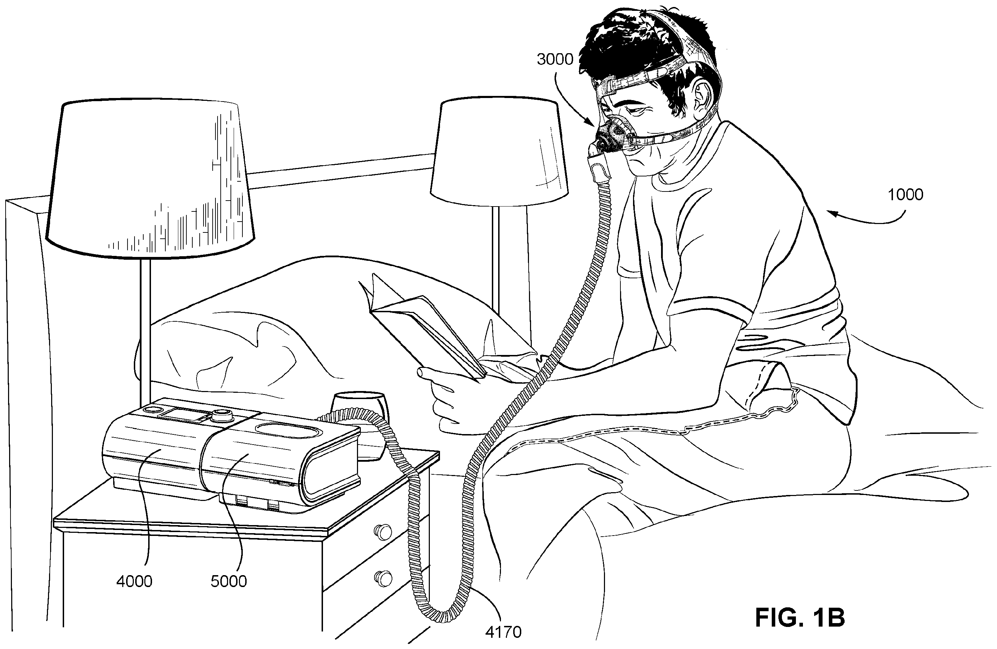

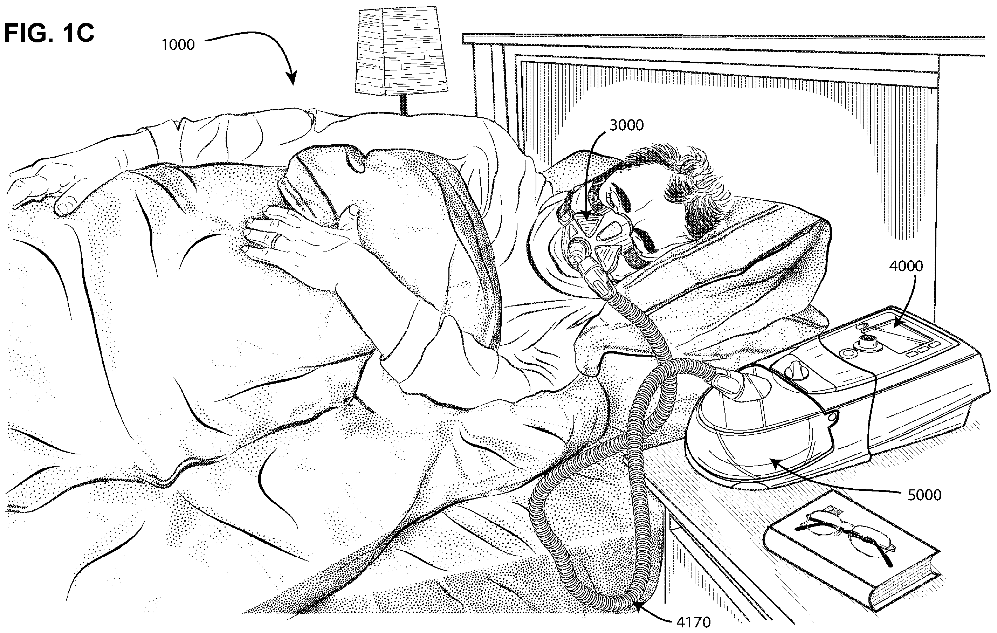

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 (see e.g., Figs. 1A to 1C).

### 5.3 PATIENT INTERFACE

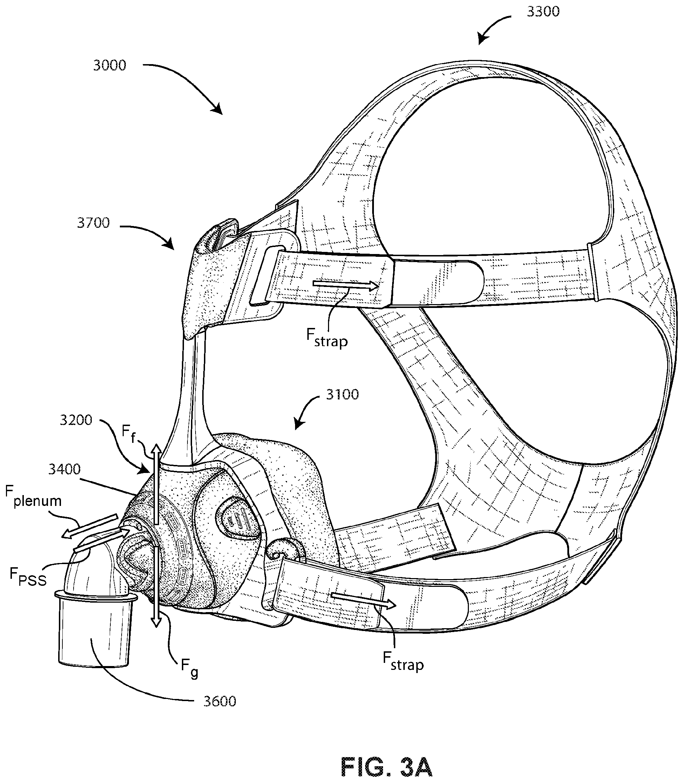

As shown in Fig. 3A, a non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

The patient interfaces 6000, 7000, 9000, 12000 may be similar to the patient interface shown in Fig. 3A, and the features and description of Fig. 3A may be applicable to any of the patient interfaces 6000, 7000, 9000, 12000.

Only some similarities and differences between the different patient interfaces may be described below. Although a certain feature may be described specifically with respect to one example, that description may be applicable to the other examples.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

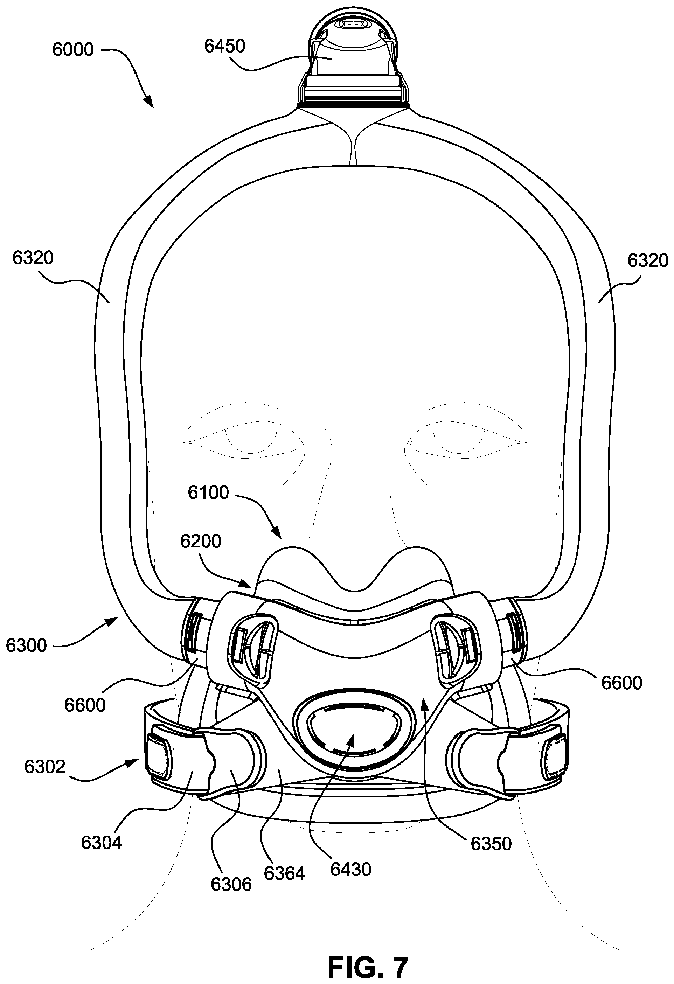

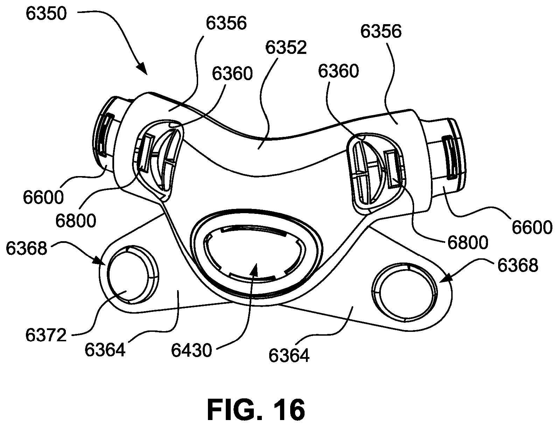

As is described in greater detail below, in certain forms of the present technology the seal forming structure 6100 comprises a first seal forming structure 6101 connected to an oral portion 6201 of the plenum chamber 6200 and constructed and arranged to form seal with a region of the patient's face surrounding an entrance to the patient's mouth, and a second seal-forming structure 6102 connected to a nasal portion 6202 of the plenum chamber 6200 constructed and arranged to form a seal with a region of the patient's face surrounding an entrance to the patient's nose (see e.g., Figs. 7-18). The phrase "connected to" is used herein to refer to portions or components which are formed as a single piece as well as to portions or components which are formed separately and subsequently joined together. In some cases components may be connected by an intermediate component.

In certain forms, the first seal forming structure 6101 seals independently against the patient's face from the second seal forming structure 6102.

In certain forms, the first seal forming structure 6101 and the second seal forming structure 6102 cooperate to form a single common seal against the patient's face.

In one form the target seal-forming region is located on an outside surface of the seal-forming structure 6100.

In certain forms of the present technology, the seal-forming structure 6100 is constructed from a biocompatible material (e.g. silicone rubber, textile, foam, etc.).

A seal-forming structure 6100 in accordance with the present technology may be constructed from a soft, flexible, resilient material (e.g., silicone, textile, foam, etc.). The seal-forming structure 6100 may also be constructed from multiple soft, flexible, resilient materials. For example, a portion of the seal-forming structure 6100 may be silicone and another portion may be textile.

In certain forms of the present technology, a system is provided comprising more than one a seal-forming structure 6100, each being configured to correspond to a different size and/or shape range. For example the system may comprise one form of a seal-forming structure 6100 suitable for a large sized head, but not a small sized head and another suitable for a small sized head, but not a large sized head.

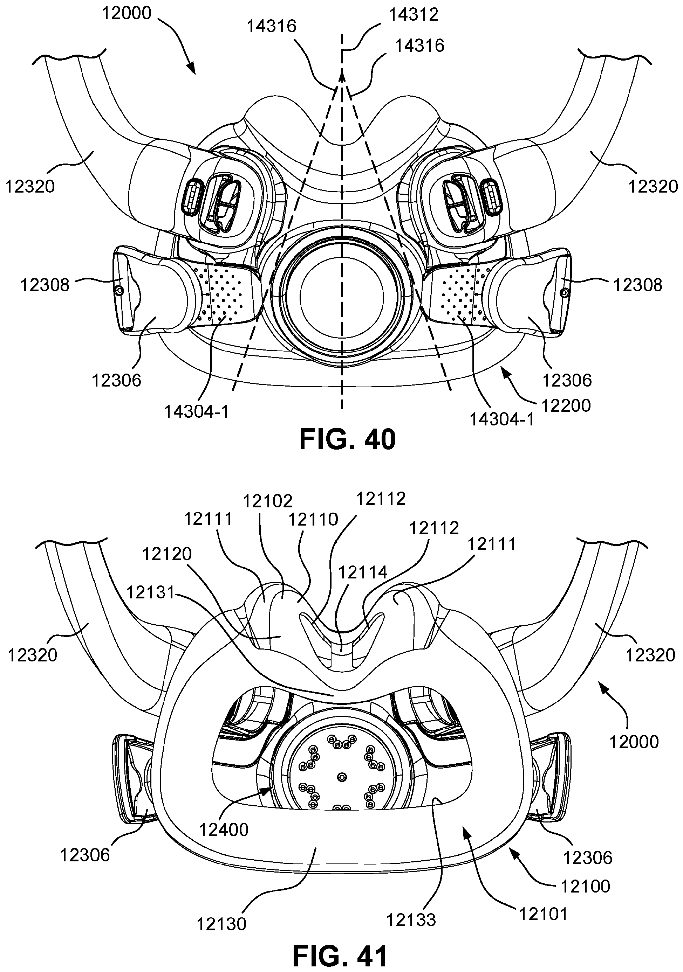

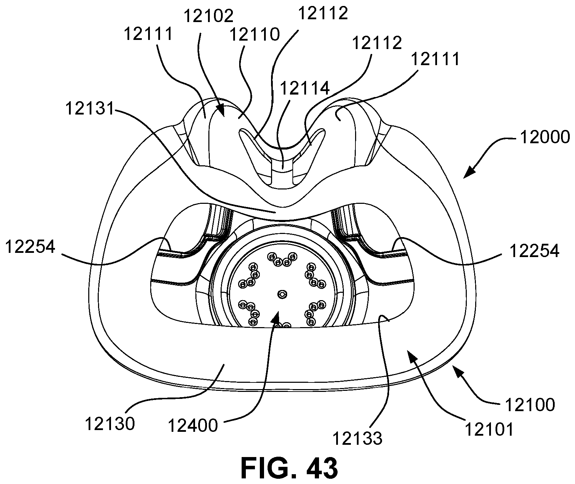

As shown in Figs. 41 and 43, the seal-forming structure 12100 of the patient interface 12000 is substantially similar to the seal-forming structure 6100. For example, the seal-forming structure 12100 includes a first seal forming structure 12101 and a second seal forming structure 12102.

In the illustrated example, the first and second seal forming structures 12101, 12102 may be formed from a single piece of material and may include substantially no transitions or corners between the two portions. For example, the first and second seal forming structures 12101, 12102 may be molded together (e.g., using silicone) to form the single piece, they may be formed from a single piece of textile material, or any other similar process.

### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nasal region

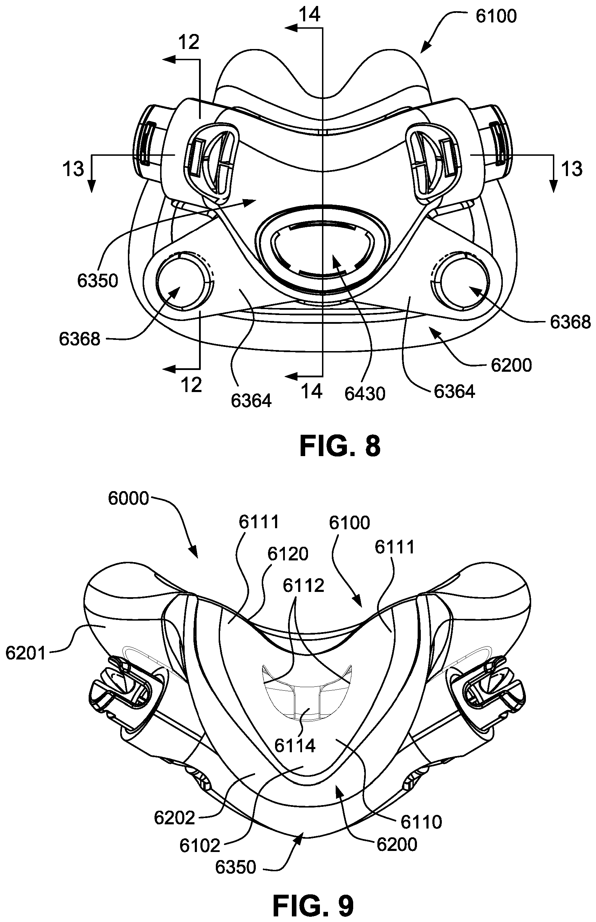

Referring next to Figs. 9 to 11, in certain forms of the present technology, the second seal forming structure 6102 comprises a central portion 6110 configured to seal to surfaces of the patient's nose in use. The central portion may seal to an inferior periphery of the patient's nose (e.g. surrounding the patient's nares) and to the patient's lip superior. In examples a portion of the seal forming structure 6100 may engage the patient's septum. The second seal forming structure 6102 may further comprise lateral portions 6111 on lateral sides of the central portion 6110. In examples, the seal forming structure 6102 may be configured to contact the patient's face below the bridge of the nose or below the pronasale.

In some forms, the central portion 6110 may include nasal openings 6112 that conveys pressurized breathable gas to the patient's nares. There may be one nasal opening 6112 for each nostril (although there may be a single nasal opening). A periphery of the nasal openings 6112 may seal against the patient's nose (e.g., against the patient's alar rims).

As shown in Fig. 9, some forms of the central portion 6110 may include a bridge portion 6114 formed between the nasal openings 6112. In use, the bridge portion 6114 may contact the patient's columella and/or subnasale region. The bridge portion 6114 may also contact the patient's nose proximate to the pronasale, but may not contact the ridge of the patient's nose. The bridge portion 6114 may seal against the patient's nose so that an entire perimeter of each nasal opening 6112 seals against the patient's nose (e.g., in order to limit leaks). The bridge portion 6114 may also limit the patient's nose from extending into the plenum chamber 6200.

In some forms, a patient interface 7000 may include only a second seal forming structure 7102, and may be referred to as a nasal only mask (described in more detail below). The second seal forming structure 7102 may form a complete sealing perimeter in order to seal completely around the patient's nasal openings. The patient's mouth may remain exposed to ambient pressure while the patient wears the patient interface 7000.

As shown in Figs. 41 and 43, a central portion 12110 of the second seal forming structure 12102 may include a bridge portion 12114 formed between the nasal openings 12112. As described with respect to the central portion 6110, the bridge portion 12114 may contact the patient's columella at least partially between the patient's pronasale and subnasale. The bridge portion 12114 may not contact the patient's nose beyond or substantially beyond the patient's pronasale in order to avoid contact with the ridge of the patient's nose. The bridge portion 12114 may seal against the patient's nose so that an entire perimeter of each nasal opening 12112 seals against the patient's nose (e.g., in order to limit leaks). The bridge portion 12114 may also limit the patient's nose from extending into the plenum chamber 12200.

In some forms, the bridge portion 12114 may be substantially flat between the nasal openings 12112. This may be as a result of a molding process which gives the bridge portion 12114 its shape. In some examples, the bridge portion 12114 may be in a taut position prior to use by the patient. In other examples, the bridge portion 12114 may be at least partially slack prior to use, and may be under tension as a result of contact with the patient's nose.

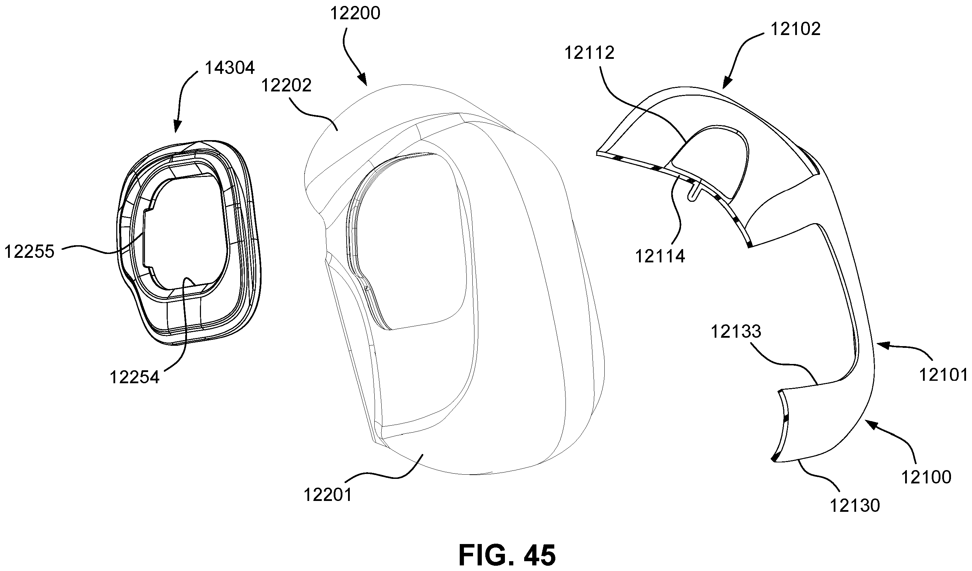

As illustrated in Fig. 45, some examples of the bridge portion 12114 may be crimped in order to apply localized tension to the bridge portion 12114 prior to use. For example, the second seal forming structure 12102 may be constructed initially with a relaxed bridge portion 12114, and a crimp may be applied during the manufacturing process in order to increase the tension in the bridge portion 12114. In some forms, the bridge portion 12114 may be crimped in order to allow the second seal forming structure 12102 (or the entire seal-forming structure 12100) to be constructed from a textile material with complex curvatures (e.g., curvatures along multiple, non-parallel axes). The crimped bridge 12114 may limit interactions between the various complex curvatures in order to limit the occurrence of leak-forming creases across the surface of the second seal forming structure 12102. The process of crimping is described in WO 2021/207799.

In some forms, the crimp may be applied to the bridge portion 12114 using an adhesive (e.g., glue). In some forms, the crimp may be applied to the bridge portion 12114 using stitching. In some forms, the crimp may be applied to the bridge portion 12114 using ultrasonic welding. In some forms, the crimp may be applied to the bridge portion 12114 using radio-frequency (RF) welding. In some forms, multiple techniques may be used to form the crimp on the bridge portion 12114.

In some forms, the central portion 12110 may include a positive curvature between the lateral portions 12111. The central portion 12110 may have a substantially small radius of curvature in order to have a tight fit around the patient's nose.

### 5.3.1.3 Oral region

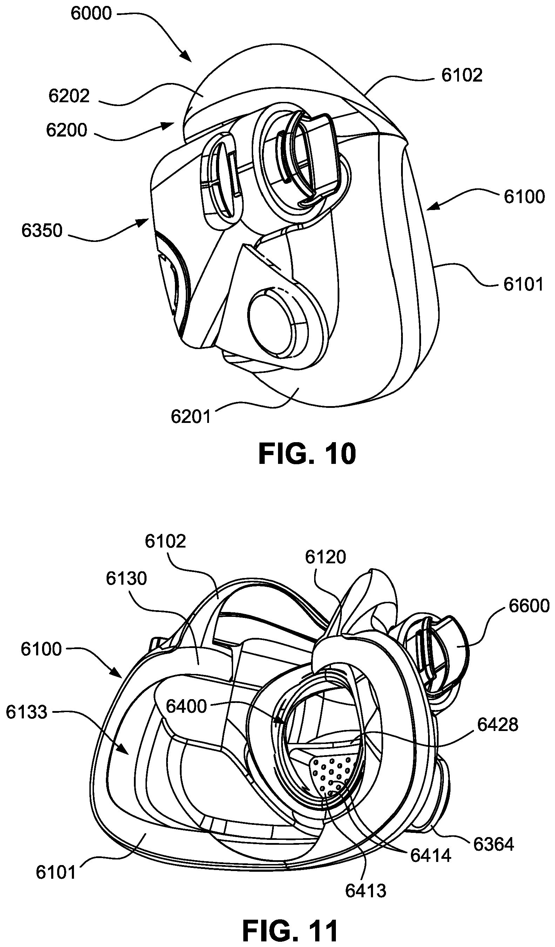

As is described above, Fig. 11 shows one form of the non-invasive patient interface 6000 comprises a first seal-forming structure 6101 that forms a seal in use at least partly around the patient's mouth. The first seal forming structure 6101 may form a seal on an inferior region of the patient's face (e.g., the patient's lip inferior and/or the supramenton).

The seal-forming structure 6100 comprises a lip inferior portion 6130 which forms a seal against the lip inferior and/or supramenton of the patient. The lip inferior portion 6130 may be connected to (e.g. contiguous with) a lip superior portion 6131, which forms a seal against the lip superior of the patient. The connection between the lip inferior portion 6130 and the lip superior portion 6131 may form an oral hole 6133.

The seal-forming structure 6100 comprises a relatively low wall thickness (compared to other portions of the interface), for example less than 0.7mm, at a periphery of the oral hole 6133, the lip inferior portion 6130 of the seal forming structure which lies against the inferior region, and at least the centre of the lip inferior portion 6130. The low wall thickness in these locations assists in achieving an effective, comfortable seal. The seal-forming structure 6100 in these regions is able to readily conform to any complex geometry.

In some forms of the technology the oral hole 6133 is substantially trapezoidal rather than oval or elliptical, in order to more accurately correspond to a shape of the patient's face (e.g., wider beneath the patient's mouth and narrower proximate to the patient's nose). This shape of oral hole may allow the interface 6000 to be particularly compact, and not be substantially wider than a width of the patient's nares.

As shown in Figs. 41 and 43, the first seal forming structure 12101 may have a substantially similar shape as the first seal forming structure 6101. For example, the oral hole 12133 may have a substantially rectangular or elliptical shape in order fit the patient's mouth. The lip inferior portion 12130 may be continuous with the lip superior portion 12131 as described above, which may limit seams or other discontinuities that could otherwise cause discomfort.

### 5.3.1.4 Boundary between nasal and oral regions

With particular reference to Figs. 9 and 11, in one form of the technology, the boundary between the first sealing forming structure 6101 and the second seal forming structure 6102 forms or comprises a corner or ridge 6120. The corner or ridge 6120 may provide an at least partially sharp boundary between the first and second seal-forming structures 6101, 6102. The corner or ridge 6120 may be rounded, but may include a small radius of curvature.

The corner or ridge 6120 may form a partition between the lip superior portion 6131 of the first seal forming structure 6101 and the central portion 6110 of the second seal forming structure 6102. In use, the corner or ridge 6120 may engage the patient's face above the lip superior and immediately below the nose. The sharp boundary may allow the corner or ridge 6120 to contact the subnasale, but the slight radius of curvature may not significantly decrease patient comfort (e.g., because the corner or ridge 6120 digs into the patient's face).

In some forms, the ridge 6120 forms a relatively sharp angle between the first and second seal forming structures 6101, 6102. The sharp angle reduces the likelihood of creases forming in the first and/or second seal forming structures 6101, 6102 on or adjacent to the corner or ridge 6120 when the mask is donned and therapy is applied. Some oro-nasal patient interfaces which do not use such a structure may require a very thin, rounded formation in this area which may be less resistant to creasing. By contrast, the corner or ridge 6120 may be stiffer, and may hold its shape better, than such interfaces and may therefore seal better against the concavities and creases present around the patient's nose. This effect may be enhanced in embodiments which are provided with support portions, for example support portions 6260 as described herein, which resist or oppose compression of this region.

In some forms of the technology the radius of the corner or ridge 6120 may be less than 2mm, for example around 1.75mm. In one form of the technology the radius may vary from approximately 1.75mm in the centre of the ridge to approximately 0.75mm at the lateral portions.

The angle formed by the first and second sealing structures may be about 20 degrees to about 90 degrees, for example about 36 degrees.

In some forms of the technology, the corner or ridge 6120 may extend across substantially an entire boundary 6103 between the first seal forming structure 6101 and the second seal forming structure 6102. In embodiments the corner or ridge 6120 may engage the patient's face at least approximate the entrances to the nares, for example where the ala meets the face above the lip superior.

As shown in Figs. 41 and 43, a boundary 12120 between the first and second seal forming portions 12101, 12102 may include a smooth or substantially smooth transition. The smooth surface between along the boundary 12120 may promote patient comfort because sharp surfaces are reduced. Alternatively, the boundary 12120 could be formed as a corner or ridge like in Fig. 9.

### 5.3.1.5 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

As shown in Fig. 7, the seal-forming structure 6100 may contact the patient's face in order to minimize contact with the nose bridge region or on the nose-ridge region of the patient's face. In some example, the seal-forming structure 6100 may be situated so that the patient's pronasale is exposed in use. This may increase patient comfort because sensitive regions along the nose bridge region or on the nose-ridge region of the patient's face are not in a pressurized environment.

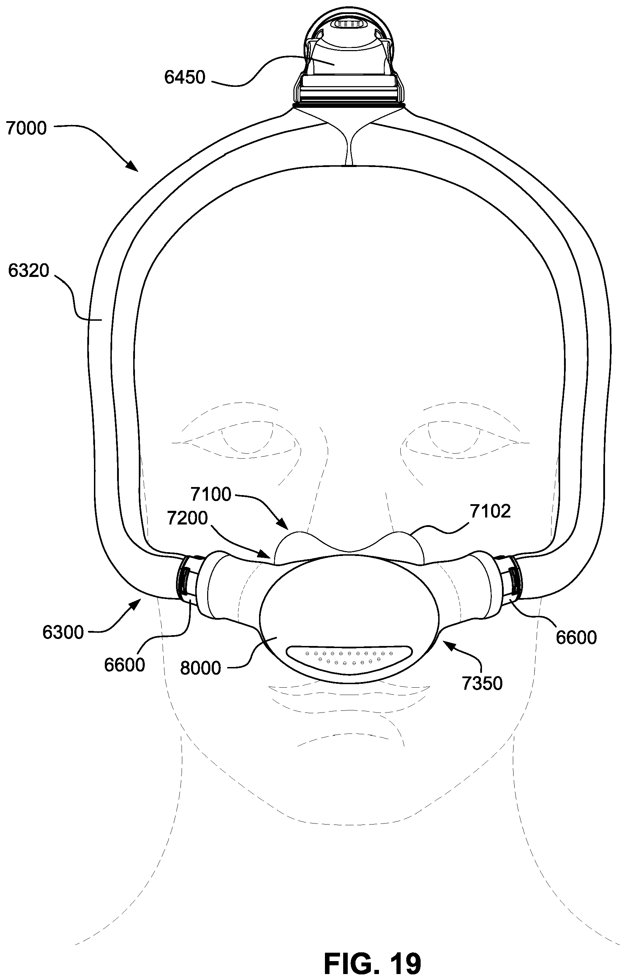

As shown in Figs. 19, 26, 27, and 38, the seal-forming structures 6100, 7100, 9100, and/or 12100 may similarly minimize contact with the nose bridge region or the nose-ridge region of the patient's face.

### 5.3.1.6 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

As described above, the upper lip region may assist in forming a seal at least partially around the patient's nares, and at least partially around the patient's mouth (e.g., in a full-face patient interface). The upper lip region may also assist in forming a seal around only the patient's nares (e.g., in a nasal-only patient interface).

### 5.3.1.7 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.8 Forehead region

In one form, the seal-forming structure that forms a seal in use on a forehead region of the patient's face. In such a form, the plenum chamber may cover the eyes in use.

### 5.3.1.9 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.2 Plenum chamber

As shown in Fig. 3A, the plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 (or at least a portion of the plenum chamber 3200) and the seal-forming structure 3100 are formed from a single homogeneous piece of material (e.g., molded silicone, woven textile, etc.). A combination of the seal-forming structure 3100 and the plenum chamber 3200 may be considered a cushion.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 5.3.2.1 Flexible Shell

In some forms of the technology, the plenum chamber 6200 may include a shell 6250, which may be constructed from a rigid material such as polycarbonate. The rigid material may provide support to the seal-forming structure 6100.

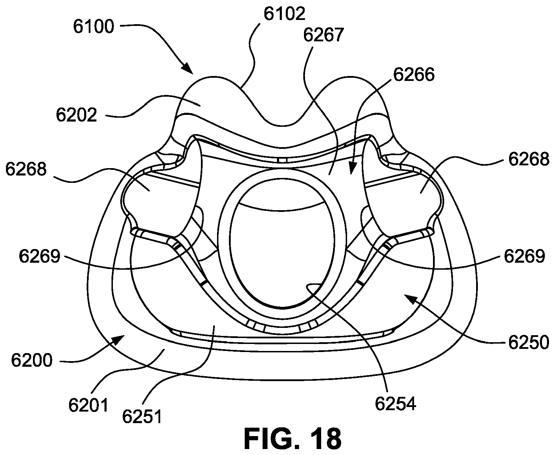

As shown in Fig. 18, the shell 6250, or portions of the shell 6250, of other forms of the technology may be somewhat flexible (e.g., constructed from a soft, flexible, resilient material like silicone, textile, foam, etc.). For example, in examples the shell 6250 may be formed from a material which has a Young's modulus of 0.4 GPa or lower, for example foam. In some forms of the technology the shell 6250 may be made from a material having Young's modulus of 0.1GPa or lower, for example rubber. In other forms of the technology the shell 6250 may be made from a material having a Young's modulus of 0.7MPa or less, for example between 0.7MPa and 0.3MPa. An example of such a material is silicone.

In examples, the shell 6250 and one or both of the first and second seal forming structures 6101, 6102 may be formed from the same material (e.g., silicone, textile, etc.). The shell 6250 and the seal-forming structures 6101, 6102 may be removable from one another or may be a single homogeneous piece of material.

In some forms of the technology (see e.g., Fig. 18), the shell 6250 may be constructed substantially entirely from a flexible material, which may provide the shell 6250 with the greatest freedom of movement (i.e., substantially no rigid and/or thickened portions that limit bending). The shell 6250 may necessitate that one or more components are added to provide a required stiffness in one or more areas or regions of the shell 6250 (e.g., in order to limit creasing of the seal-forming structure 6100 proximate to the nasal alar region). For example, one or more of a vent module; a connection port; a headgear connector; a headgear connector connected to a rigidizing arm and a rigidizing member may be connected to the shell 6250 in such a way as to increase the stiffness of the plenum chamber 6200 in the area adjacent the component, for example as described further below. In some forms of the technology such components may be releasably connectable to the flexible shell 6250.

Additionally or alternatively one more components may be permanently connected to the shell 6250, for example by bonding and/or overmolding. The rigidizing member may also serve to increase the stiffness and/or support the shape of the seal forming structure 6100. In certain forms of the present technology, the permanently connected rigidizing members may be dedicated stiffening members or rigidizing members (e.g., with no other function).

In some forms of the technology the shell 6250 may be generally flexible but may comprise stiffening portions having greater thickness than immediately adjacent portions of the shell 6250. Such stiffening portions may be configured as ribs or bands, for example extending laterally across the shell and/or extending in a superior-inferior direction, although many other configurations are possible. In some forms the shell may comprise a substantially rigid portion, for example manufactured from polycarbonate, as well as a somewhat flexible portion.

In some forms of the technology it may be preferable for a central portion 6251 of the anterior side of the oral portion 6201 of the plenum chamber to have a greater stiffness than the remainder of the plenum chamber 6200. In some forms of the technology the area of increased stiffness may be immediately inferior to the nasal portion 6202, and/or immediately superior to the oral portion 6201. In one form of the technology, a portion of, or the entirety of, the first anterior wall portion 6240 may be an area of increased stiffness, rather than an area of increased flexibility. Providing increased stiffness in one or more of these areas may provide shape stability and may limit the extent to which the shell 6250 deforms as a result of headgear forces. Excessive deformation may result in the second seal forming structure 6102 occluding the nares. Avoiding such deformation may be particularly advantageous to patients with relatively wide noses, and may be less important, or in some cases undesirable, for patients with narrow noses. In addition, the areas of increased stiffness described may assist in reducing torsional deformation of the interface which may otherwise result in one side of the second seal forming structure 6102 losing contact with the patient's nose, thereby creating a leak path.

### 5.3.2.1.1 Single opening

### 5.3.2.1.1.1 Full-face interface

As shown in Figs. 7 to 18, the plenum chamber 6200 may be a single opening plenum chamber 6200, and may include only one opening for conveying air to and from the patient. In other words, the pressurized breathable gas enters the plenum chamber 6200 through the same opening that waste gas (e.g., exhaled carbon-dioxide) exits the plenum chamber 6200.

In the illustrated forms, the plenum chamber 6200 forms part of a full-face patient interface 6000 (e.g., a full-face mask, an ultra-compact full-face mask, etc.), which includes the first and second seal forming structures 6101, 6102 described above.

As shown in Fig. 18, the plenum chamber 6200 includes an opening 6254, which as described above, may be used to convey gas into and out of the plenum chamber 6200.

In some forms, the opening 6254 includes a rounded shape. This may include an elliptical shape, as in the illustrated example, or may include a circular shape. In other forms, the opening 6254 may be symmetric about only a single axis.

In some forms, the opening 6254 may be substantially centered on the plenum chamber 6200. For example, an axis of symmetry (e.g., the major axis) of the elliptical opening 6254 may pass through a center of the plenum chamber 6200.

In some forms, the opening 6254 may be disposed on the oral portion 6201 of the plenum chamber 6200. For example, an axis through the opening 6254 (e.g., perpendicular to the opening 6254) may be aligned with the patient's mouth while the patient interface 6000 is in use.

In some forms, the plenum chamber 6200 may include a groove 6266, which may be formed as a recessed portion of the anterior surface, but may not extend into the volume of the plenum chamber 6200.

In some forms, the opening 6254 may be disposed within the groove 6266, and may be further recessed relative to the anterior-most surface of the plenum chamber 6200.

With continued reference to Fig. 18, the opening 6254 may be disposed in a central portion 6267 of the groove 6266. The central portion 6267 may be the most recessed portion of the groove 6266 (e.g., which may position the opening 6254 in closer proximity to the patient's mouth).

In some forms, the superior portion of the groove 6266 may extend to a transition between the oral portion 6201 and the nasal portion 6202 of the plenum chamber 6200 (e.g., the superior-most portion of the groove 6266 may be substantially opposite the ridge 6120 of the seal-forming structure 6100).

In some forms, the opening 6254 may be approximately the same height as the central portion 6267 of the groove 6266. In other words, there may be a substantially small gap between the edge of the opening 6254 and the edge of the groove 6266.

In certain forms, the central portion 6267 of the groove 6266 may have a generally triangular or trapezoidal shape. In other words, the central portion 6267 may be wider on one side and narrower on an opposite side. In the illustrated example, the central portion 6267 may be wider on a superior end (e.g., proximate to the nasal portion 6202 of the plenum chamber 6200), and narrower on the inferior end.

In one form, the depth of the central portion 6267 may decrease toward the center (e.g., toward the opening 6254). In other words, the central portion 6267 may have a curved surface, which may include a substantially positively domed curvature.

In some forms, the groove 6266 may also include at least one side portion 6268. In the illustrated example, the groove 6266 includes two side portions 6268, one positioned on either side (e.g., lateral sides) of the central portion 6267.

In some forms, the side portions 6268 may be substantially smaller than the central portion 6267. For example, the side portions 6268 may extend a shorter distance than the central portion 6267 in the superior-inferior direction, and/or in the lateral direction.

In some forms, a transition 6269 may separate each side portion 6268 from the central portion 6267. The transition 6269 may be an edge or corner to clearly delineate the boundary of the central portion 6267 for each side portion 6268.

In certain forms, the side portions 6268 may also have a substantially positively domed curvature, but the curvature may be different from the central portion 6267. For example, the central portion 6267 may include a larger radius of curvature than each of the side portions 6268. The transitions 6269 may form a boundary between the different curvatures.

### 5.3.2.1.1.2 Nasal-only interface

As shown in Fig. 19, the plenum chamber 7200 is included in a nasal-only patient interface 7000 (e.g., nasal pillows, nasal prongs, a nasal cradle, etc.), which may be similar to the full-face patient interface 6000 described above. Similar features may include the same reference number, plus "4000". Only some similarities and differences are described below.

The nasal-only patient interface which includes only the second seal forming structure 7102 described above. In other words, the seal-forming structure 7100 seals only around the patient's nose, and the patient's mouth is exposed to the ambient environment in use. The volume of the plenum chamber 7200 is therefore smaller than the volume of the plenum chamber 6200, and the patient interface 7000 overall may be more compact.

The plenum chamber 7200 may be a single opening plenum chamber 7200, and may include only one opening for conveying air to and from the patient. In other words, the pressurized breathable gas enters the plenum chamber 7200 through the same opening that waste gas (e.g., exhaled carbon-dioxide) exits the plenum chamber 7200.

### 5.3.2.1.2 Multiple opening

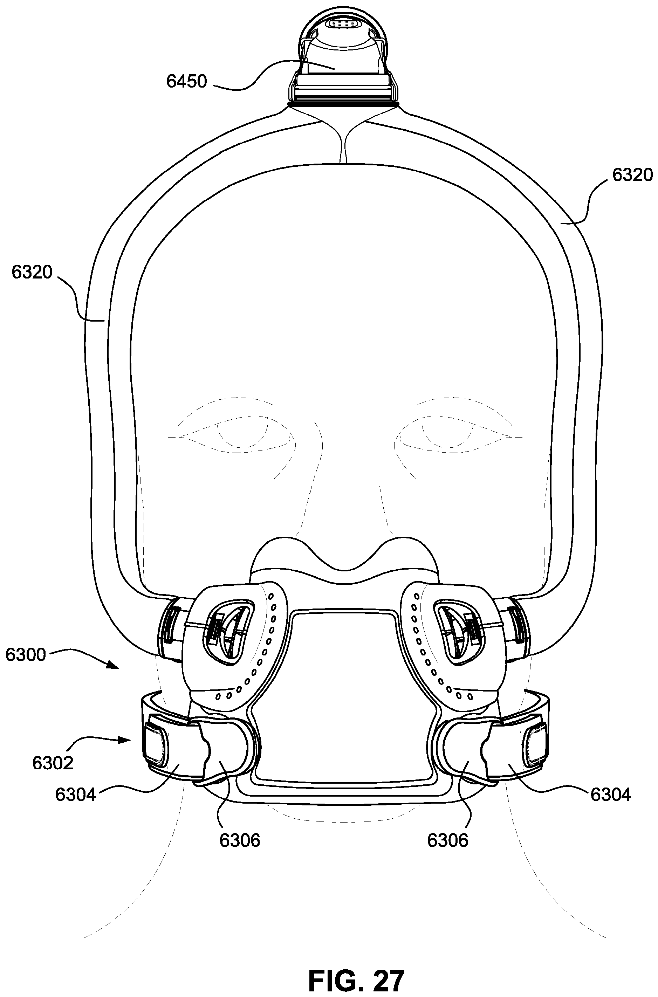

As shown in Figs. 27 to 35, the plenum chamber 9200 may be a dual opening plenum chamber 9200, and may include a pair of openings for conveying air to and from the patient. The pressurized breathable gas may enter the plenum chamber 9200 through the same opening that waste gas (e.g., exhaled carbon-dioxide) exits the plenum chamber 9200. In other words, each opening may serve as both an inlet and an outlet for the plenum chamber 9200.

In the illustrated forms, the plenum chamber 9200 is included in a full-face patient interface 9000 (e.g., a full-face mask, an ultra-compact full-face mask, etc.), which includes the first and second seal forming structures 9101, 9102 described above.

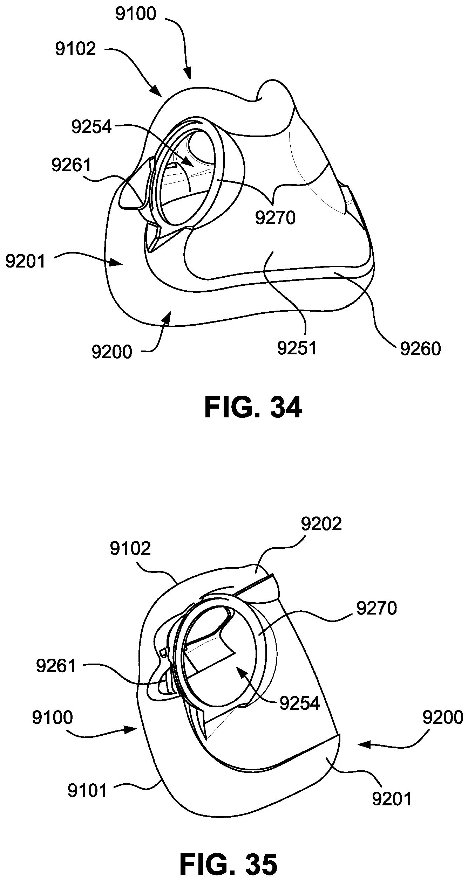

As shown in Figs. 34 and 35, the plenum chamber 9200 includes a pair of plenum chamber inlet ports 9254, which as described above, may be used to convey gas into and out of the plenum chamber 9200. The plenum chamber inlet ports 9254 may be disposed on opposite sides (e.g., left and right sides) of the plenum chamber 9200.

In some forms, the oral portion 9201 of the plenum chamber 9200 may have a substantially negatively domed curvature (e.g., when facing the anterior surface). The plenum chamber inlet ports 9254 may be positioned on the curved surface of the central portion 9251 of the plenum chamber 9200, and may be on either side of an apex of the curvature. The plenum chamber inlet ports 9254 may be aligned so that a single axis may pass through both plenum chamber inlet ports 9254. The axis may be substantially perpendicular with the patient's sagittal plane.

In some forms, each plenum chamber inlet port 9254 includes a rounded shape. This may include an elliptical shape, as in the illustrated example, or may include a circular shape or any similar shape. In other forms, each plenum chamber inlet port 9254 may be symmetric about only a single axis. Additionally, in other forms, the plenum chamber inlet ports 9254 may not be uniform with one another.

In some forms, the plenum chamber inlet port 9254 may be disposed on the oral portion 9201 of the plenum chamber 9200. In the illustrated example, each plenum chamber inlet port 9254 may extend to the transition between the oral portion 9201 and the nasal portion 9202 of the plenum chamber 9200.

In some forms, each plenum chamber inlet port 9254 may protrude from the anterior surface of the plenum chamber 9200. In other words, the plenum chamber inlet ports 9254 may be more anterior than at least some other portion of the plenum chamber 9200.

In certain forms, each plenum chamber inlet port 9254 may be inclined with respect to a center of the plenum chamber 9200. For example, each plenum chamber inlet port 9254 may be inclined with respect to the patient's sagittal plane (e.g., which passes through a center of the plenum chamber 9200) while in use. In other words, an axis perpendicular to the plenum chamber inlet port 9254 and passing through a center of the plenum chamber inlet port 9254 is inclined with respect to the patient's sagittal plane.

In some forms, each plenum chamber inlet port 9254 is inclined between approximately 1° and approximately 90° with respect to the sagittal plane. In some forms, each plenum chamber inlet port 9254 is inclined between approximately 10° and approximately 80° with respect to the sagittal plane. In some forms, each plenum chamber inlet port 9254 is inclined between approximately 20° and approximately 70° with respect to the sagittal plane. In some forms, each plenum chamber inlet port 9254 is inclined between approximately 30° and approximately 60° with respect to the sagittal plane. In some forms, each plenum chamber inlet port 9254 is inclined between approximately 40° and approximately 50° with respect to the sagittal plane. In some forms, each plenum chamber inlet port 9254 is inclined approximately 45° with respect to the sagittal plane.

In some forms, each plenum chamber inlet port 9254 includes a substantially planar surface 9270 that extends around the circumference of the plenum chamber inlet port 9254. A width of the planar surface 9270 may be less than a width of the plenum chamber inlet port 9254.

In some forms, a groove 9260 may be formed on the oral portion 9201 of the plenum chamber 9200. The groove 9260 may extend around an outer portion of the plenum chamber 9200, and may leave a central portion of the plenum chamber 9200 un-recessed.

In some forms, the plenum chamber inlet ports 9254 may be disposed within, or adjacent to, the groove 9260. The groove 9260 may intersect outermost sides (i.e., the posterior sides) of the plenum chamber inlet port 9254, and may not intersect innermost sides (i.e., the anterior sides) of the plenum chamber inlet port 9254. The groove 9260 also may not extend onto the nasal portion 9202 of the plenum chamber 9200.

In certain forms, the groove 9260 may extend around the entire plenum chamber 9200. In other words, the groove 9260 may form a closed perimeter (e.g., with the central portion 9251 at least partially bounded by the groove 9260).

In certain forms, the groove 9260 includes an outer portion 9261, which may be disposed adjacent to the plenum chamber inlet port 9254. The outer portion 9261 may extend along the plenum chamber 9200 at least partially in the posterior direction. The outer portion 9261 may extend at least partially along the height of the plenum chamber inlet port 9254, but may not extend substantially beyond the plenum chamber inlet port 9254 in the superior or inferior directions.

In some forms, the groove 9260 is recessed relative to a remainder of the outer surface of the plenum chamber 9200. The recessed groove 9260 may not extend substantially into the plenum chamber 9200 and obstruct the patient's face. The groove 9260 may have substantially the same depth throughout its perimeter.

The plenum chamber inlet ports 9254 may therefore project beyond both the recessed surface of the groove 9260, as well as beyond the remaining surface of the plenum chamber 9200. In other words, the plenum chamber inlet ports 9254 may be raised relative to the adjacent surface of the plenum chamber 9200. The plenum chamber inlet port 9254 may project away from the patient's face while the plenum chamber 9200 is being worn.

In some forms, the plenum chamber 9200 may be constructed in multiple sizes, in order to assist in interfacing with different patients (who may have different shaped heads). For example, the plenum chamber 9200 may include a narrow cushion (see e.g., Fig. 34) or a wide cushion (see e.g., Fig. 35) in order to fit a wide range of patients.

In some forms, the plenum chamber 9200 may include a lip 9290, which projects inwardly from the plenum chamber inlet port 9254. The lip 9290 may be recessed from the anterior surface of the plenum chamber 9200 (e.g., disposed closer to the patient than the anterior surface of the plenum chamber).

In some forms, the depth of the wide cushion of the plenum chamber 9200 may smaller than the depth of the narrow cushion of the plenum chamber 9200. For example, a distance of the between the anterior surface and posterior surface of the wide cushion may be less than the distance between the anterior and posterior surfaces of the narrow cushion. In some examples, the patient's face (e.g., the patient's nose) may be closer to the anterior surface of the plenum chamber 9200 in the wide cushion, than in the narrow cushion.

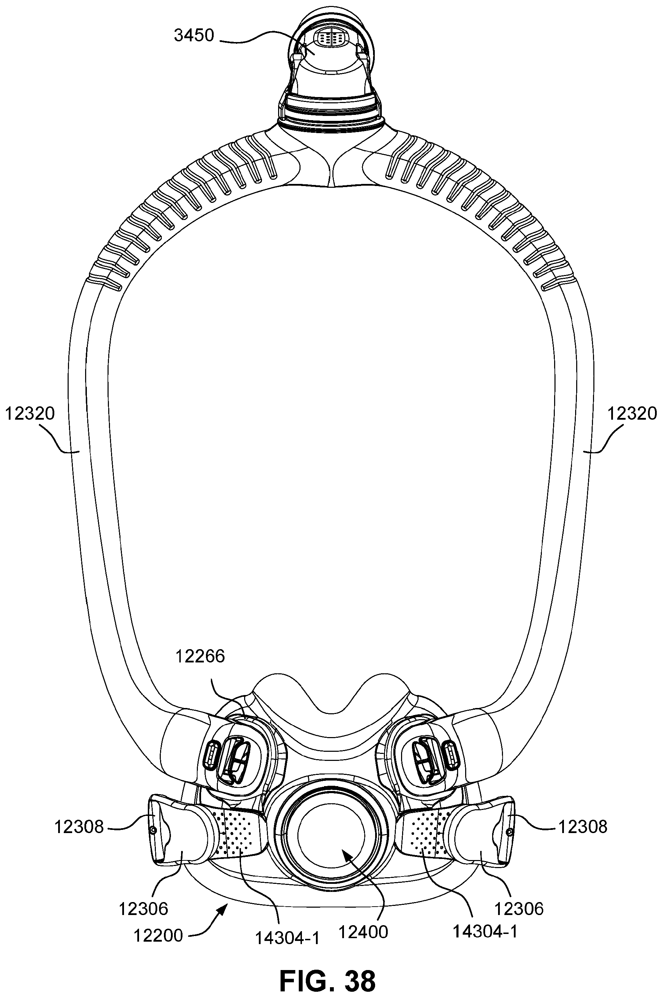

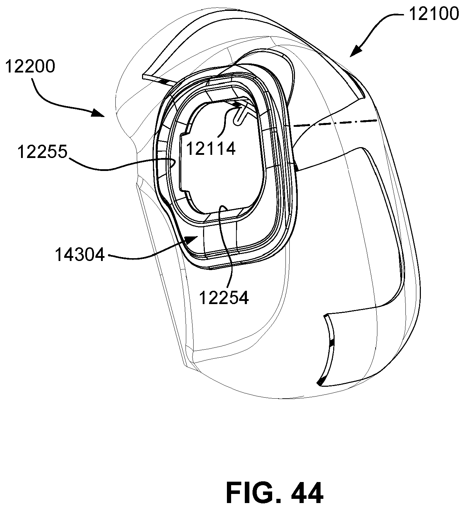

As shown in Figs. 38 to 45, a plenum chamber 12200 may include more than two openings for conveying air to and/or from the patient. The waste gas (e.g., exhaled carbon-dioxide) may exit the plenum chamber 12200 at least partially through at least one different opening than the pressurized breathable gas enters the plenum chamber 12200. In other words, at least one opening may serve entirely as an inlet or as an outlet for the plenum chamber 12200.

Although the plenum chamber 12200 includes at least one additional opening as compared to the plenum chamber 9200, the shape and the structure of the plenum chambers 9200, 12200 may be similar. Only some similarities and differences are described below.

In the illustrated forms, the plenum chamber 12200 is included in a full-face patient interface 12000 (e.g., a full-face mask, an ultra-compact full-face mask, etc.), which includes the first and second seal forming structures 12101, 12102 described above.

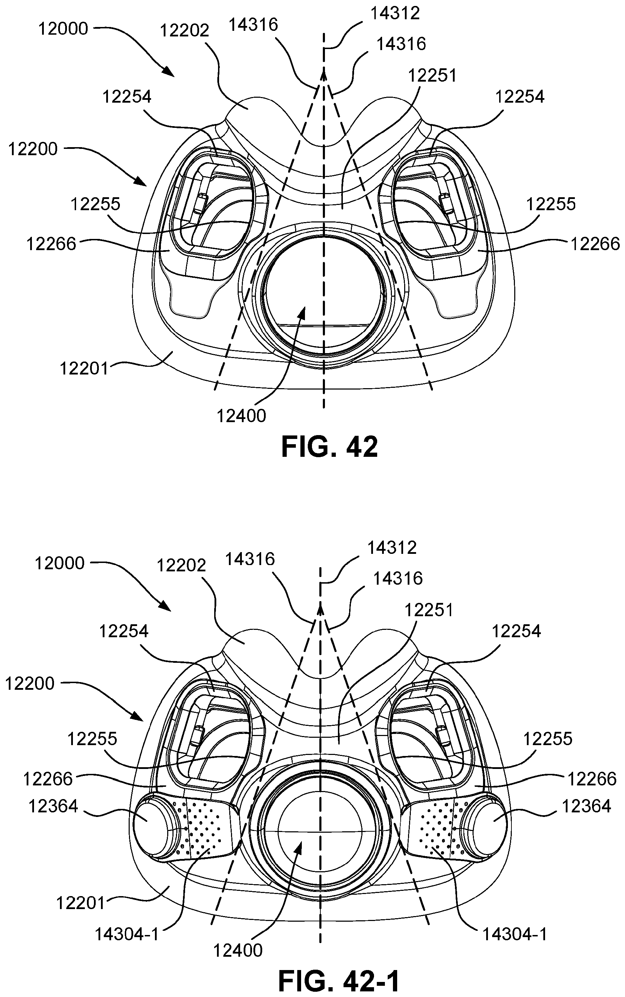

As shown in Figs. 42 and 43, the plenum chamber 12200 includes a pair of plenum chamber inlet ports 12254, which as described above, may be used to convey gas into and/or out of the plenum chamber 12200. The plenum chamber inlet ports 12254 may be disposed on opposite sides (e.g., left and right sides) of the plenum chamber 12200.

In some forms, the oral portion 12201 of the plenum chamber 12200 may have a substantially negatively domed curvature (e.g., when facing the anterior surface). The plenum chamber inlet ports 12254 may be positioned on the curved surface of the central portion 12251 of the plenum chamber 12200, and may be on either side of an apex of the curvature. The plenum chamber inlet ports 12254 may be aligned so that a single axis may pass through both plenum chamber inlet ports 12254. The axis may be substantially perpendicular with the patient's sagittal plane.

In some forms, each plenum chamber inlet port 12254 includes a partially rectangular shape. For example, the plenum chamber inlet port 12254 may include at least one substantially straight side. The corners between the different sides may also be rounded. In the illustrated example, each plenum chamber inlet port 12254 may include one curved side 12255. The curved side 12255 may be disposed proximate to a center of the plenum chamber 12200, and may extend generally in the superior-inferior direction. The remainder of the illustrated sides of the plenum chamber inlet port 12254 may be substantially straight sides, although any number of the sides may be curved. In other examples, the plenum chamber inlet ports 12254 may include an elliptical shape (like the plenum chamber inlet port 9254 of Fig. 34), a circular shape, or any similar shape.

As shown in Fig. 42, each plenum chamber inlet port 12254 may be symmetric about only a single axis. For example, an axis bisecting the curved side 12255 of each respective plenum chamber inlet port 12254 may form an axis of symmetry. As described below, this may help prevent improper connection of a conduit. Additionally, in other forms, the plenum chamber inlet ports 12254 may not be uniform with one another and/or the plenum chamber inlet ports 12254 may not have any axes of symmetry.

In some forms, the plenum chamber inlet port 12254 may be disposed on the oral portion 12201 of the plenum chamber 12200. In the illustrated example, each plenum chamber inlet port 12254 may extend proximate to the transition between the oral portion 12201 and the nasal portion 12202 of the plenum chamber 12200.

In some forms, the perimeter of the plenum chamber inlet ports 12254 may be substantially flush with the remainder of the central portion 12251. This may help to maintain a substantially small device footprint. For example, material surrounding the plenum chamber inlet ports 12254 may not extend substantially far from the patient's face and obstruct the patient's view while the patient interface 12000 is in use. In other examples, material surrounding the plenum chamber inlet ports 12254 may protrude from the central portion 12251 similar to the plenum chamber 9200 illustrated in Fig. 34.

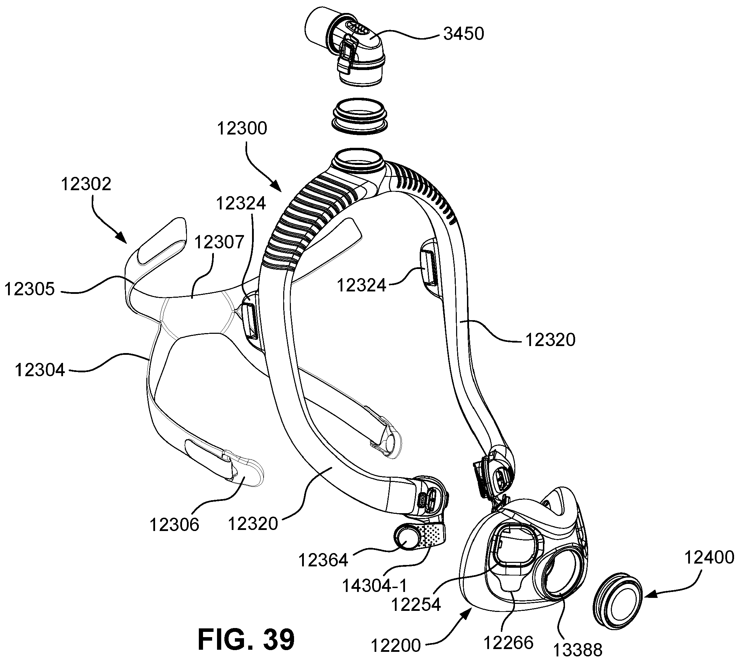

In some forms, the plenum chamber 12200 may also include at least one vent opening 13388 (see e.g., Fig. 39). The vent opening 13388 may be disposed in a center of the plenum chamber 13388. For example, the vent opening 13388 may be disposed between the plenum chamber inlet ports 12254.

In some forms, the vent opening 13388 may be disposed inferior to at least a portion of each plenum chamber inlet port 12254. For example, the vent opening may be disposed proximate to an inferior-most portion of the plenum chamber 12254.

In certain forms, the vent opening 13388 may have a rounded perimeter. For example, the vent opening 13388 may have a circular perimeter. In other examples, the vent opening 13388 may have an elliptical perimeter, or it may have a perimeter formed from a different polygonal shape (e.g., triangle, rectangle, etc.). These polygonal shapes may have angled corners, or they may have rounded corners.

In some forms, the vent opening 13388 may be aligned with the patient's mouth while the patient interface is in use. In other words, the vent opening 13388 may be disposed directly in front of the patient's mouth when he is wearing the patient interface 12000. Air exhaled by the patient (e.g., through his mouth) may travel directly toward the vent opening 13388.

In some forms, the material surrounding the vent opening 13388 may be substantially flush with the central portion 12251 of the plenum chamber 12200. This may help to maintain a substantially small device footprint. For example, material surrounding the central portion 12251 may not extend substantially far from the patient's face and obstruct the patient's view while the patient interface 12000 is in use. In other examples, material surrounding the vent opening 13388 may protrude from the central portion 12251 similar to the plenum chamber 9200 illustrated in Fig. 34.

As shown in Fig. 45, the plenum chamber inlet ports 12254 may be formed in a clip 14304. The clip 14304 may be formed from a different material than the plenum chamber 12200. For example, the plenum chamber 12200 may be constructed from a flexible material (e.g., silicone, textile, etc.) as described in any of the previous examples. The clip 14304 may be formed from a more rigid material (e.g., plastic). In some forms, the clip 14304 may be formed (e.g., molded) with the remained of the plenum chamber 12200 so that it is not separable.

In some forms, the clip 14304 may provide rigidity to the plenum chamber 12200. For example, the clips 14304 may help to retain the shape of the plenum chamber inlet ports 12254 when external forces are applied. This may help to limit leaks that may occur as a result of stretching of the plenum chamber inlet ports 12254.

In some forms, a center line 14312 of the plenum chamber 12200 may lie on the sagittal plane of the patient when the patient interface 12000 is in use. The center line 14312 may represent an axis of symmetry for the vent opening 13388. Fold lines 14316 may extend along the central portion 12251 between each respective plenum chamber inlet port 12254 and the vent opening 13388 so that each fold line 14316 intersects the center line 14312, but does not intersect the plenum chamber inlet ports 12254 or the vent opening 13388.

As shown in Fig. 42, the fold lines 14316 may intersect the center line 14312 off of the plenum chamber 12200. In other words, the location of intersection between the different lines may not be situated on the surface of the plenum chamber 12200, and may instead be located in the space around the plenum chamber 12200. For example, the location of intersection may be superior to the nasal portion 12201 (e.g. when the plenum chamber 12200 is in use).

In some forms, each fold line 14316 may intersect the center line 14312 at an angle of about 1° to about 75 °. In some forms, each fold line 14316 may intersect the center line 14312 at an angle of about 2° to about 60°. In some forms, each fold line 14316 may intersect the center line 14312 at an angle of about 5° to about 25°. In some forms, each fold line 14316 may intersect the center line 14312 at an angle of about 10° to about 15°.

In some forms, the angle of intersection between each fold line 14316 and the center line 14312 may be larger in a plenum chamber 12200 including the clip 14304 as compared to a plenum chamber 12200 including the clip 14308.

In some forms, the plenum chamber 12200 may be able to bend as a result of forces (e.g., tension) applied to the patient interface 12000. Because neither fold line 14316 intersects the vent opening 13388 or the respective plenum chamber inlet port 12254, bending the plenum chamber 12200 (i.e., about the fold lines 14316) will not substantially distort the shape of the vent opening 13388 or the plenum chamber inlet ports 12254. This may help to maintain the connection between any component inserted into the vent opening 13388 or the plenum chamber inlet ports 12254 and the plenum chamber 12200.

Additionally, the intersection location being spaced apart from the surface of the plenum chamber 12200 may be more comfortable for the patient. For example, the location where the fold lines 14316 and the center line 14312 intersect may be narrow after bending occurs, which could cause discomfort in the patient. Because no material is located at this point in the plenum chamber 12200, the superior most portion of the plenum chamber 12200 and seal-forming structure 12100 (i.e., the nasal portions 12102, 12201) may be wider and less disruptive to the patient. For example, the patient interface 12000 may not occlude, pinch, and/or cause substantial discomfort as a result of bending the plenum chamber 12200 about the fold lines 14316.

As shown in Figs. 38 and 39, certain forms of the plenum chamber 12200 may include a groove 12266. Unlike the groove 6266 in Fig. 18 that is disposed in a center of the plenum chamber 6200, the groove 12266 may be disposed long the sides of the plenum chamber 12200.

In some forms, the plenum chamber 12200 may include a pair of grooves 12266. Each groove 12266 may be disposed proximate to one of the plenum chamber inlet ports 12254. Each groove 12266 may form a partially recessed surface.

In certain forms, the clips 14304 may be removable from the central portion of the plenum chamber 12200. The grooves 12266 may each be sized to receive one of the clips 14304. Each clip 14304 may fit snuggly within the respective groove 12266, which may be accomplished using a press fit, a friction fit, or a snap fit. For example, each clip 14304 may be pressed into the plenum chamber 12200 and retained through frictional forces.

In certain forms, an area of each groove 12266 may be larger than an area of each plenum chamber inlet port 12254. Additionally, the shape of each groove 12266 may not correspond to the shape of each plenum chamber inlet port 12254 (although they may). For example, each plenum chamber inlet port 12254 may be proximate a superior end of the respective groove 12266. The groove 12266 may extend toward an inferior portion of the plenum chamber 12200 beyond the perimeter of the respective plenum chamber inlet port 12254. In some forms, the width of each groove 12266 may change along the superior-inferior direction. For example, each groove 12266 may be narrower proximate to the inferior portion of the plenum chamber 12200 and may be wider proximate to the superior portion of the plenum chamber 12200 (e.g., where the plenum chamber inlet port 12254 is located). Each groove 12266 may have substantially the same depth throughout (although the depth may be varied).

For example, the groove 12266 illustrated in Fig. 39 is larger than the clip 14304, both radially larger than the plenum chamber inlet port 12254 and larger in the inferior direction.

As shown in Fig. 42-1, certain forms of the clip 14308 may be sized to fill substantially all of the groove 12266. The clip 14308 may be similar to the clip 14304 and may include the plenum chamber inlet port 12254. The clip 14308 may be larger than an opening for the plenum chamber inlet port 12254 so that it extends from a superior portion toward an inferior portion of the plenum chamber 14308. In other words, the clip 14308 may include the shape of the clip 14304 in addition to an inferior section for connecting to a headgear strap (e.g., the arm 14304-1; described below). When connected to the plenum chamber 12200, a portion of the groove 12266 radially outside of the plenum chamber inlet port 12254 may remain unexposed, but the inferior portion of the groove 12266 may be covered (e.g., by the arm 14304-1). The superior portion of the clip 14308 (e.g., similar in shape to the clip 14304) may provide similar rigidity benefits to the plenum chamber as the clip 14304 (e.g., helps to maintain the shape of the plenum chamber inlet port 12254). The inferior portion of the clip 14308 may be positioned within the groove 12266 but may not extend into the pressurized volume of the plenum chamber 12200. In some forms, the clips 14308 may be removable from the groove 12266 (e.g., in order to facilitate cleaning). However, the clips 14308 may also be fixed within the groove 12266 (e.g., through a molding process, with an adhesive, etc.).

As shown in Fig. 42, certain forms of the plenum chamber 12200 may include fold lines 14316 that do not intersect the groove 12266, or have a substantially small intersection with the respective groove 12266. This may assist in limiting disconnection as a result of bending the plenum chamber 12200 during normal use.

In other forms, the fold lines 14316 may at least partially intersect the grooves 12266. This may allow the patient to bend the plenum chamber 12200 while not in use in order to make removing a component from the groove 12266 easier (e.g., to more easily facilitate cleaning).

### 5.3.3 Positioning and stabilising structure

As shown in Fig. 3A, the seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face (i.e., Fₚₗₑₙᵤₘ).

In one form the positioning and stabilising structure 3300 provides a retention force to overcome the effect of the gravitational force on the patient interface 3000.

With continued reference to Fig. 3A, the positioning and stabilizing structure 3300 provides a force F_{PSS} that assists in maintaining the plenum chamber 3200 in the sealing position on the patient's face. The positioning and stabilizing force F_{PSS} may be the resultant force from the various force vectors of the different elements of the positioning and stabilizing structure 3300. For example, headgear straps may individually provide a strap force Fₛₜᵣₐₚ in order to hold the seal-forming structure 3100 against the patient's face. The force Fₛₜᵣₐₚ may also be directed at least partially in the superior direction in order to overcome the gravitational force F_{g}. The gravitational force F_{g} may be specifically shown for the seal-forming structure 3100 and the plenum chamber 3200, but gravity would act on the entirely of the patient interface 3000 (i.e., in the same direction as the illustrated gravitational force F_{g}).

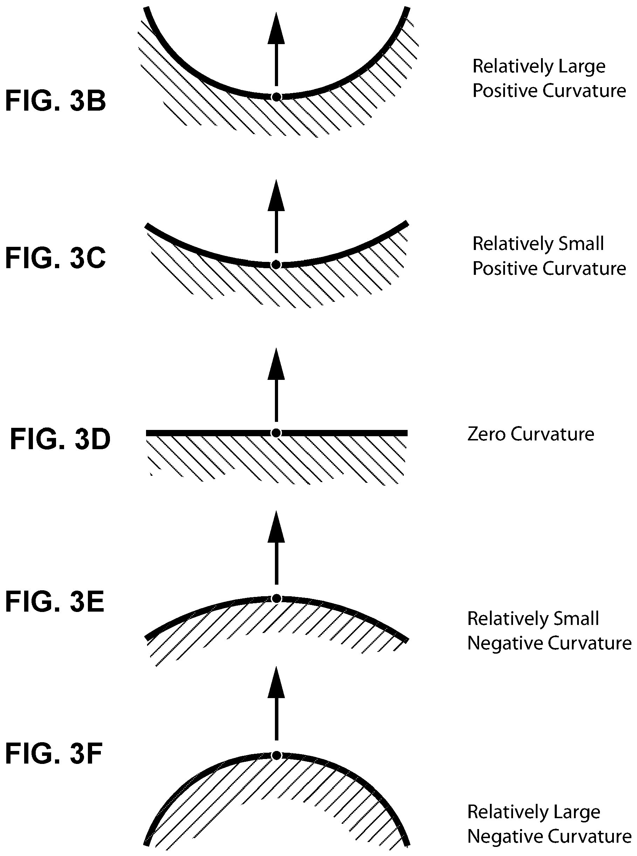

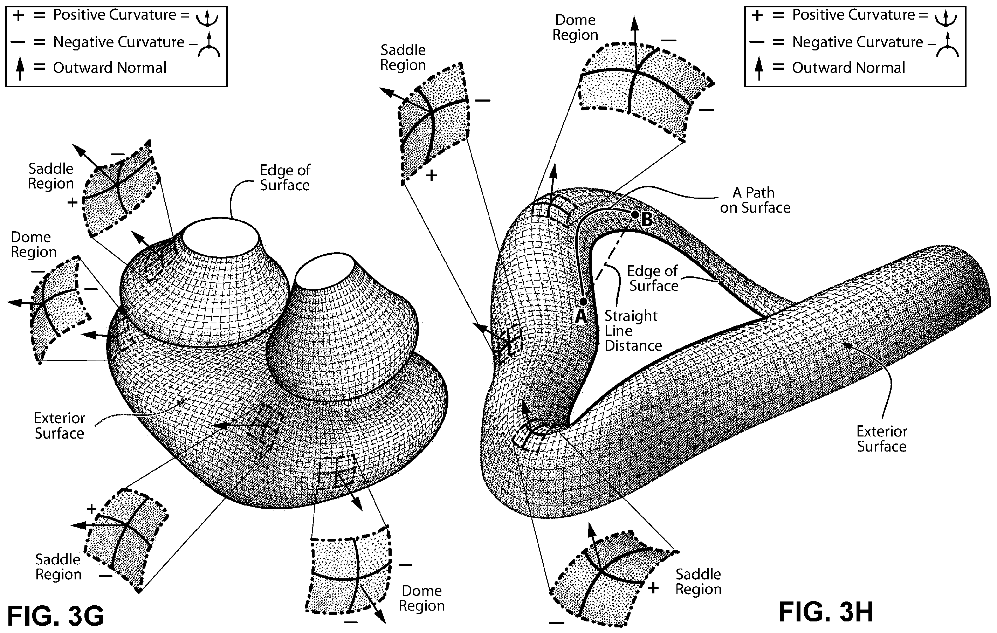

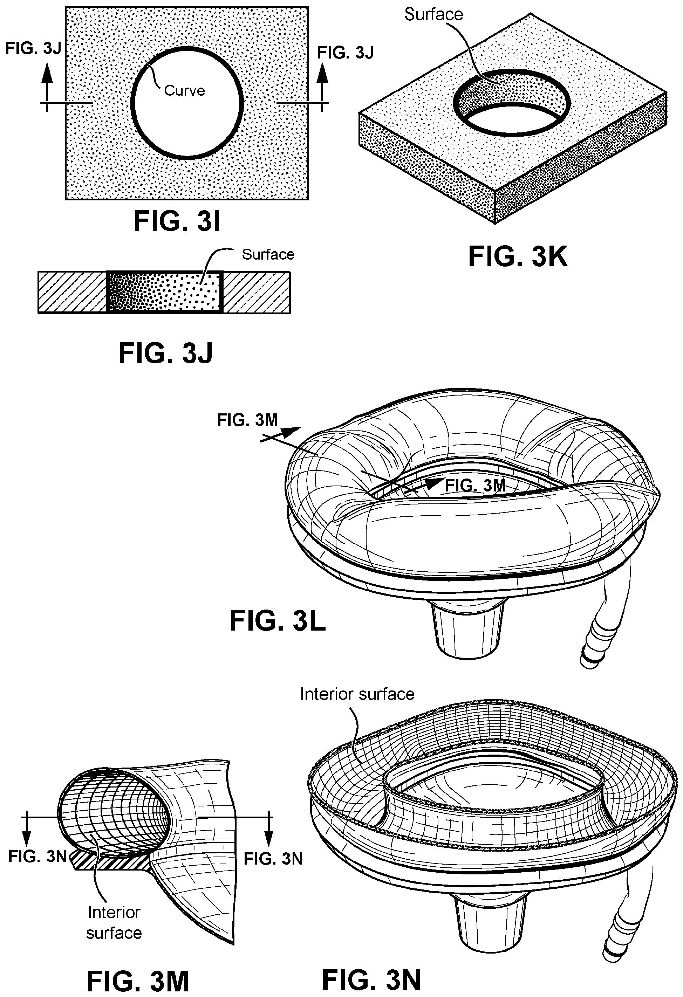

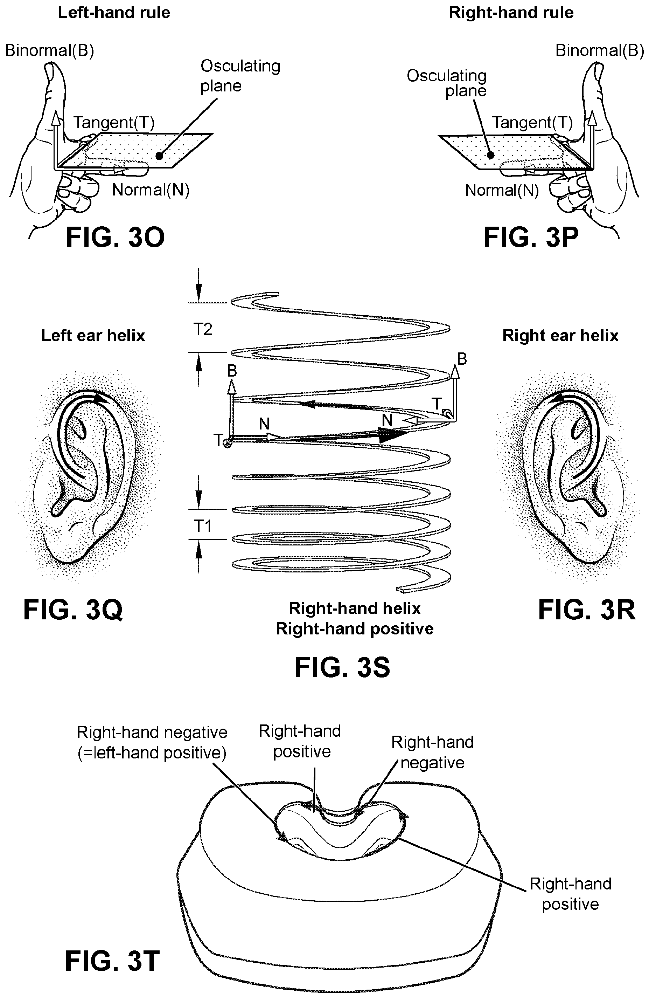

The gravitational force F_{g} may be opposed by a frictional force F_{f}, which may act in a direction directly opposite of the gravitational force F_{g}. As gravity pulls the seal-forming structure 3100 and the plenum chamber 3200 in the inferior direction (as viewed in Fig. 3), the frictional force F_{f} would act in the superior direction (e.g., against a patient's face). For example, the patient may experience the frictional force F_{f} against his lip superior (and/or other surfaces of the patient's face in contact with the seal-forming structure 3100) in order to oppose the motion in the inferior direction (which may help to stabilizing the cushion in place). Although the frictional force F_{f} is shown specifically opposing the gravitational force F_{g} of the seal-forming structure 3100 and the plenum chamber 3200, components of an overall frictional force (not shown) would also oppose the gravitational force F_{g} associated with the positioning and stabilizing structure 3300 and any other portions of the patient interface 3000. A force of friction can act along any place where the patient interface 3000 contacts the patient's skin (or hair). The frictional force F_{f} extends in the opposite direction of the gravitational force F_{g} and along the patient's skin (or hair).

In some forms, the sum of the various forces may equal zero so that the patient interface 3000 is at equilibrium (e.g., not moving along the patient's face while in use). Specifically, the gravitational force F_{g} and the blowout force Fₚₗₑₙᵤₘ tend to move the seal-forming structure 3100 away from the desired sealing position. The positioning and stabilizing force F_{PSS} is applied in order to counteract the gravitational force F_{g} and the blowout force Fₚₗₑₙᵤₘ (as well as any frictional forces F_{f}) and keep the seal-forming structure 3100 properly situated. Although the positioning and stabilizing force F_{PSS} may exceed the sum of the other forces and still maintain the seal-forming structure 3100 in an appropriate sealing position, patient comfort may be sacrificed. Maximum patient comfort may be achieved when the net force on the patient interface 3000 is zero and the positioning and stabilizing force F_{PSS} is exactly strong enough to achieve this. As described below, various positions of the patient's head while using the patient interface 3000 may determine the positioning and stabilizing force F_{PSS} necessary to achieve equilibrium.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap.

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 5.3.3.1 Frame

As shown in Figs. 7 to 17, a frame (e.g., frame 6350) may be coupled to a plenum chamber (e.g., plenum chamber 6200) in order to assist in maintaining the therapeutically effective position of the seal-forming structure. The plenum chambers illustrated in Figs. 7 to 18 may be specifically used with conduit headgear, although plenum chambers having an elbow 6500 connected in front of the patient's face may also be used.

In some forms, the frame may be constructed from a rigid or semi-rigid material (e.g., a TPE material, like Hytrel^{™}), and provides support to the seal-forming structure and/or the plenum chamber. For example, the frame may assist in maintaining the shape of the seal-forming structure and/or the plenum chamber in order to reduce leaks of pressurized air as a result of folding and/or creasing as the seal-forming structure engages the patient's face.

In some forms, the frame may be removable coupled to the plenum chamber. A patient may use the same frame with multiple plenum chambers. This may be useful when the patient is first beginning the therapy, and is trying different sized plenum chambers, in order to find an appropriate fit. Removing the frame may also be helpful when cleaning the patient interface, as the different elements of the patient interface may be cleaned separately, to help ensure a more thorough clean.

### 5.3.3.1.1 Single opening

### 5.3.3.1.1.1 Four point connection

As shown in Figs. 7 to 13, a frame 6350 may be coupled to the plenum chamber 6200 of the full-face patient interface 6000.

The frame 6350 may include a central portion 6352 with a substantially similar shape to the central portion 6267 of the groove 6266 on the plenum chamber 6200. For example, the central portion 6352 may have a substantially triangular or trapezoidal shape.

In some forms, the central portion 6352 may have a curvature substantially opposite the curvature of the central portion 6267 of the groove 6266. For example, Figs. 7, 8, and 10 illustrates that an anterior side of the central portion 6352 may include a negatively domed curvature. The central portion 6352 of the frame 6350 therefore may be at least partially spaced from the central portion 6267 of the groove 6266.

As shown in Fig. 18, certain forms of the superior and inferior edges of the central portion 6267 of the groove 6266 may include a negatively domed curvature corresponding to the curvature of the central portion 6352 of the frame 6350. In other words, while the center of the groove 6366 may curve inwardly toward the patient, the superior and inferior edges may curve away from the patient in order to better receive and interface with the central portion 6352 of the frame 6350.

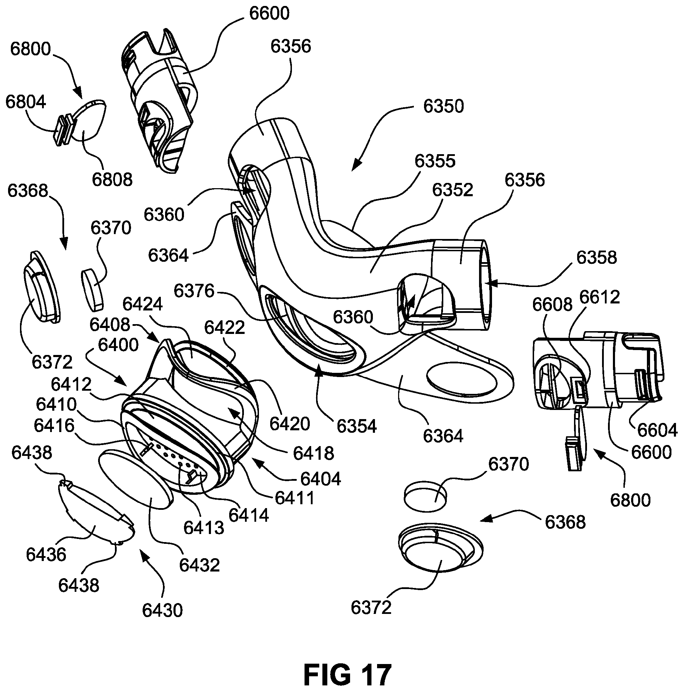

As shown in Fig. 17, some forms of the central portion 6352 may include an aperture 6354, disposed on an anterior surface of the frame 6350. The aperture 6354 may include a generally round shape. For example, the aperture 6354 may include an elliptical opening, although the aperture 6354 may also include a circular to other rounded opening.

In certain forms, the aperture 6354 may be disposed in a center of the central portion 6352. The aperture 6354 may be aligned with the opening 6254 when the frame 6350 is coupled to the plenum chamber 6200.

In certain forms, the aperture 6354 may be approximately the same size as the opening 6254, but may be oriented in a different direction than the opening 6254. For example, the aperture 6354 may be approximately 90° offset from the opening 6254. In other words, a major axis of the elliptical aperture 6354 may be along a substantially horizontal direction (e.g., when the patient interface 6000 is worn by the patient), and a major axis of the elliptical opening 6254 may be along a substantially vertical axis (e.g., when the patient interface 6000 is worn by the patient).

The frame 6350 may also include a posterior aperture 6355 disposed on a posterior surface of the central portion 6352, and connected with the aperture 6354 through the central portion 6352 of the frame 6350 (e.g., along a passageway). The posterior aperture 6354 may be substantially the same size and orientation as the opening 6254, and may interface with the opening 6254 in a sealing arrangement.

In some forms, at least one side portion or arm 6356 extends laterally from the central portion 6352 of the frame 6350. In the illustrated example, the frame 6350 includes a pair of arms 6356, one on either lateral side (i.e., left hand and right hand sides) of the frame 6350.

In some forms, the arms 6356 may be integrally formed with the central portion 6352. There may also be a smooth transition (e.g., no sharp corners) between the central portion 6352 and each of the arms 6356. In other examples, a transition between the arms 6356 and the central portion 6352 may be stepped, and/or the arms 6356 and the central portion 6352 may be connected but not integrally formed (e.g., constructed from different materials).

In some forms, the arms 6356 may extend in a posterior direction from the central portion 6352. In other words, the arms 6356 extend out of plane with respect to the central portion 6352 so that in use, the arms 6356 are more posterior than the central portion 6352.

In some forms, the arms 6356 may include a complementary shape (e.g., length and/or curvature) to the side portions 6268 of the groove 6260. For example, the arms 6356 may include an elliptic cylindrical shape, which may interface with the positively domed shape of the respective side portions 6268 of the groove 6260.

In some forms, the end of each arm 6356 may include a connection opening 6358. The connection opening 6358 may include a circular (e.g., elliptical) cross section, and may be adapted to receive a connection port 6600.

In some forms, each arm 6356 may include a vent opening 6360, which may be disposed between the central portion 6352 and the respective connection opening 6358. As described in more detail below, a portion of each connection port 6600 may be aligned with the respective vent opening 6360.

In some forms, each vent opening 6360 may be disposed on an anterior surface of the respective arm 6356, and may face away from the patient while the patient interface 6000 is in use.

In some forms, at least one inferior connector or wing 6364 is connected to the central portion 6352 of the frame 6350 and extends in a laterally outwardly direction.

In some forms, the frame 6350 may include two wings 6364, one on either side of the frame 6350 (e.g., a left side and a right side). Each wing 6364 may extend in a similar lateral direction as the respective arm 6356, but may be spaced apart from the respective arm 6356. The wings 6364 may extend at least partially in an inferior direction so that they are inferior to the arms 6356.

In certain forms, each wing 6364 may be connected to the central portion 6352 of the frame 6350 in a cantilevered configuration. A fixed end of each wing 6364 may be connected to the central portion 6352 proximate to the aperture 6354. Each wing 6364 may extend away from the central portion 6352 in order to not contact the respective arm 6356. The free end of each wing 6364 may be more inferior than each arm 6356, and may be in a common vertical plane (e.g., parallel to the patient's sagittal plane).

In certain forms, the wings 6364 may be disposed more anterior than the arms 6356. For example, the arms 6356, as described above, may extend in a posterior direction (e.g., as a result of the curvature of the frame 6350). The wings 6364 however, may not include the same curvature as the remainder of the frame 6350, and may include minimal posterior extension, as compared to the arms 6356.

In some forms, an engagement mechanism 6368 may be coupled to each of the wings 6364. For example, the engagement mechanism 6368 may be coupled proximate to the free end of each wing 6364.

In certain forms, the engagement mechanism 6368 may be a magnetic engagement mechanism, and may be used to magnetically couple to other portions of the positioning and stabilizing structure 6300. In other forms, the engagement mechanism 6368 may include a mechanical engagement (e.g., a snap fit, friction fit, press fit, etc.), hook and loop material, or any similar means of engagement.

In certain forms, the magnetic engagement mechanism 6368 includes a magnet 6370 (or magnetic material), and a cover 6372 for enclosing the magnet 6370 against the respective wing 6364. The cover 6372 may be constructed from a nonmagnetic material (e.g., plastic), but may allow the magnet 6370 to magnetically interact with another element.

In some forms, the central portion 6352 may include a posterior lip 6376, which may extend radially inward from an outer perimeter of the aperture 6354. The posterior lip may extend around the entire perimeter of the aperture 6354.

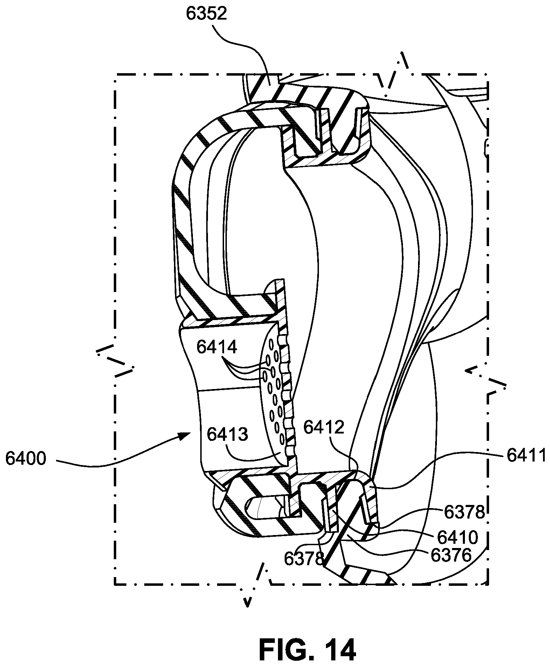

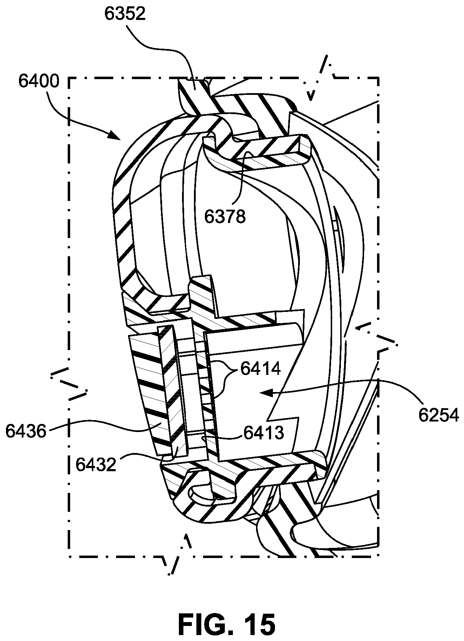

As shown Figs. 14 and 15, the posterior lip 6376 may be recessed from the anterior side of the frame 6350. In other words, the posterior lip 6376 may be disposed within the volume of the frame 6350, and may be closer to the patient than the anterior surface of the frame 6350 while the patient interface 6000 is in use.

In some forms, the posterior lip 6376 may have a substantially rectangular shape in cross-section. In some examples, the posterior lip 6376 may also be tapered proximate to the free end (e.g., in order to facilitate engagement of another member like a frame 6350).

In some forms, the posterior lip 6376 may be semi-rigid (e.g., because of its increased thickness). This may permit the posterior lip 6376 to flex, but also provides structural support to limit bending in the clockwise and counter-clockwise directions.

As shown in Fig. 14, some forms of the frame 6350 may include a surface 6378 on either side of the posterior lip 6376. The length of each surface 6378 may be substantially less than the height of the posterior lip 6376.

In some forms, the anterior surface 6378 may be wider on a superior end of the central portion 6352 than on an inferior end. For example, the anterior surface of the central portion 6352 may be inclined so that the superior region may be further away from the posterior lip 6376 than the inferior region. However, the posterior lip 6376 may be disposed in a plane substantially perpendicular to the patient's sagittal plane while the patient interface 6000 is in use.

As shown in Fig. 15, some forms of the central portion 6352 of the frame 6350 may include a single surface 6378. In other words, the surface 6378 may form the inner perimeter of the posterior aperture 6355 (see e.g., Fig. 17), and may not be divided by a posterior lip 6376. The surface 6378 may be at least partially recessed relative to the anterior surface of the frame 6350.

In some forms, the frame 6350 may be connected to the plenum chamber 6200 with a mechanical engagement. This may include, but is not limited to, a press fit, a friction fit, and/or a snap fit.

The posterior aperture 6355 may be aligned with the opening 6254 of the plenum chamber 6200. The posterior aperture 6355 and the opening 6254 may be approximately the same size and orientation, which may allow the surface of the frame 6350 that forms the posterior aperture 6355 to translate into through the opening 6254.

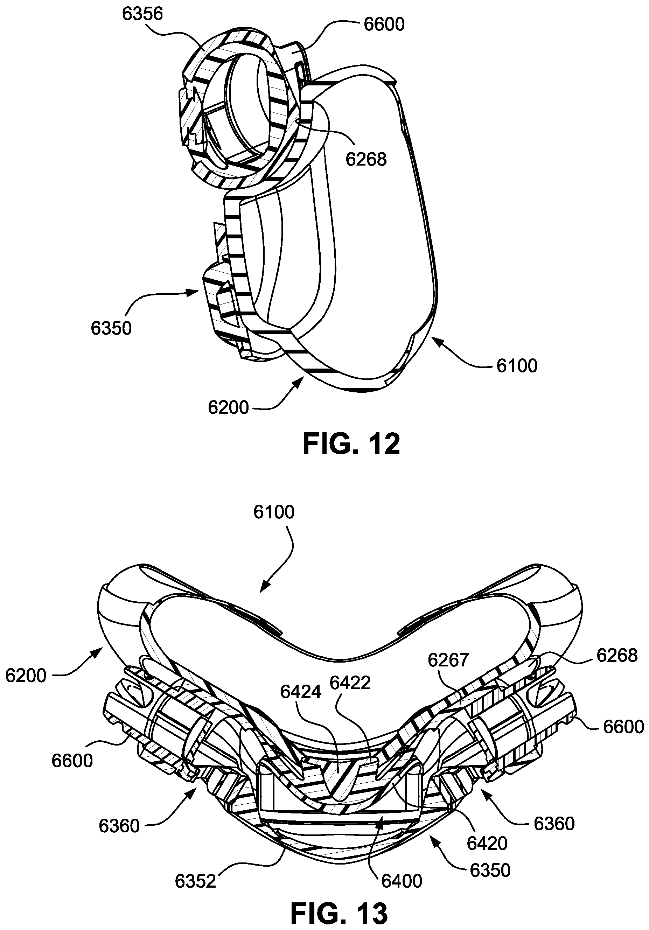

As shown in Figs. 12 and 13, the frame 6350 moves through the opening 6254 and brings the central portion 6352 into engagement with the central portion 6267 of the groove 6266. As described previously, the central portion 6352 of the frame 6350 and the central portion 6267 of the groove 6266 may include opposite curvatures, which may prevent the central portion 6352 of the frame 6350 from completely contacting the central portion 6367 of the groove 6366. However, edges around the central portion 6367 of the groove 6366 may correspond to edges of the central portion 6352 of the frame 6350, which may allow at least a portion of the frame 6350 to lie in contact with the groove 6366.

In certain forms, the mechanical engagement creates a sealing engagement so that airflow may be limited from passing through the opening 6254, but not through the posterior aperture 6355. In this case, a sealing engagement between the edges of the central portion 6352 of the frame 6350 and the central portion 6267 of the groove 6366 may not be necessary, although the interface may include a sealing arrangement (e.g., as a secondary seal).

Moving the frame 6350 through the opening 6254 also causes the arms 6356 of the frame 6350 to move toward the plenum chamber 6200, and specifically to move into engagement with the respective side portions 6268 of the groove 6266. In some forms, the similar size and shape of the arms 6356 and the side portions 6268 may cause the arms 6356 to be press fit, friction fit, and/or snap fit into the respective side portion 6268.

In some forms, the connection ports 6600 may be spaced apart from the plenum chamber 6200 when the arms 6356 are connected to the side portions 6268. As illustrated in Figs. 9 and 10, the connection ports 6600 may extend laterally beyond the side portions 6268 of the groove 6266. Additionally, the connection ports 6600 may extend mostly in the lateral direction, while the plenum chamber 6200 may curve in the posterior direction, thereby creating a gap between the connection ports 6600 and the plenum chamber 6200. These gaps may permit easier engagement between the connection ports 6600 and fluid conduits 6320.

In some forms, the wings 6364 may be similarly spaced from the surface of the plenum chamber 6200. The cantilevered structure of the wings 6364 may allow for flexion in the anterior-posterior direction (e.g., because of a force applied by headgear).

In other forms, the plenum chamber 6200 may be overmolded onto the frame 6350, and the two pieces may not be separable.

### 5.3.3.1.1.2 Two point connection

### 5.3.3.1.1.2.1 Nasal-only patient interface

As shown in Figs. 19 to 25, a frame 7350 may be coupled to the plenum chamber 7200 of the nasal-only patient interface 7000. The frame 7350 may be similar to the frame 6350

The frame 7350 may include a central portion 7352 that has a similar shape to the plenum chamber 7200. For example, the central portion 7352 may have a substantially elliptical shape, which may correspond to the shape and orientation of the opening 7254 of the central portion 7352.

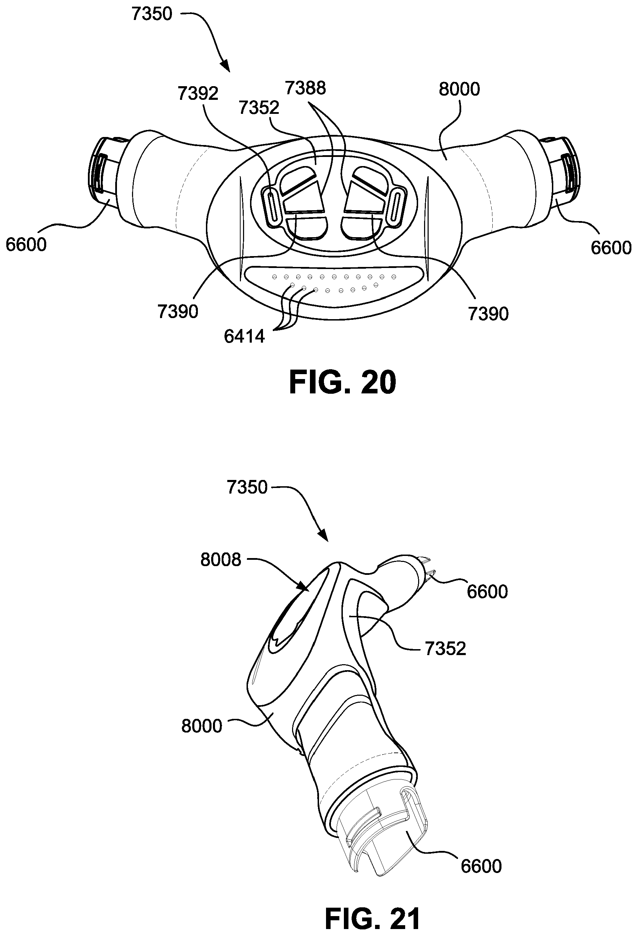

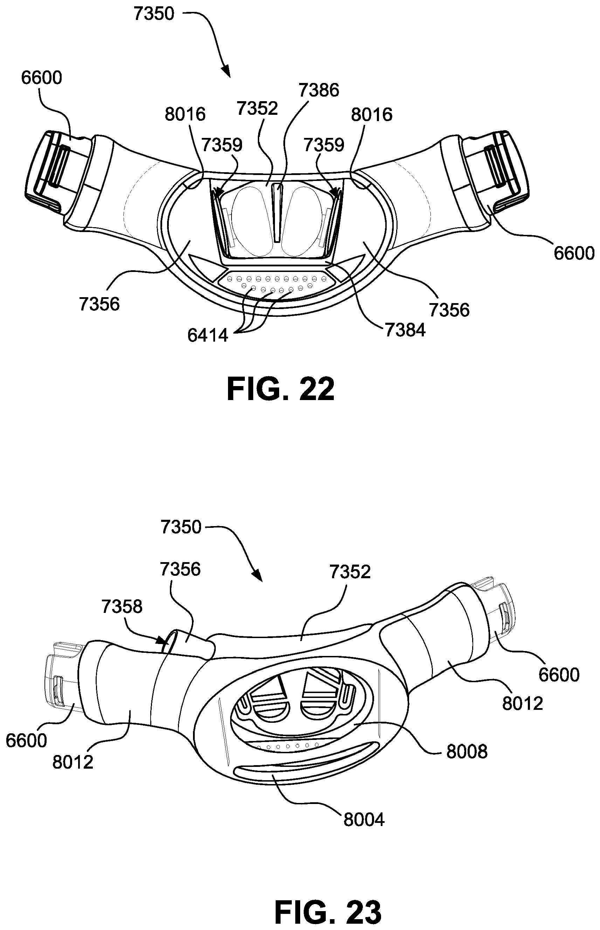

As shown in Figs. 20 and 22, some forms of an anterior side of the central portion 7352 may include a negatively domed curvature. Additionally, the posterior side of the central portion 7352 may include a positively domed curvature.

As shown in Fig. 21, certain forms of the radius of curvature of the anterior side of the central portion 7352 may be different from the radius of curvature of the posterior side of the central portion 7352. The different radii of curvature may form a varying thickness between the edge and middle of the central portion 7352 (e.g., thickness is greater proximate the edges of the central portion 7352). For example, the posterior side of the central portion 7352 may include a smaller radius of curvature, which may form a deeper pocket of space on the posterior side of the frame 7350.

In some forms, at least one side portion or arm 7356 extends laterally from the central portion 7352 of the frame 7350. In the illustrated example, the frame 7350 includes a pair of arms 7356, one on either lateral side (i.e., left hand and right hand sides) of the frame 7350.

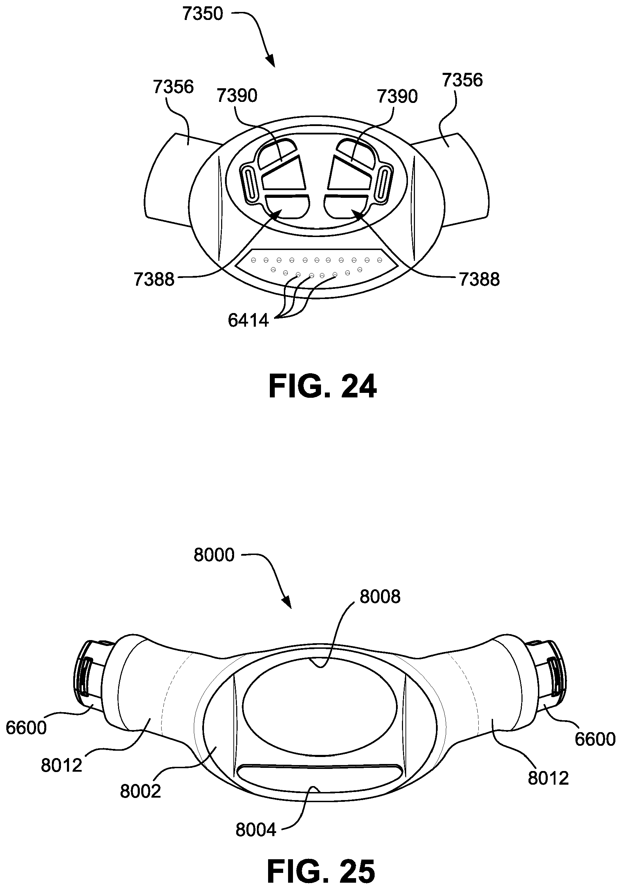

As shown in Figs. 23 and 24, some forms of the arms 7356 may be integrally formed with the central portion 7352. There may be a stepped transition between the arms 7356 and the central portion 7352. As shown in Figs. 22-24, each arm 7356 is narrower than the central portion 7352, and may be flush with neither the anterior nor the posterior sides of the central portion 7352. In other forms, there may be a smooth transition (e.g., no sharp corners) between the central portion 6352 and each of the arms 6356., and/or the arms 6356 and the central portion 6352 may be connected but not integrally formed (e.g., constructed from different materials).

As shown in Fig. 23, some forms of the end of each arm 7356 may include a connection opening 7358. The connection opening 7358 may include a circular (e.g., elliptical) cross section, and may be adapted to connect with a connection port 6600. The connection opening 7358 may also include a rounded-rectangular cross-section, or any other similar shape.

As shown in Fig. 22, each arm 7356 may extend into the volume of the central portion 7352. For example, each arm 7356 may extend on either side of an outer edge of the central portion 7352. The stepped interface described above, may at least partially recess each arm 7356 relative to the posterior side of the central portion 7352.

In some forms, each arm 7356 may extend toward a middle of the central portion 7352 so that outlets 7359 of the arms 7356 (e.g., opposite the connection openings 7358) are disposed proximate to one another, and may be oriented in a facing relationship with respect to each other.

In some forms, a dividing wall 7384 may be disposed on an interior surface of the central portion 7352 of the frame 7350. The dividing wall 7384 may extend between the outlets 7359 of each of the arms 7356. For example, the dividing wall 7384 may extend between inferior edges of each of the arms 7356. In this position, the dividing wall 7384 may not obstruct the opening of the outlet 7359.

In certain forms, the dividing wall 7384 may be a substantially planar surface, and may be substantially perpendicular to the patient's sagittal plane while the patient interface 7000 is in use. The dividing wall 7384 may also not extend substantially more posterior or inferior than the arms 7356.

In some forms, a deflecting wall 7386 may be disposed on an interior surface of the central portion 7352 of the frame 7350. The deflecting wall 7386 may be spaced apart (e.g., evenly spaced) from the outlet 7359 on either arm 7356.

In certain forms, the deflecting wall 7386 may include a substantially vertical orientation, and may extend in the posterior direction. For example, the deflecting wall 7386 may at least partially intersect an axis extending through either of the arms 7356 (e.g., an axis extending from the connection opening 7358 to the outlet 7359). The deflecting wall 7386 may also be oriented substantially perpendicularly with respect to the dividing wall 7384.

In certain forms, the deflecting wall 7386 may include a triangular shape, and may be wider (e.g., extend more posterior) proximate an upper edge as compared to the lower edge. The upper end of the deflecting wall 7386 may be disposed proximate to the upper edge of the central portion 7352, as well as substantially coplanar with the upper edge of each of the outlets 7359. The upper end of the deflecting wall 7386 may also extend proximate to the posterior edge of each of the outlets 7359.

In some forms, the central portion 7352 includes vent openings 6414 (described below), which may allow for the output of air through the frame 6350 (e.g., carbon-dioxide washout). The vent openings 6414 may be spaced apart from the outlets 7359 of the arms 7356, and may be disposed inferior to the dividing wall 7384.

As shown in Fig. 19, some forms of the frame 7350 may be permanently coupled to the plenum chamber 7200. For example, the material of the plenum chamber 7200 may be overmolded onto the frame 7350 so that the frame 7350 cannot be separated from the plenum chamber 7200.

In other forms, the frame 7350 may be removable from the plenum chamber 7200. Similar to the frame 6350, the frame 7350 may mechanically engage the plenum chamber 7200 with a press fit, friction fit, or snap fit. The plenum chamber 7200 may include an opening (not shown), which is sized substantially similarly to the central portion 7352 in order to receive the central portion 7352.

In some forms, the arms 7356 may be spaced apart from the plenum chamber 7200 when the frame 7350 is coupled to the plenum chamber 7200. Unlike the plenum chamber 6200, the nasal-only plenum chamber 7200 may not include a groove, meaning there may be no place on the plenum chamber 7200 for receiving the arms 7356.

As shown in Figs. 23 and 25, some forms of a sleeve 8000 may be coupled to the frame 7350, and substantially cover at least a portion of the frame 7350. As shown in Fig. 23, the sleeve 8000 may include a central portion 8002 shaped similarly to the central portion 7352 of the frame 7350. The central portion 8002 of the sleeve 8000 may include a first or vent opening 8004, which may be slightly larger than the total area of the vents 6414, in order to not obstruct the vents 6414 (e.g., airflow exiting the plenum chamber 7200 through the vents 6414).

In some forms, the sleeve 8000 may include sleeve arms 8012, which may be connected to the central portion 8002, and shaped similarly to the arms 7356 of the frame 7350 (e.g., a similar cross-section). Each sleeve arm 8012 may include an opening 8016, which may the respective arm 7356. The sleeve arms 8012 may be longer than the arm 7356, so that the arm 7356 of the frame 7350 does not extend to an end of the sleeve arm 8012.

As shown in Fig. 22, some forms of the connection ports 6600 are connected to ends of each of the sleeve arms 8012, opposite the respective opening 8016. Each of the connection ports 6600 may be permanently connected to the sleeve arms 8012, although in other examples the connection ports 6600 may be removable. In either case, the connection ports 6600 may be removable connected to the frame 7350 via the sleeve 8000.

In some forms, the sleeve 8000 may be constructed from the same material as the plenum chamber 7200 and/or the seal-forming structure 7100. For example, the sleeve 8000 may be constructed from silicone, textile, and/or a similar material. The sleeve 8000 may be coupled to the frame 7350 in order to provide the frame 7350 with a visual and/or tactile appearance similar to the plenum chamber 7200 and/or the seal-forming structure 7100. The removability of the sleeve 8000 allows the patient to clean and/or replace the sleeve 8000.

### 5.3.3.1.1.2.2 Full-face patient interface

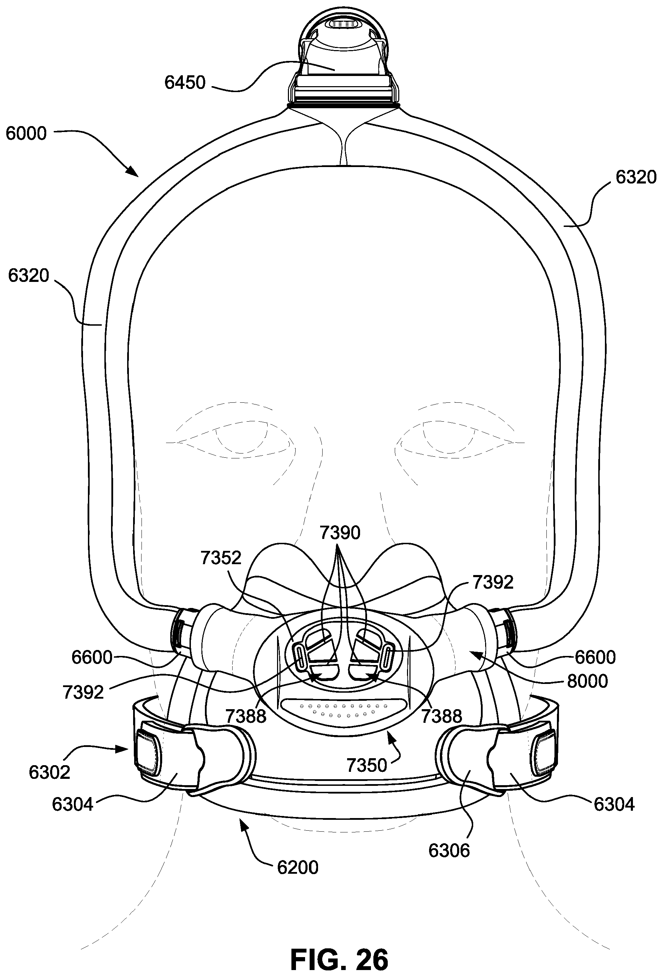

As shown in Fig. 26, the frame 7350 may also be coupled to the plenum chamber 6200 of the full-face patient interface 6000. The frame 7350 coupled to the full-face patient interface 6000 may be substantially the same as the frame 7350 connected to the nasal-only interface, described above. Only some similarities and differences are described below.

In some forms, the central portion 7352 of the frame 7350 may include at least one emergency vent opening 7388. In the illustrated example, the central portion 7352 includes a pair of vent openings 7388, which are spaced apart from one another.

In certain forms, ribs 7390 may be disposed across the vent openings 7388. For example, each emergency vent opening 7388 may include a pair of ribs 7390, each of which may extend entirely across the respective vent opening 7388. The ribs 7390 may be disposed with a variety of orientations.

In certain forms, the central portion 7352 may also include tab openings 7392, which may be disposed proximate to the emergency vent openings 7388. For example, the central portion 7352 may include a pair of tab openings 7392 (e.g., one for each emergency vent opening 7388). Each tab opening 7392 may be disposed adjacent to the respective emergency vent opening 7388. For example, each tab opening 7392 may be disposed between the respective emergency vent opening 7388 and respective arm 7356.

In some forms, the sleeve 8000 may include a second or emergency vent opening 8008, which may be slightly larger than the total area of the emergency vent openings 7388 and the tab openings 7392, in order to not obstruct the emergency vent openings 8008.

### 5.3.3.1.2 Dual opening

### 5.3.3.1.2.1 Narrow frame

As shown in Figs. 27 to 30, the narrow frame 9350 may be used with a narrow plenum chamber 9200. The frame 9350 may be similar to the frames 6350, 7350 described above, and only some similarities and differences are described below.

In some forms, the frame 9350 includes a central portion 9360 that is coupled to the plenum chamber 9200. The central portion 9360 may have a partially triangular annulus shape, and may have a profile that corresponds to the shape of the plenum chamber 9200 (e.g., approximating a positive domed curvature). An opening of the triangular annulus may be smaller than the opening of the annulus shaped frame 6350. In other words, the frame 9350 may contact a larger area of the plenum chamber 9200.

In one form, the central portion 9360 may be removable coupled to the plenum chamber 9200. Removing the frame 9350 may be helpful when cleaning the patient interface 9000, as the different elements of the patient interface 9000 may be cleaned separately, to help ensure a more thorough clean.

In some forms, the frame 9350 further includes at least one connection point 9364, which may assist in indirectly connecting the headgear straps 6304 to the plenum chamber 9200 and/or seal-forming structure 9100.

In certain forms, the frame 9350 includes two connection points 9364. The connection points 9364 may be disposed at an inferior portion of the patient interface 9000 while worn by the patient. The headgear straps may couple to each of the connection points 9364.

In certain forms, the connection points 9364 may be magnetic, and a magnet (e.g., a magnetic member 6306) with an opposite polarity as the connection points 9364 may be used to removably connect headgear straps to the frame 9350.

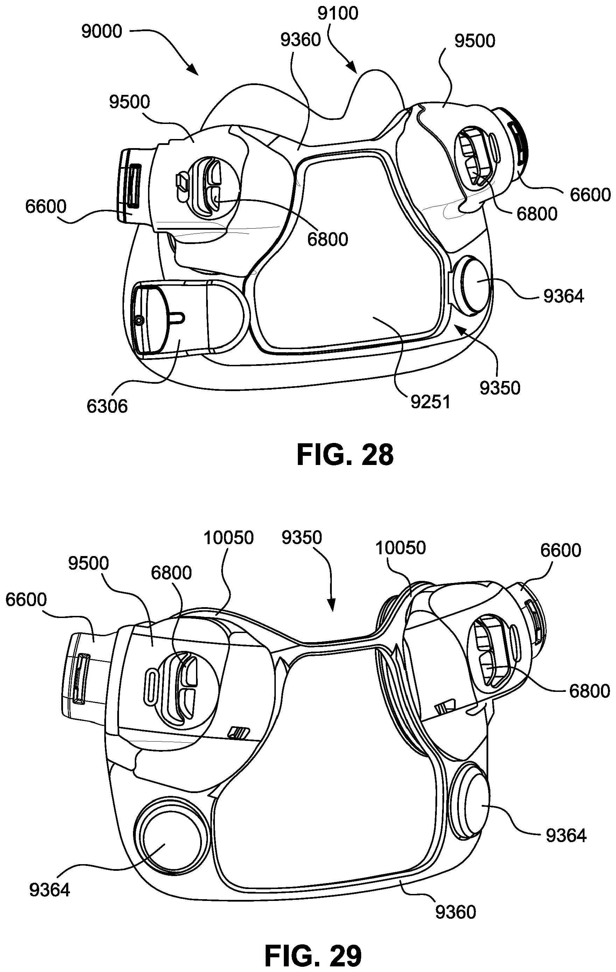

As shown in Fig. 28, the central portion 9360 of the frame 9350 may be positioned within the groove 9260. The shape of the central portion 9360 may substantially correspond to the shape of the groove 9260, which may assist the patient in properly orienting the frame 9350 with respect to the plenum chamber 9200 (e.g., in examples where the frame 9350 is removably coupled to the plenum chamber 9200).

As shown in Fig. 34, the plenum chamber inlet ports 9254 may have a substantially elliptical shape. The plenum chamber inlet ports 9254 may also be disposed toward sides of the central portion 9251 of the plenum chamber 9200. For example, the plenum chamber inlet ports 9254 may be obliquely oriented with respect to the sagittal plane when the patient interface 9000 is worn by the patient. The plenum chamber inlet ports 9254 may also be disposed proximate to the second seal forming structure 9102.

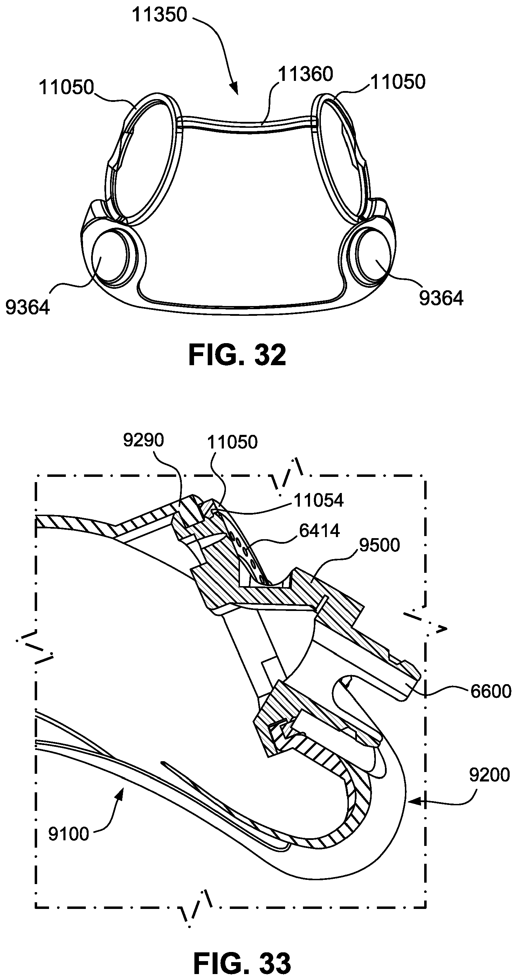

Similar to Fig. 32, some forms of the frame 9350 may include annular portions 10050, which may include an opening having substantially the same size as the plenum chamber inlet ports 9254. The annular portions 10050 may be disposed at a superior region of the frame 9350. For example, the annular portions 10050 may be larger than the plenum chamber inlet ports 9254 in order to allow each plenum chamber inlet port 9254 to be received within the respective annular portion 10050.

In some forms, the width of the groove 9260 may be substantially the same width as the frame 9350, so that the frame 9350 may be positioned within the groove 9260, and the annular portions 10050 may receive the plenum chamber inlet ports 9254.

In certain forms, the frame 9350 may not engage the groove 9260 with a press fit, friction fit, and/or snap-fit. In other words, the frame 9350 may not be securely coupled to the plenum chamber 9200, and/or the frame 9350 may be able to move within the groove 9260 (e.g., slide in the lateral direction and/or in the superior-inferior direction).

As shown in Fig. 29, conduit connection structures 9500 may couple to each of the plenum chamber inlet ports 9254. The conduit connection structures 9500 may have a substantially elliptical opening that substantially corresponds to the size of the plenum chamber inlet ports 9254.

In some forms, each conduit connection structure 9500 may be coupled to the respective plenum chamber inlet port 9254 with a press fit, friction fit, and/or snap-fit, which may prevent accidental disengagement between the conduit connection structures 9500 and the plenum chamber inlet ports 9254. Each conduit connection structure 9500 and plenum chamber inlet port 9254 may also form a sealing engagement in order to substantially limit leaks.

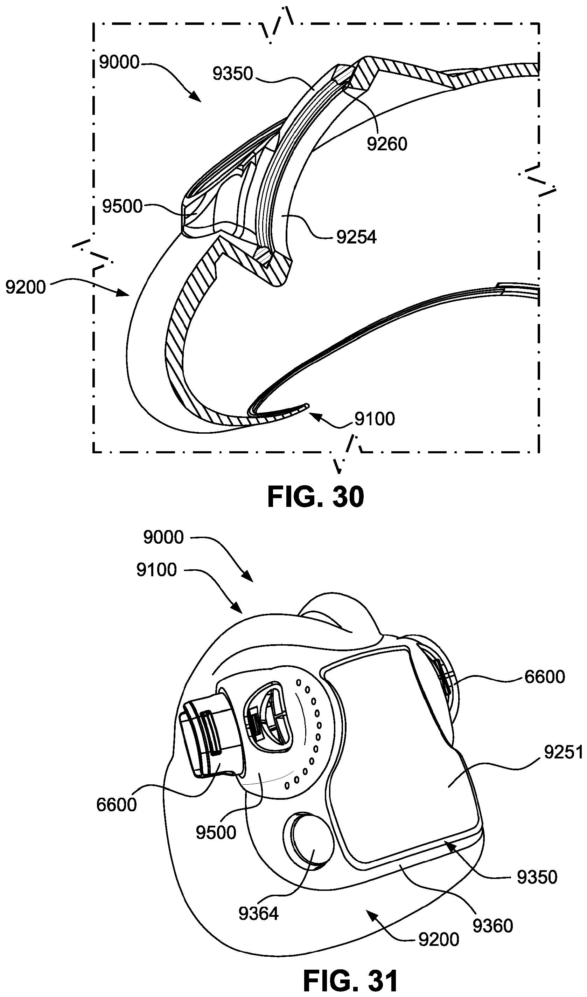

As shown in Fig. 30, some forms of the frame 9350 may be sandwiched between the conduit connection structures 9500 and the plenum chamber inlet ports 9254. The press fit, friction fit, and/or snap-fit may secure the frame 9350 within the groove 9260, and limit the translational ability of the frame 9350 relative to the plenum chamber 9200. This may allow the frame 9350 to provide the structural support to the plenum chamber 9200.

In one form, the conduit connection structures 9500 may be separate elements from the frame 9350. In other words, the frame 9350 may be positioned within the groove 9260, and each conduit connection structure 9500 may separately engage the respective plenum chamber inlet port 9254 in order to sandwich the frame 9350, and press the frame against the surface of the plenum chamber 9200. Having the conduit connection structures 9500 separate from the frame 9350 may promote easier cleaning.

In one form, the conduit connection structures 9500 may be integrally formed with the frame 9350. In other words, the frame 9350 may be positioned within the groove 9260, while the conduit connection structures 9500 are simultaneously aligned with the plenum chamber inlet ports 9254. Having the conduit connection structures 9500 integrally formed with the frame 9350 may promote easier and/or simpler assembly.

In some forms, the force needed to disengage the conduit connection structures 9500 from the plenum chamber inlet ports 9254 may be greater than the typical force applied to the plenum chamber 9200. Therefore, the plenum chamber 9200 may be able to bend and/or flex without causing accidental disengagement between the conduit connection structures 9500 from the plenum chamber inlet ports 9254. Since the frame 9350 is sandwiched between the conduit connection structures 9500 and the plenum chamber inlet ports 9254, bending and/or flexing of the plenum chamber 9200 may not cause accidental disengagement between the frame 9350 and the groove 9260.

In certain forms, the patient may be able to use the outer portions 9261 of the groove 9260 in order to assist in removing the frame 9350 from the plenum chamber 9200 (see e.g., Fig. 34). The conduit connection structures 9500 may contact the outer portions 9261, but may not engage the outer portions 9261. For example, the patient may be able to separate the conduit connection structure 9500 from the groove 9260 by bending the plenum chamber 9200. The patient may then be able to lift the frame 9350 away from the plenum chamber 9200 using the conduit connection structure 9500 for leverage.

### 5.3.3.1.2.2 Wide frame

As shown in Figs. 32 and 33, a frame 11350 may be used to engage a wide variant of the plenum chamber 9200. The frame 11350 may be similar to the frame 9350. Only some similarities and differences between the frame 11350 and the frame 9350 will be discussed below.

In some forms, the central opening of the frame 11350 (e.g., radially within the central portion 11360) may be larger than the comparable opening of the frame 9350. This may be to assist in accommodating the larger surface area of the wide cushion of the plenum chamber 9200 (i.e., because it extends across a wider length of the patient's face).

In some forms, the frame 11350 may include either an indent or a protrusion along an inner surface of the annular portions 11050. In the illustrated example, each annular portion 11050 may include a protrusion 11054 (see e.g., Fig. 33). The projection 11054 (or indent) may engage a complimentary feature 11058 on a conduit connection structure 9500 (described in detail below).

### 5.3.3.2 Conduit clips

As shown in Figs. 38 to 45, the patient interface 12000 may be usable without a frame and may still effectively seal and maintain a therapeutically effective pressure within the plenum chamber 12200. Instead of a frame, clips 14304, 14308 may be connected to the plenum chamber 12200 (e.g., either removably or permanently). As described above, the clips 14304, 14308 may be constructed from a material that is more rigid than the plenum chamber 12200 (e.g., plastic), and may add rigidity to the plenum chamber 12200 in a similar way to any of the frames described above.

As shown in Figs. 42-45, clips 14304 (or clips 14308 in Fig. 42-1) may be connected to the plenum chamber 12200. The clips 14304 include the opening to form the plenum chamber inlet port 12554. The size of each clip 14304 may substantially correspond to the respective opening in the plenum chamber 12200. In other words, a body of the clip 14304 may not extend substantially beyond the perimeter of the plenum chamber inlet port 12554 in the radial direction. The clips 14304 may therefore remain proximate to the nasal portion 12201 of the plenum chamber 12200, and my not extend entirely inferior to the vent opening 13388 in use.

In some forms, each clip 14304 is positioned in a mold prior to the insertion (e.g., injection) of a liquid material, so that the material forming the plenum chamber 12200 flows around and solidifies around the clips 14304. This may keep each of the clips 14304 in place and effectively seals around an outer perimeter of the clips 14304 so that airflow is limited to only pass through the plenum chamber inlet ports 12554.

In some forms, the plenum chamber 12200 is formed separately from the clips 14304, and the clips 14304 may be removably connected to the plenum chamber 12200. Openings in the plenum chamber 12200 may be slightly smaller than an outer perimeter of each clip 14304 so that the clips can be inserted with a press fit (or alternatively a snap fit or a friction fit). The snug engagement between the clips 14304 and the plenum chamber 12200 may create a sealing engagement to limit airflow around the clips 14304.

In either form, the clips 14304 may be located at discrete locations on the plenum chamber 12200. Because the clips 14304 are positioned at discrete locations, the plenum chamber 12200 may bend in more ways. The in use plenum chamber 12200 may be more flexible than the plenum chambers described above that include a frame because there is less rigid material limiting bending or flexing. As shown in Fig. 42, the plenum chamber 12200 may bend about the fold lines 14316. For example, the rigid material of the clips 14304 may direct the folding or movement of the plenum chamber 12220 to along the fold lines 14316 (e.g., because the rigid material of the clips 14304 may limit or prevent fold lines through the plenum chamber inlet ports 12254). The bending or flexing may allow the patient interface 12000 to fit a wide range of patient's faces.

As shown in Fig. 42-1, another form of a clip 14308 may be connected to the plenum chamber 12200. The clips 14308 are illustrated as being removably connected to the plenum chamber 12200, although like the clips 14304, the clips 14308 may be permanently connected to the plenum chamber 12200.

As described above, the clips 14308 may be larger than the clips 14304. The larger surface area of the clips 14308 may offer more support for the plenum chamber 12200 (e.g., as compared to the clips 14304). But like the clips 14304, the larger clips 14308 are disposed only at discrete locations on the plenum chamber 12200 (e.g., and do not extend around a perimeter of the plenum chamber 12200 like a frame would). This may still allow the plenum chamber 12200 to bend along the fold lines 14316 as described above. The inferior portions of the clips 14308 may alter the angle of the of the fold lines 14316 so that they do not substantially intersect the clip 14308. This may be particularly applicable when the clips 14308 are molded into the plenum chamber 12200 and are not removable, as the inferior portion of the clip 14308 would contribute to the rigidity of the plenum chamber 12200 (e.g., and thereby limit bending).

In some forms, the clips 14308 may include an inferior portion constructed as an arm 14304-1 that may be positioned within the groove 12266 when the clips 14308 are connected to the plenum chamber 12200. The arms 14304-1 may extend laterally outward and a free end may include a connection member 12364. Each arm 14304-1 may substantially fill the inferior portion of the respective groove 12266.

### 5.3.3.3 Headgear

As shown in Figs. 7, 26, and 27, some forms of the positioning and stabilizing structure 6300 includes headgear 6302, which may be worn by the patient in order to assist in properly orienting the seal-forming structure 6100 against the patient's face (e.g., in order to limit or prevent leaks).

In some forms, the headgear 6302 may be constructed from a textile material, which may be comfortable against the patient's skin. The textile may be flexible in order to conform to a variety of facial contours. Although the textile may include rigidizers along a selected length, which may limit bending, flexing, and/or stretching of the headgear 6302.

In some forms, the headgear 6302 may include inferior straps 6304, which may connect to an inferior region of the frame 6350. The inferior straps 6304 may extend along the patient's cheek toward a posterior region of the patient's head. For example, the inferior straps 6304 may overlay the Masseter muscle on either side of the patient's face. The inferior straps 6304 may therefore contact the patient's head below the patient's ears. The inferior straps 6304 may meet at the posterior of the patient's head, and may overlay the Occipital bone and/or the Trapezius muscle. The headgear 6302 may also include superior straps (not shown), which may overlay the Temporal bones, Pariental bone, and/or Occipital bone. The superior straps may also extend between the conduits 6320 (described below). A connecting strap (not shown) may extend (e.g., in the superior-inferior direction) between the superior straps and the inferior straps 6304. For example, the connecting strap may overlay the Occipital bone and/or the Pariental bone.

In some forms, the inferior straps 6304 are connected to a magnetic member 6306. For example, each inferior straps 6304 may be threaded through a magnetic member 6306, so that a length of each inferior strap 6304 may be adjusted. The magnetic members 6306 may removably connect to the magnets 6370, so that the inferior straps 6304 may be disconnected from the frame 6350, but the length of the inferior straps 6304 may not be affected.

In some forms (see e.g., Fig. 26), the magnetic members 6306 may be connected directly to the plenum chamber 6200 (via a magnet on the plenum chamber 6200), when the frame 7350 does not include the magnets.

As shown in Figs. 38 and 39, the patient interface 12000 may include headgear 12302 that is similar to the headgear 6302. In the illustrated example, the headgear 12302 may be formed with a substantially X-shape. The headgear 12302 may include inferior straps 12304 and superior straps 12305. The inferior and superior straps 12304, 12305 may be connected to a rear strap 12307. This connection may be done using stitching, ultrasonic welding, or any similar process.

The inferior straps 12304 may extend along the patient's cheek toward a posterior region of the patient's head. For example, the inferior straps 12304 may overlay the Masseter muscle on either side of the patient's face. The inferior straps 12304 may therefore contact the patient's head below the patient's ears.

The superior straps 12305 may extend from a posterior region of the patient's head toward an anterior region, and may be inclined relative to the inferior straps 12304. For example, each superior strap 12305 may be positioned proximate to the Occipital bone on one end and may extend toward the temporal bone at the other end. The superior straps 12305 may be inferior to the patient's ears proximate to the Occipital bone and may be superior to the patient's ears proximate to the temporal bone. The angle of the superior straps 12305 may avoid contact with the patient's ears in order to reduce discomfort caused by the headgear 12302.

The rear strap 12307 may overlay the Occipital bone and/or the Trapezius muscle. The rear strap 12307 may assist in anchoring the headgear 12302 to the patient's head in order to assist in maintaining the position of the seal-forming structure 12100 on the patient's face.

In some forms, the headgear 12302 may be constructed from a flexible material, like a textile and/or a foam. For example, the headgear 12302 may be constructed from a foam encased in a textile. These materials may be considered comfortable materials because they promote patient comfort (e.g., they are not irritating against the patient's skin). Using flexible materials also allows the headgear 12302 to bend and/or flex in order to conform to a wide variety of head sizes and shapes.

In some forms, the headgear 12302 may be stretchable (e.g., may have elastic properties) and may be able to increase in length in order to fit different sized patient's heads. In some forms, the entire headgear 12302 may be elastic, while in other forms, only a portion of the headgear may be elastic.

In some forms, rigidizers or rigid elements may be included in the headgear. For example, rigid elements may be encased by the outer textile material. Alternatively or additionally, rigid textiles may be used (e.g., the headgear 12302 may be constructed with at least partially rigidized threads). Although described as rigid, the rigidizers may still allow some bending or flexing so that the headgear can conform to the shape of the patient's head. Additionally, the rigidized thread may limit extension at various locations on the headgear 12302. This may assist in selectively determining where the headgear can stretch, which may create a better fit and/or increase comfort for the patient.

In some forms, the headgear 12302 may be constructed with elastic material and rigidizers may be positioned at discrete locations in order to control where stretching occurs or control a maximum length of the headgear 12302 when fully stretched. For example, the rigidizers may be positioned along at least a portion of the rear straps 12307 in order to limit elastic expansion along the posterior portion of the patient's head.

As shown in Fig. 42-1, each of the larger clips 14308 may include a connection member 12364 (e.g., on the inferior portion of the clip 14308) to removable connect to respective headgear strap clips 12306. In the illustrated example, the connection member 12364 may be a magnetic member (although other types of connectors like hook and loop material or mechanical fasteners may be used).

In some forms, the connection member 12364 may be disposed on an inferior portion of the clip 14308 (e.g., spaced apart from the plenum chamber inlet port 12254). When the clip 14308 is connected to the plenum chamber 12200, the connection member 12364 may be positioned within the groove 12266 (e.g., via the arm 14304-1). The inferior portion of the clip 14308 may add some rigidity to the plenum chamber 12200 when situated within the groove 12266. The groove 12266 may also help to maintain the position of the connection member 12364 when a force is applied (e.g., by the headgear 13302).

In some forms, the connection members 12364 may be formed as one piece with the reminder of the clip 14308. For example, a superior portion of the clip 14308 may include the plenum chamber inlet port 12254, while an inferior portion may include the connection member 12364. Both portions may be positioned within the groove 12266, and may be removable together.

In some forms, the connection members 12364 may be directly to the plenum chamber 12200. For example, the clip 14304 may not extend toward an inferior end of the plenum chamber 12200. The connection members 12364 may be connected in this area (e.g., using an adhesive, via molding, etc.) at approximately the same location as when the connection member 12364 on the larger clip 14308 is used. For example, the inferior portion may be a separately formed arm 14304-1 that is connected to the plenum chamber 12200 independently from the clip 14304. In other words, the smaller clip 14304 may be used with the arm 14304-1 in order to replicate the larger clip 14308. In this form, the clip 14304 may be removed from the plenum chamber 12200 and the arm 14304-1 may remain in place. In still other forms, the arms 14304-1 may also be removable from the plenum chamber 12200 as a separate piece from the clips 14304.

In some forms, a magnetic member 12306 may include a crossbar 12308 that may removably receive a strap of the headgear 12302. For example, the inferior straps 12304 may be folded around the crossbar 12308 and connected to themselves with hook and loop material. This may allow the patient to selectively adjust the length of the inferior straps 12304. The magnetic members 12306 may then be removably connected to the respective connection member 12364. This may allow the patient to selectively connect or disconnect the magnetic member 12306 from the respective connection member 12364 without altering the folded length of the inferior straps 12304.

When the headgear 12302 is worn by the patient, the inferior straps 12304 provide a tensile force directed in the posterior direction. This tensile force assists in providing a sufficient sealing force to keep the seal-forming structure 12100 (e.g., in particular the first seal forming structure 12101) in an appropriate sealing position in order to substantially limit leaks from occurring. The tensile force from the inferior straps 12304 may also assist in overcoming the gravitational force of the patient interface 12000 (e.g., in particular the gravitational force of the plenum chamber 12200) in order to keep the plenum chamber 12200 and the seal-forming structure 12100 at an appropriate height on the patient's face.

In some forms, conduits 12320 (described below) may include tabs 12324 through which the superior straps 12305 may be threaded through. Like the inferior straps 12304, the superior straps 12305 may be folded back on themselves and held in place using hook and loop material. Once in the connected position, the superior straps 12305 may apply a tensile force directed in the inferior and posterior direction. This tensile force may similarly help to maintain the seal-forming structure 12100 in the sealing position and help to overcome the force of gravity.

### 5.3.3.4 Conduits

Conduits, like headgear straps, may provide a force that contributes to the positioning and stabilizing force F_{PSS}. For example, each conduit may provide a force F_{conduit} directed in the posterior and respective lateral direction in order to hold the seal-forming structure 3100 against the patient's face (into the upper lip and sealing under the nose) and oppose the effect of the positive pressure in the plenum chamber 3200 to lift off the face (i.e., Fₚₗₑₙᵤₘ). The force F_{conduit} directed may also be directed at least partially in the superior direction in order to overcome the gravitational force F_{g}.

In some forms, the conduits may provide a force directed into the patient's head when the conduits are filled with pressurized air. The force may assist in gripping the patient's head. The force may be caused by the inflation of the conduits during normal use. In some forms, the force may provide a cushioning effect to the patient's head. The conduits may be designed in order to limit expansion in order to prevent over-gripping the patient's head.

The position of the patient's head may also change the gripping force of the conduits. For example, if the patient is sleeping on his side, the weight of the patient's head may compress one conduit, and the other conduit (e.g., the lateral portion not between the patient's head and a sleeping surface, like a pillow) may additionally expand in order to keep substantially the same flow rate of pressurized air.

As shown in Figs. 7, 19, 26, and 27, some forms of the patient interface 6000 may include a tube or conduit 6320 may be coupled (e.g., removably coupled or permeantly coupled) to each conduit connection structure 9500. Each conduit 6320 may convey a flow of pressurized breathable gas (e.g., from the RPT device 4000) toward the patient's airways. The flow of pressurized breathable gas may enter the plenum chamber 13200 through the conduit connection structures 9500 and the plenum chamber inlet ports 13610. The sealing engagement between each conduit connection structure 9500 and plenum chamber inlet port 13610 may limit the flow of pressurized breathable gas from leaking into the ambient through that interface.

In use, the conduits 6320 may extend along the patient's head (e.g., along the patient's cheeks and toward the superior region of the patient's head). The conduits 6320 may take the place of the superior headgear straps of the patient interface 6000. As such, the conduits 6320 may be constructed from a flexible or semi-rigid material (e.g., silicone, textile, etc.), and may be able to flex as the patient dons the patient interface 13000. The length of the conduits 6320 may not be adjustable, and all adjustment may be from the inferior straps.

In some forms, the conduits 6320 may compliment the headgear 6302, and provide additional positioning and/or stabilizing to the seal-forming structure 6100. Straps of the headgear 6302 may not extend the same location of the patient's head as the conduits 6320 (e.g., textile straps may not extend in a substantially superior direction along the patient's cheeks toward a superior region of the patient's head). Instead, the conduits 6320 may provide the positioning force to pull the seal-forming structure 6100 against the patient's face.

As shown in Figs. 38 to 41, the conduits 12320 may connect to the plenum chamber 12200 via the clips 14304, 14308. The conduits 12320 may provide the flow of pressurized breathable gas directly into the plenum chamber 12200 through the plenum chamber inlet ports 12254.

In some forms, the conduits 12320 may be permanently connected to the clips. The conduits 12320 including the arms 14304-1 may be connected to the clips 14304. The conduits 12320 that are separate from the arms 14304-1 may be used with the larger clips 14308 (e.g., which includes the arms 14304-1). The conduits 12320 may be disconnected from the plenum chamber 12200 by removing the clips 14304, 14308 from the respective groove 12266. Forming the clip 14308 and the conduit 12320 as one piece may make disassembling and reassembling the patient interface 12000 easier and/or may reduce the occurrence of missing parts.

In some forms, the conduits 12320 may be removably connected to the clips. This may be the case when the conduits 12320 are used with the clips 14304 that are integrally formed with the plenum chamber 12200 (although both the clips 14304 and the conduits 12320 may be removably connected to the plenum chamber 12200). The conduits 12320 may be disconnected from the clips 14304 so that the conduits 12320 can be cleaned and/or to assist the patient in removing the plenum chamber 12200.

As described above, the groove 12266 may be larger than the clip 14304. This may assist in accommodating both the conduit 12320 and the arm 14304-1. For example, the conduit 12320 may be radially larger than the plenum chamber inlet port 12254. The wider portion of the groove 12266 may allow the conduit to fit within the groove 12266 during connection. For example, the conduit 12320 may be mechanically connected to the clip 14304 (e.g., via a snap-fit, press fit, friction fit, etc.) as well as to the groove 12266 (e.g., via a snap-fit, press fit, friction fit, etc.). In addition, the inferior portion of the groove 12266 may be large enough to receive the arm 14304-1 as described above.

In forms where the conduits 12320 are removable from the clips 14304, the clips 14304 may remain connected to the plenum chamber 12200 after the conduits 12320 have been removed. The clips 14304 may be separately removable from the plenum chamber 12200 (e.g., to facilitate cleaning), or the clips 14304 may be permanently connected to the plenum chamber (e.g., via an adhesive).

In some forms, the conduits 12320 may act like the straps of the headgear 12302 and apply a force to the plenum chamber 12200 (through the clips 14304, 14308). Like the inferior straps 12304 assist in providing a force to the first seal forming structure 12101 because the connection member 12364 is disposed at an inferior portion of the plenum chamber 12200, the conduits 12320 may assist in providing a force to the second seal forming structure 12102 because the clips 14304, 14308 are located at a superior portion of the plenum chamber 12200. The conduits 12320 may provide a tensile force directed in the superior and posterior directions, and may also help to counteract the gravitational force of the plenum chamber 12200.

As shown in Fig. 39, the arms 14304-1 may be formed as one piece with the conduits 12320. For example, the arms 14304-1 may be removable from the plenum chamber 12200 with the conduits 12320. As described above, each arm 14304-1 may be positioned within the groove 12266 when connected to the plenum chamber 12200. When the conduits 12320 are disconnected from the clip 14304, the arms 14304-1 may also be removed from the groove 12266. This may allow the entire positioning and stabilizing structure 12300 to be removed from the plenum chamber 12200 at substantially the same time.

In this form, each conduit 12320 is connected to a clip 14304, as the larger clip 14308 is not needed with the arm 14304-1 connected to the conduits 12320.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO2 by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

As shown in Figs. 7 to 17, a vent 6400 may be a module coupled to the frame 6350. Specifically, the vent 6400 may be positioned within the aperture 6354 in a sealing arrangement in order to limit the flow of air around the vent 6400.

In some forms, the vent 6400 may be constructed from a rigid or semi-rigid material. For example, the vent 6400 may be constructed from a plastic material (e.g., Apec 1745 and/or Makrolon 2458), and/or any similar material.

As shown in Fig. 17, the vent 6400 may include an inferior portion or vent body 6404 and a superior portion or vent arm 6408. The vent body 6404 and the vent arm 6408 may be integrally formed with one another.

In some forms, the vent body 6404 may include a round (e.g., elliptical) shape that may substantially correspond to the shape of the aperture 6354 of the frame 6350.

In some forms, the vent body 6404 may include an anterior rim 6410, a posterior rim 6411, and a recessed groove 6412 disposed between the anterior and posterior rims 6410, 6411. The anterior rim 6410 may include a shape that substantially corresponds to the anterior portion of the aperture 6354, and the posterior rim 6411 may substantially correspond to the posterior portion of the aperture 6354.

In some forms, the vent body 6404 may include a recessed surface 6413, which may be recessed in the posterior direction with respect to the anterior rim 6410. The recessed surface 6413 may be substantially aligned with the posterior rim 6411. Vent holes 6414 may be positioned on the recessed surface 6413, and may allow fluid communication through the recessed surface 6413.

In certain forms, retaining tabs 6416 may be disposed between the anterior rim 6410 and the recessed surface 6413 of the vent body 6404. The retaining tabs 6416 may be spaced apart along an inner perimeter of the vent body 6404.

In some forms, the vent arm 6408 may extend from the posterior portion of the vent body 6404 (e.g., proximate to the posterior rim 6411) in a superior direction. The vent arm 6408 may be formed with a substantially U-shape.

In some forms, the vent arm 6408 may include an anterior lip 6420, a posterior lip 6422, and a recessed groove 6424 disposed between the anterior and posterior lips 6420, 6422. The anterior lip 6420 may include a shape that substantially corresponds to an anterior portion of the posterior aperture 6355, and the posterior lip 6422 may substantially correspond to the posterior portion of the posterior aperture 6355.

In certain forms, the U-shaped vent arm 6408 may partially form an opening 6418. For example, an inner perimeter of the anterior lip 6420 and a surface of the vent body 6404 may form the perimeter of the opening 6418.

In some forms, the vent 6400 may be coupled to the frame 6350 using a mechanical connection (see e.g., Figs. 14 and 15). This may be a snap-fit, or any similar connection like a press-fit or friction-fit. In other examples, the vent 6400 may be permanently formed with the frame 6350.

To couple the removable vent 6400 to the frame 6350, the vent arm 6408 may be positioned so that they extend through the aperture 6354 of the central portion 6352 of the frame 6350 (see e.g., Fig. 11). In other words, the vent 6400 may be oriented so that the vent arm 6408 extends along a horizontal plane. In this orientation, the vent arm 6408 may be more posterior, but not more superior, than the vent body 6404. In this orientation, the vent arm 6408 includes a larger horizontal dimension than a vertical dimension, and is able to fit through the aperture 6354 into the volume of the frame 6350.

The vent arm 6408 may be moved through the interior of the frame 6350, and toward the posterior aperture 6355. When the vent body 6404 is positioned proximate to the aperture 6354, the vent 6400 may be pivoted so that the vent arm 6408 is once again more superior than the vent body 6404. Since the vent arm 6408 is disposed proximate to the posterior aperture, the pivoting movement is permitted (e.g., because the U-shaped vent arm 6408 is similarly shaped to the posterior opening 6355).

Pivoting the vent 6400 may also cause the anterior and posterior rims 6410, 6411 of the vent body 6404, and the anterior and posterior lips 6420, 6422 on either side of the respective aperture 6354, 6355. The recessed groove 6412 of the vent body 6404 may contact the inner perimeter of the aperture 6354, and the recessed groove 6424 of the vent arm 6408 may contact an inner perimeter of the posterior aperture 6355. The engagement of the lips to the inner perimeters of the apertures may create the snap-fit used to retain the vent 6400 to the frame 6350.

In forms where the frame 6350 does not include a lip (see e.g., Fig. 15), the vent 6400 may be coupled to the frame 6350 with a friction fit or press fit, instead of with a snap fit.

In this position, the vent 6400 is secured to the frame 6350, and may prevent accidental disengagement (e.g., while the patient is sleeping). However, the patient may be able to remove the vent 6400 from the frame 6350 (e.g., by reversing the steps above) so that the vent 6400 pivots out of engagement with the frame 6350. This may allow the patient to clean the vent 6400 and the frame 6350.

As shown in Fig. 11, the posterior lip 6422 may extend to a posterior side of the posterior aperture 6355, but may not extend substantially into the plenum chamber 6200. For example, the posterior lip 6422 may be substantially flush with the surrounding surface of the plenum chamber 6200, which may reduce or eliminate contact between the patient's face and the vent 6400, while the patient interface 6000 is being worn.

In some forms, an airflow path of the pressurized breathable gas may extend along the arms 6356 of the frame and toward the opening 6418 between the vent body 6404 and the vent arm 6408. Specifically, the pressurized breathable gas may flow toward the middle of the central portion 6352. The airflow paths flow above the vent body 6404, and anterior to the vent arm 6408, at which point the two airflow paths collide. The constant flow of air may limit airflow leaving one arm 6356 from continuing down the other arm 6356. Additionally, an anterior surface of the central portion 6352 limits airflow in the anterior direction away from the patient. Thus, the only path for both streams of air is through the opening 6418, which directs the airflow into the plenum chamber 6200, where it may be inhaled through the patient's mouth and/or nose.

In certain forms, the recessed surface 6413 of the vent body 6404 is also recessed in the anterior direction away from the posterior lip 6422 of the vent arm 6408. Thus, a peripheral surface 6428 of the vent body 6404 may separate the opening 6418 from the vent holes 6414. This may assist in limiting the pressurized breathable gas from immediately exiting the plenum chamber through the vent holes 6414 without being inhaled by the patient. Instead, the peripheral surface 6428 directs the pressurized breathable gas initially away from the vent holes 6414, but the vent holes 6414 are positioned proximate to the patient's mouth, so that exhaled gas may be directed toward the vent holes 6414, and away from the opening 6418.

As shown in Figs. 22, 22, and 24, some forms of the frame 7350 may include vent openings 6414 directly on the surface of the central portion 7352. The vent openings 6414 may be spaced apart from the emergency vent openings 7388. For example, the dividing wall 7384 may separate the vent openings 6414 from the emergency vent openings 7388.

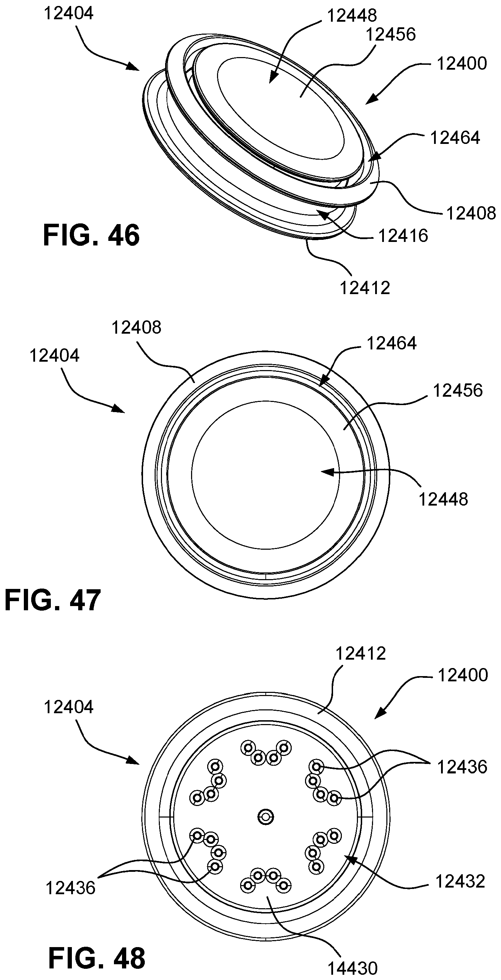

As shown in Figs. 46 to 50, an alternate example of a vent 12400 may be used with the patient interface 12000. The vent 12400 may have a substantially similar shape to the vent opening 13388 (e.g., a substantially circular shape).

In the illustrated examples (e.g., Fig. 38), the vent 12400 may be used with a full-face mask (e.g., an ultra-compact full-face mask) and may be positioned proximate to the patient's mouth (e.g., the vent 12400 may intersect the sagittal place and may be aligned with the patient's mouth). However, in other examples, the vent 12400 may be used with a different type of mask (e.g., a nasal mask like what is illustrated in Figs. 3X or 19)

With continued reference to Figs. 46 to 50, the vent 12400 may include a vent housing 12404, which may be configured to engage with the vent opening 13388. The vent housing 12404 may be constructed from a rigid material or a semi-rigid material. For example, the vent housing 12404 may be constructed from plastic, metal, or any similar material.

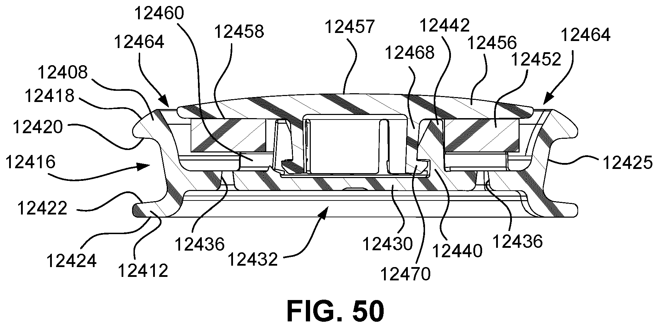

As shown in Fig. 46, the vent housing 12404 may include an anterior surface 12408, a posterior surface 12412, and a groove 12416. The anterior surface 12408 faces away from the patient's face in use, and may be positioned outside the pressurized volume of the plenum chamber 12200. The posterior surface 12412 is disposed opposite to the anterior surface 12408. In use, the posterior surface 12412 may face the patient and may be disposed within the pressurized volume of the plenum chamber 12200. The groove 12416 may be formed between the anterior and posterior surfaces 12408, 12412. For example, the anterior and posterior surfaces 12408, 12412 may have a larger width than at least a portion of the remainder of the vent housing 12404. As shown in Fig. 50, these widths may be approximately the same, although the width of the posterior surface 12412 may be slightly larger. The surface of the groove 12416 has a smaller width so it is recessed from the edges of the anterior and posterior surfaces 12408, 12412.

As shown in Fig. 50, in some forms the anterior surface 12408 may be at least partially inclined. For example, an anterior face 12418 of the anterior surface 12408 may be inclined toward the outer edge. The posterior face 12420 of the anterior surface 12408 (e.g., the face that faces the posterior surface 12412) may be substantially flat (e.g., substantially perpendicular to the sagittal plane in use).

With continued reference to Fig. 50, the anterior face 12422 and the posterior face 12424 of the posterior surface 12412 may be substantially parallel to each other (e.g., not inclined). The faces 12422, 12424 of the posterior surface 12412 may also be substantially parallel to the posterior face 12420 of the anterior surface 12408.

In some forms, a wall 12425 of the vent housing 12404 within the groove 12416 may be angled between the anterior surface 12408 and the posterior surface 12412. For example, the width of the groove 12416 may be larger proximate to the posterior surface 12412.

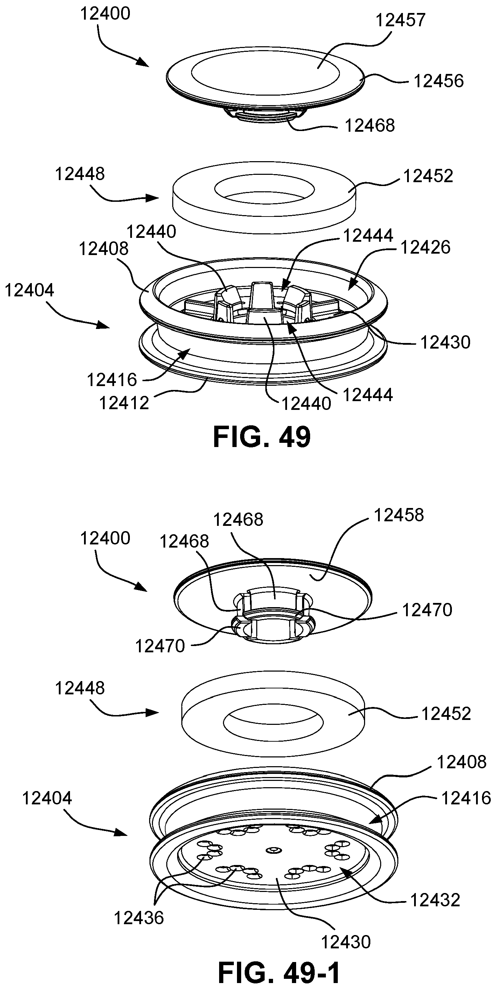

As shown in Fig. 49, the vent housing 12404 may include an opening 12426. For example, the anterior surface 12408 may be ring-shaped and include an inner and an outer width (e.g., an inner and outer diameter). The area within the inner width may be devoid of the anterior surface 12408.

Returning to Fig. 50, the vent housing 12404 may include a recessed surface 12430 within the opening 12426. The recessed surface 12430 may be spaced apart from the anterior surface 12408 in order to create the space for the opening 12426. In the illustrated example, the recessed surface 12430 extends across the inner width.

In some forms, the recessed surface 12430 may also be recessed from the posterior surface 12412. Thus, an additional opening 12432 may be formed in the vent housing 12404. In the illustrated example, the recessed surface 12430 may be disposed closer to the posterior surface 12412, therefore making the opening 12428 larger than the additional opening 12432. Although in other examples, the recessed surface 12430 may be equally spaced between the anterior and posterior surfaces 12408, 12412, or the recessed surface 12430 may be closer to the anterior surface 12408.

With continued reference to Fig. 50, the recessed surface 12430 may include at least one vent hole 12436 (e.g., a plurality of vent holes 12436). The vent holes 12436 may provide fluid communication between the opening 12428 and the additional opening 12430. In other words, fluid may flow through the vent housing 12404 by way of the vent holes 12436.

The plurality of vent holes 12436 may be spread out around the recessed surface 12430. As shown in Fig. 48, the vent holes 12436 may be spaced around the entire perimeter of the recessed surface 12430. This may allow for a more uniform flow around the perimeter of the recessed surface 12430. In other words, fluid can flow substantially evenly through the vent body 12404.

In some forms, the vent holes 12436 may be clustered in groups around the perimeter of the recessed surface 12430. For example, the vent holes 12436 may clustered in various groups of four. In the illustrated example of Fig. 48, the vent holes may be clustered in a U-shape or C-shape, or may form a trapezoidal shape (e.g., a line connecting all of the vent holes 12436 may form a trapezoid). The vent holes 12436 in a given cluster may be closer together than adjacent clusters. Each cluster of vent holes 12436 may be spaced apart from other clusters 12436, so that spaces in between the clusters do not allow airflow to pass through the recessed surface.

In other forms, the clusters may have any number of vent holes 12436 (e.g., 2, 3, 4, 5, etc.) and/or different clusters may have different numbers of vent holes 12436 (e.g., some vent holes 12436 may be clustered in groups of three and adjacent vent holes 12436 may be clustered in groups of four).

In some forms, each cluster of vent holes 12436 may be spaced apart from adjacent clusters of vent holes 12436 by at least 0.1 mm. In some forms, each cluster of vent holes 12436 may be spaced apart from adjacent clusters of vent holes 12436 by at least 0.5 mm. In some forms, each cluster of vent holes 12436 may be spaced apart from adjacent clusters of vent holes 12436 by at least 1 mm. In some forms, each cluster of vent holes 12436 may be spaced apart from adjacent clusters of vent holes 12436 by at least 1.9 mm.

In still other forms, there may be no clusters and the vent holes 12436 may all be evenly spaced around the perimeter of the recessed surface 12430.

In some forms, ends of the vent openings 12436 facing the additional opening 12432 may have a radius of about 0.01 mm to about 10 mm. In some forms, ends of the vent openings 12436 facing the additional opening 12432 may have a radius of about 0.1 mm to about 5 mm. In some forms, ends of the vent openings 12436 facing the additional opening 12432 may have a radius of about 0.25 mm to about 1 mm. In some forms, ends of the vent openings 12436 facing the additional opening 12432 may have a radius of about 0.5 mm.

In some forms, a path of each vent opening 12436 through the recessed surface 12430 may be inclined. In some forms, each vent opening 12436 through the recessed surface 12430 may be about 1° to about 60°. In some forms, each vent opening 12436 through the recessed surface 12430 may be about 5° to about 45°. In some forms, each vent opening 12436 through the recessed surface 12430 may be about 10° to about 20°. In some forms, each vent opening 12436 through the recessed surface 12430 may be about 14°.

In some forms, ends of the vent openings 12436 facing the opening 12426 may have a radius of about 0.01 mm to about 10 mm. In some forms, ends of the vent openings 12436 facing the opening 12426 may have a radius of about 0.1 mm to about 5 mm. In some forms, ends of the vent openings 12436 facing the opening 12426 may have a radius of about 0.25 mm to about 1 mm. In some forms, ends of the vent openings 12436 facing the opening 12426 may have a radius of about 0.425 mm

As shown in Figs. 49 and 50, some forms of the recessed surface 12430 may include a connecting feature 12440. The connecting feature 12440 may be a plurality of prongs that are oriented in a circular shape (although any other shape is possible).

In some forms, the connecting features 12440 may extend from the recessed surface 12430 and into the opening 12428. The connection features 12440 may not extend beyond the anterior surface 12408. In other words, the connecting features 12440 may not extend outside of the opening 12428.

In some forms, the connecting features 12440 may be positioned radially within the anterior surface 12408. For example, the connecting features 12440 may be spaced apart from the wall 12425.

In certain forms, the connecting features 12440 may be disposed proximate to the center of the anterior surface 12408. In other words, the width across the connecting features 12440 may be small, so that each connecting feature 12440 is substantially close to the center of the anterior surface 12408. This may also space the connection features 12440 well apart from the wall 12440.

In some forms, the connecting features 12440 may include a finger 12442 at a free end. The finger 12442 may form an overhang that may be used to retain an additional feature (e.g., with a snap-fit). As shown in Fig. 49-1, each connecting feature 12440 may include a separate finger 12442, although there may be a single, continuous finger 12442 if the connecting feature 12440 forms a continuous perimeter.

As shown in Fig. 49, the connecting features 12440 may be spaced apart from one another. For example, an equally spaced gap 12444 may be disposed between the each adjacent connecting feature 12440 (although the connecting features 12440 may be unequally spaced in other examples). In some forms, the gaps 12444 may serve as water drainage channels. This may allow water or other cleaning fluid to drain out of the center of the vent housing 12404 (e.g., between the connecting features 12440) when the patient cleans the vent housing 12404.

In use, the vent body 12404 may be connected to the plenum chamber 12200 by inserting the vent body 12404 through the vent opening 13388. As described previously, the plenum chamber 12200 may be constructed from a flexible material (e.g., silicone) and may be able to bend and/or stretch. In some forms, the width of the wall 12425 of the vent housing 12404 may be approximately equal to the width of the vent opening 13388. The outer widths of the anterior and posterior surfaces 12408, 12412 may therefore be greater than the width of the vent opening 13388. The patient may stretch, or otherwise manipulate, the plenum chamber 12200 in order to expand the vent opening 13388 so that the posterior surface 12412 may fit through the vent opening 13388. The plenum chamber 12200 may then return to its relaxed position and may contact the wall 12425 of the vent housing 12404. In other examples, the vent body 12404 may be permanently connected to the plenum chamber 12200 (e.g., the flexible material of the plenum chamber 12200 may be molded around the vent body 12404).

As shown in Fig. 50, the plenum chamber 12200 may be received within the space of the groove 12416 in order to retain the vent body 12404 within the vent opening 13388. For example, the plenum chamber 12200 may include a lip 13392 around at least a portion of the perimeter of the vent opening 13388. For example, the lip 13392 may be an inwardly facing lip, and may include a free end facing an interior of the plenum chamber 12200.

In some forms, the lip 13392 may extend between the posterior face 12420 of the anterior surface 12408 and the anterior face 12422 of the posterior surface 12412. The lip 13392 may contact (e.g., be compressed between) the two faces 12420, 12422 in order to assist in retaining the vent body 12404. The lip 13392 may include a similar incline as the wall 12425, and may be substantially flush with the wall 12425 while in engagement.

In some forms, the vent body 12404 may act as a support structure for the plenum chamber 12200 while positioned within the vent opening 13388. As described above, the plenum chamber 12200 may be constructed entirely (or almost entirely) from a flexible material. In other words, the plenum chamber 12200 may not include a frame and may be capable of freely bending in any direction. The rigid or semi-rigid material of the vent body 12404 may provide some rigidity to the plenum chamber 12200 when inserted into the vent opening 13388.

In some forms, the vent body 12404 may limit being along the center line 14312. The vent body 12404 may act as a frame and provide rigidity to a central portion of the plenum chamber 12200. This may allow the plenum chamber 12200 to better fit against the patient's nose because the plenum chamber 12200 is limited from folding along the center line 14312 and pinching the nose.

In some forms, the rigidity, size, and/or shape of the vent body 12404 may direct bending of the plenum chamber 12200 toward along the fold lines 14316. As described above, the fold lines 14316 do not intersect the vent opening 13388. Bending along either fold line 14316 may not substantially affect the position of the lip 13392 relative to the vent body 12404 (e.g., the lip 13392 may remain substantially flush with the wall 12425). Movement of the plenum chamber 12200 along the fold lines 14316 therefore may not substantially affect a seal formed between the lip 13392 and the vent body 12404.

To remove the vent body 12404, the patient may bend and/or flex the plenum chamber 12200 in order to expand the vent opening 13388 and move the lip 13392 out of engagement with the anterior and posterior surfaces 12408, 12412.

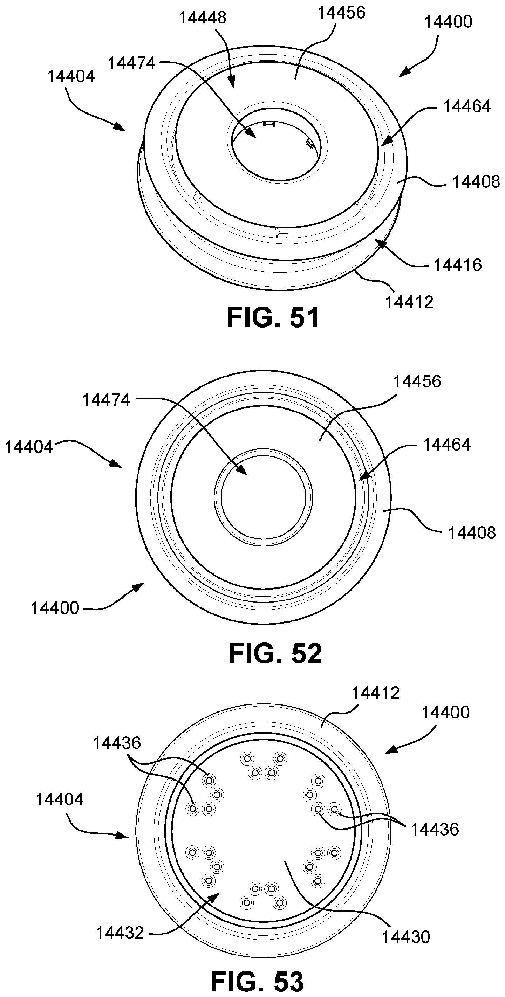

Figs. 51 to 55 illustrate an alternate example of a vent 14400, which may be similar to the vent 12400. Similar features may be labelled with similar reference numbers plus "2000". Only some similarities and differences are described below. The vent 14400 may have a substantially similar shape to the vent opening 13388 (e.g., a substantially circular shape).

With continued reference to Figs. 51 to 55, the vent 14400 may include a vent housing 14404, which may be configured to engage with the vent opening 13388. The vent housing 14404 may be constructed from a rigid material or a semi-rigid material. For example, the vent housing 14404 may be constructed from plastic, metal, or any similar material.

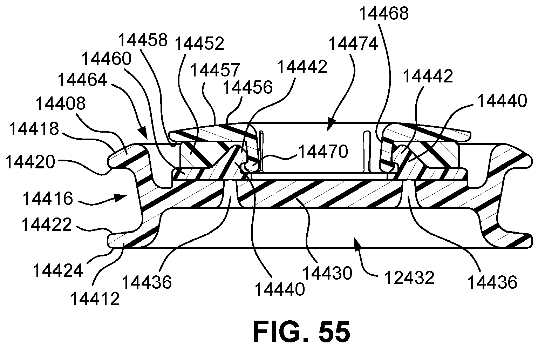

As shown in Fig. 51, the vent housing 14404 may include an anterior surface 14408, a posterior surface 14412, and a groove 14416. The anterior surface 14408 faces away from the patient's face in use, and may be positioned outside the pressurized volume of the plenum chamber 12200. The posterior surface 14412 is disposed opposite to the anterior surface 14408. In use, the posterior surface 14412 may face the patient and may be disposed within the pressurized volume of the plenum chamber 12200. The groove 14416 may be formed between the anterior and posterior surfaces 14408, 14412. For example, the anterior and posterior surfaces 14408, 14412 may have a larger width than at least a portion of the remainder of the vent housing 14404. As shown in Fig. 55, these widths may be approximately the same, although the width of the posterior surface 14412 may be slightly larger. The surface of the groove 14416 has a smaller width so it is recessed from the edges of the anterior and posterior surfaces 14408, 14412.

As shown in Fig. 55, in some forms the anterior surface 14408 may be at least partially inclined. For example, an anterior face 14418 of the anterior surface 12408 may be inclined toward the outer edge. The posterior face 14420 of the anterior surface 14408 (e.g., the face that faces the posterior surface 14412) may be substantially flat (e.g., substantially perpendicular to the sagittal plane in use).

With continued reference to Fig. 55, the anterior face 14422 and the posterior face 14424 of the posterior surface 14412 may be substantially parallel to each other (e.g., not inclined). The faces 14422, 14424 of the posterior surface 14412 may also be substantially parallel to the posterior face 14420 of the anterior surface 14408.

In some forms, a wall 14425 of the vent housing 14404 within the groove 14416 may be angled between the anterior surface 14408 and the posterior surface 14412. For example, the width of the groove 14416 may be larger proximate to the posterior surface 14412.

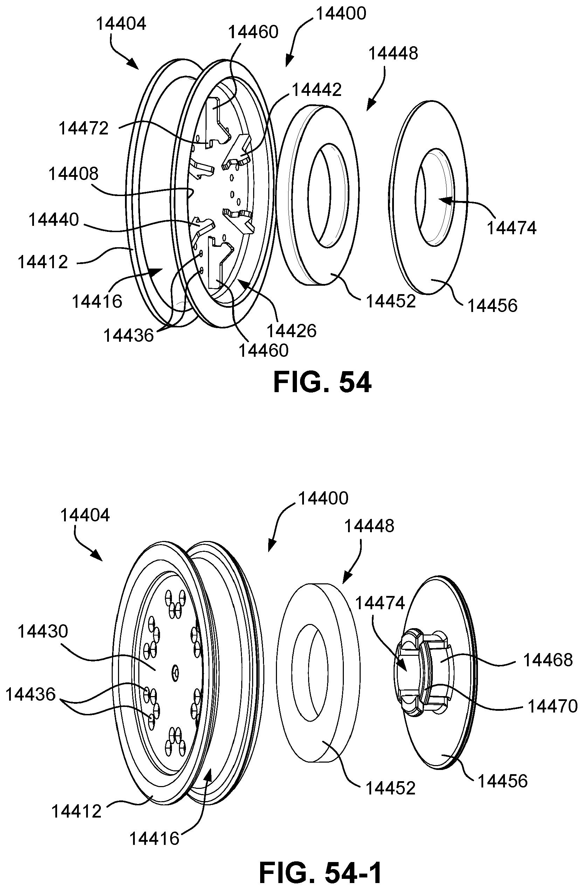

As shown in Fig. 54, the vent housing 14404 may include an opening 14426. For example, the anterior surface 14408 may be ring-shaped and include an inner and an outer width (e.g., an inner and outer diameter). The area within the inner width may be devoid of the anterior surface 14408.

Returning to Fig. 55, the vent housing 14404 may include a recessed surface 12430 within the opening 14426. The recessed surface 14430 may be spaced apart from the anterior surface 14408 in order to create the space for the opening 14426. In the illustrated example, the recessed surface 14430 extends across the inner width.

In some forms, the recessed surface 14430 may also be recessed from the posterior surface 14412. Thus, an additional opening 14432 may be formed in the vent housing 14404. In the illustrated example, the recessed surface 14430 may be disposed closer to the posterior surface 14412, therefore making the opening 14428 larger than the additional opening 14432. Although in other examples, the recessed surface 14430 may be equally spaced between the anterior and posterior surfaces 14408, 14412, or the recessed surface 14430 may be closer to the anterior surface 14408.

With continued reference to Fig. 55, the recessed surface 14430 may include at least one vent hole 14436 (e.g., a plurality of vent holes 14436). The vent holes 14436 may provide fluid communication between the opening 14428 and the additional opening 14430. In other words, fluid may flow through the vent housing 14404 by way of the vent holes 14436.

The plurality of vent holes 14436 may be spread out around the recessed surface 14430. As shown in Fig. 48, the vent holes 14436 may be spaced around the entire perimeter of the recessed surface 14430. This may allow for a more uniform flow around the perimeter of the recessed surface 14430. In other words, fluid can flow substantially evenly through the vent body 14404.

In some forms, the vent holes 14436 may be clustered in groups around the perimeter of the recessed surface 14430. For example, the vent holes 14436 may clustered in various groups of four. In the illustrated example of Fig. 53, the vent holes may be clustered in substantially the same shape as the vent holes 12436 of Fig. 48.

In other forms, the clusters may have any number of vent holes 14436 (e.g., 2, 3, 4, 5, etc.) and/or different clusters may have different numbers of vent holes 14436 (e.g., some vent holes 14436 may be clustered in groups of three and adjacent vent holes 14436 may be clustered in groups of four).

As shown in Figs. 54 and 55, some forms of the recessed surface 14430 may include a connecting feature 14440. The connecting feature 14440 may be a plurality of prongs that are oriented in a circular shape (although any other shape is possible).

In some forms, the connection features 14440 may extend from the recessed surface 14430 and into the opening 14428. The connection features 14440 may not extend beyond the anterior surface 14408. In other words, the connecting features 14440 may not extend outside of the opening 14428.

In some forms, the connecting features 14440 may include a finger 14442 at a free end. The finger 14442 may form an overhang that may be used to retain an additional feature (e.g., with a snap-fit). As shown in Fig. 54, each connecting feature 14440 may include a separate finger 14442, although there may be a single, continuous finger 14442 if the connecting feature 14440 forms a continuous perimeter.

With continued reference to Fig. 54, the connecting features 14440 may be spaced apart from one another. For example, an equally spaced gap 14444 may be disposed between the each adjacent connecting feature 14440 (although the connecting features 14440 may be unequally spaced in other examples). In some forms, the gaps 14444 may serve as water drainage channels. This may allow water or other cleaning fluid to drain out of the center of the vent housing 14404 (e.g., between the connecting features 14440) when the patient cleans the vent housing 14404.

In some forms, the connecting features 14440 may be more spaced apart than the connecting features 12440. For example, the vent body 14404 may have the same number of connecting features 14440 as the vent body 12404. However, the distance between adjacent connecting features 14440 may be larger than the distance between adjacent connecting features 12440. An area formed between the connecting features 14440 may also be larger than the area formed between the connecting features 12440 (e.g., as a result of the increased space between the connecting features 14440).

In some forms, a rib 14460 may be disposed on the recessed surface 14430 and integrally formed with each connecting feature 14440. Each rib 14460 may protrude into the opening 14426. Each rib 14460 may also extend in radially outside of the respective connecting feature 14440. In the illustrated form, the ribs 14460 are spaced apart from a wall of the vent body 14404 that forms the opening 14426. Each rib 14460 may have a substantially rectangular cross section.

In some forms, each connecting feature 14440 may be inclined relative to the respective rib 14460. In some forms, each connecting feature 14440 may extend about 1° to about 90° relative to the respective rib 14460. In some forms, each connecting feature 14440 may extend about 10° to about 75° relative to the respective rib 14460. In some forms, each connecting feature 14440 may extend about 25° to about 50° relative to the respective rib 14460. In some forms, each connecting feature 14440 may extend about 45° relative to the respective rib 14460.

In use, the vent body 14404 may be connected to the plenum chamber 12200 in substantially the same way as the vent body 12404 is connected to the plenum chamber 12200. The patient may stretch, or otherwise manipulate, the plenum chamber 12200 in order to expand the vent opening 13388 so that the posterior surface 14412 may fit through the vent opening 13388. The plenum chamber 12200 may then return to its relaxed position and may contact the wall 14425 of the vent housing 14404. Additionally, the lip 13392 of the plenum chamber 12200 may be used to retain the vent body 14404 in position.

Because the vent body 14404 is substantially the same size as the vent body 12404, the vent 14400 may similarly limit bending along the center line 14312 of the plenum chamber 12200, and may direct bending toward the fold lines 14316.

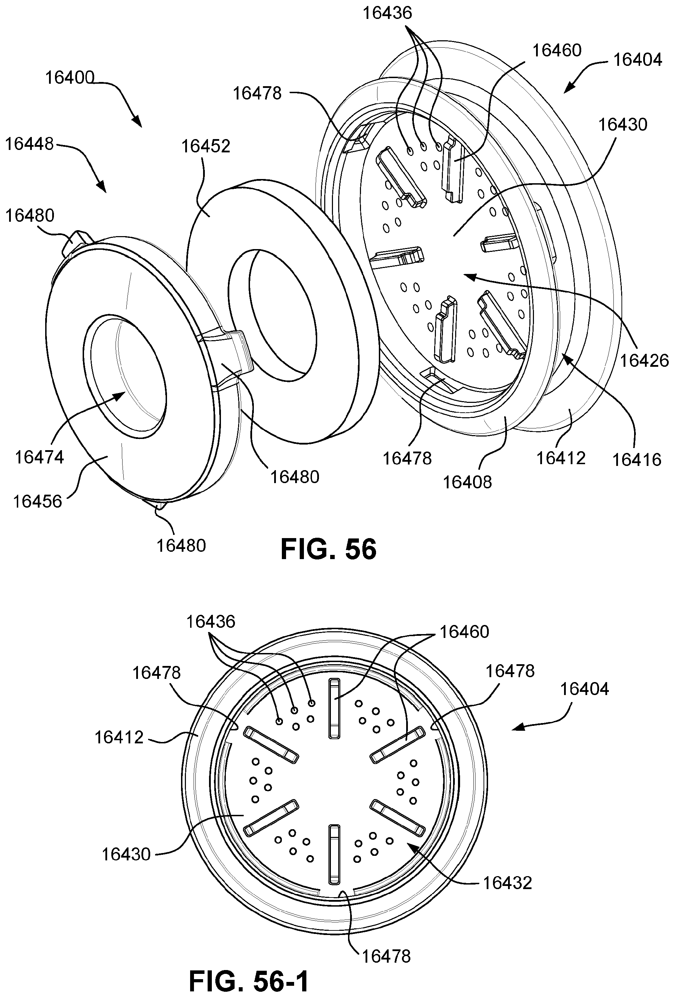

Figs. 56 to 56-3 illustrate an alternate example of a vent 16400, which may be similar to the vent 14400. Similar features may be labelled with similar reference numbers plus "2000". Only some similarities and differences are described below. The vent 16400 may have a substantially similar shape to the vent opening 13388 (e.g., a substantially circular shape).

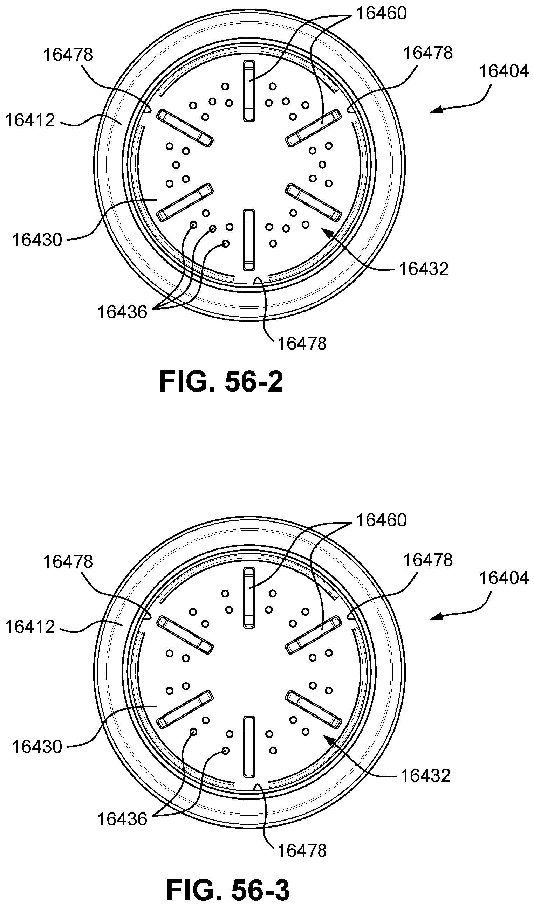

As shown in Fig. 56, the vent body 16404 may include a plurality of connection holes 16478. The connection holes 16478 may be disposed within the opening 16426 in the wall of the vent body 16404. For example, the connection holes 16478 may extend into the groove 16416. The illustrated example may include three connection openings 16478, although any number may be included. The connection openings 16478 may be evenly spaced around the perimeter of the vent body 16404.

As shown in Fig. 56-1, one form of the vent body 16404 may include the vent holes 16436 arranged in a first pattern or cluster. Each cluster may include five vent holes 16436. The cluster may be arranged in a trapezoidal shape. For example, a first row may include two vent holes 16436 and a second row may include three vent holes 16436. The first row may be radially inside of the second row.

As shown in Fig. 56-2, another form of the vent body 16404 may include vent holes 16436 arranged in a second pattern or cluster. Each cluster may include five vent holes 16436. The cluster may be arranged in a trapezoidal shape. For example, a first row may include two vent holes 16436 and a second row may include two vent holes 16436. A third or middle row may include one vent hole 16436. The first row may be radially inside of the second row. Additionally, the distance between the vent holes 16436 in the first row may be less than the distance between the vent holes 16436 in the second row.

As shown in Fig. 56-3, another form of the vent body 16404 may include vent holes 16436 arranged in a third pattern or cluster. Each cluster may include four vent holes 16436. The cluster of Fig. 56-3 may be similar to the cluster of Fig. 56-2, except the cluster of Fig. 56-3 may not include the single vent hole 16436 in the third row.

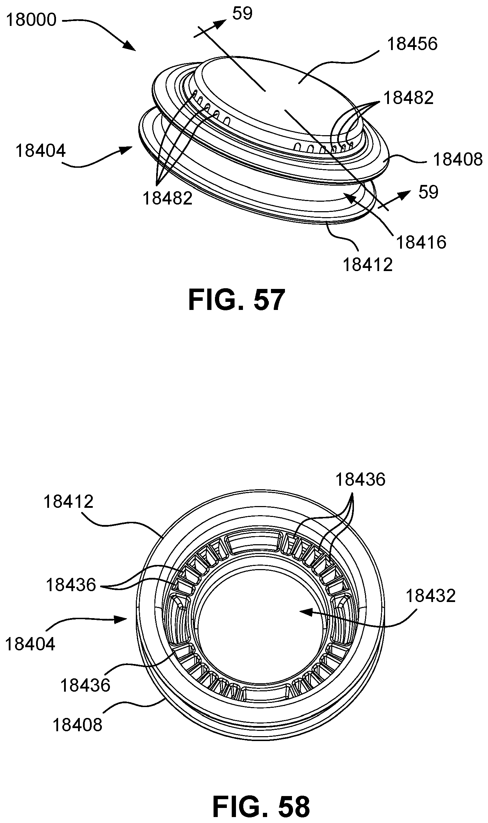

Figs. 57 and 58 3 illustrate an alternate example of a vent 18400, which may be similar to the vent 12400. Similar features may be labelled with similar reference numbers plus "6000". Only some similarities and differences are described below. The vent 18400 may have a substantially similar shape to the vent opening 13388 (e.g., a substantially circular shape).

As shown in Fig. 58, the vent holes 18436 may include a non-circular shape which may assist in improved diffusivity. For example, the vent holes 18436 may have a generally trapezoidal shape, and a radially inner end may have a smaller width than a radially outer end. Additionally, corners of the vent holes 18436 may be rounded. In other forms, the vent holes 18436 may be rectangular (e.g., with rounded corners), or any other shape.

In some forms, the vent holes 18436 may not form an entire perimeter of the vent body 18404. For example, the vent holes 18436 may be clustered together (e.g., in groups of four, five, or six), and no vent holes 18436 may be disposed between the clusters.

### 5.3.4.1 Diffuser

As shown in Figs. 7 and 8, a diffuser 6430 may be used with any of the patient interfaces. The diffuser 6430 may assist with limiting the decibel output from any of the patient interfaces. Specifically, the diffuser 6430 may assist in limiting the decibel level associated with air output from one of the patient interfaces (e.g., exhaled air), although the diffuser 6430 may limit the decibel level of at any point in the patient interface.

In the illustrated example, the diffuser 6430 is show with the patient interface 6000, although as described above, any of the different patient interfaces may utilize a diffuser 6430.

As shown in Fig. 17, the diffuser 6430 may include a dampening member 6432 and a cover 6436. The dampening member 6432 and the cover 6436 may be coupled to the frame 6350. Specifically, the dampening member 6432 and the cover 6436 may be coupled to the frame 6350 indirectly through the vent 6400.

In some forms, the dampening member 6432 may be constructed from a textile. The textile may be a breathable material, and allow airflow to pass through with substantially small resistance (e.g., in order to limit breathing disruptions). For example, the dampening member 6432 may be a fleece.

In some forms, the dampening member 6432 may be positioned against the recessed surface 6413 in order to be positioned proximate to the vent openings 6414. The air exiting the plenum chamber 6200 through the vent openings 6414 may pass directly into the dampening member 6432.

In certain forms, the dampening member 6432 may be positioned radially within the retaining tabs 6416. In other words, the retaining tabs 6416 may contact the outer perimeter of the dampening member 6432. The width between the retaining tabs 6416 may be slightly larger than width of the of the dampening member 6432, which may assist in maintaining the position of the dampening member 6432 relative to the vent body 6404. For example, the dampening member 6432 may be connected to the vent body 6404 by press-fitting the dampening member 6432 between the retaining tabs 6416.

In some forms, the cover 6436 may be constructed from a rigid or semi-rigid material. For example, the cover 6436 may be constructed from the same material as the vent body 6404.

In some forms, the cover 6436 may be substantially the same size as the recessed surface 6413. The cover 6436 may substantially cover the dampening member 6432 when coupled to the vent body 6404. The cover 6436 may limit accidental removal of the dampening member 6432 from the vent body 6404.

In certain forms, the cover 6436 may include tabs 6438 disposed around an outer perimeter of the cover 6436. The tabs 6438 may engage with the vent body 6404 in order to provide a press fit, friction fit, and or snap fit. The tabs 6438 may also include an angular position that substantially corresponds to the angular position of the retaining tabs 6416. The tabs 6438 may engage a surface of the retaining tabs 6416 in order to limit the translational movement of the cover 6436 toward the recessed surface 6413 (e.g., in order to limit compression of the dampening member 6432).

When the patient exhales, air may exit the plenum chamber through the vent openings 6414, which include relatively small diameters. The air passing through the vent openings 6414 may make noise (e.g., a whistling noise), which may disturb the sleep of the patient's bed partner. To assist in reducing the noise, the dampening member 6432 is positioned adjacent to the vent openings 6414, which allows air traveling to the ambient to pass through the dampening member 6432. The material of the dampening member 6432 helps to muffle the noise of the air flowing through the vent openings 6414. There may be gaps around the perimeter of the cover 6436 between the tabs 6438 where the exit the vent 6400 to the ambient.

As shown in Figs. 46 to 50, a diffuser 12448 may be used with the vent housing 12404. The diffuser 12448 may assist with limiting the decibel output from any of the patient interface 12000 (or any other patient interface). Specifically, the diffuser 12448 may assist in limiting the decibel level associated with air output from the patient interface 12000 (e.g., exhaled air), although the diffuser 12448 may limit the decibel level of at any point in the patient interface.

As shown in Figs. 49 and 49-1, the diffuser 12448 may include a dampening member 12452 and a cover 12456. The dampening member 12452 and the cover 12456 may be coupled to the vent body 12404. The diffuser 12448 may be similar to the diffuser 6430, and only some similarities and differences may be described.

In some forms, the dampening member 12452 may be constructed from a textile. The textile may be a breathable material, and allow airflow to pass through with substantially small resistance (e.g., in order to limit breathing disruptions). For example, the dampening member 12452 may be a fleece. In other forms, the dampening member 12452 may be constructed from a foam material, of from any other material that can be used to dampen noise.

In some forms, the dampening member may be about 0.1 mm to about 25 mm thick (e.g., measured perpendicular to the recessed surface 12430 when fully assembled). In some forms, the dampening member may be about 0.5 mm to about 10 mm thick. In some forms, the dampening member may be about 1 mm to about 5 mm thick. In some forms, the dampening member may be about 2.5 mm thick.

The dampening member 12452 may include a generally ring shape, and may be positionable (e.g., removably positionable) within the opening 12426 of the vent body 12404. For example, the dampening member 12452 may be positioned radially outside of the connecting features 12440. In some forms, the dampening member 12452 may contact an outer surface of the connecting features 12440. The dampening member 12452 may be snuggly positioned against the opening 12426 against the connecting features 12440 (e.g., in order to limit the dampening member from sliding within the opening 12426.

In some forms, a distance between an inner edge and an outer edge of the dampening member 12452 may be about 0.1 mm to about 25 mm. In some forms, a distance between an inner edge and an outer edge of the dampening member 12452 may be about 1 mm to about 15 mm. In some forms, a distance between an inner edge and an outer edge of the dampening member 12452 may be about 2 mm to about 10 mm. In some forms, a distance between an inner edge and an outer edge of the dampening member 12452 may be about 5.5 mm.

In some forms, the dampening member 12452 may be spaced apart from the recessed surface 12430 during use. For example, at least one rib 12460 may be disposed on the recessed surface 12430. The ribs 12460 may be radially outside of the connecting features 12440 and between the vent openings 12436 (e.g., between the cluster of vent holes 12436). For example, a rib 12460 may be disposed between each of the clusters of vent holes 12436. The dampening member 12452 may contact the ribs 12460 in order to create a space between the vent openings 12436 and the dampening member 12452. Creating a space between the dampening member 12452 and the vent holes 12436 may reduce impedance of the air exiting or entering the vent holes 12436 in the opening 12426. This may be useful if the dampening member 12460 becomes saturated and air exiting the plenum chamber 12200 is unable to easily flow through the dampening member 12460. Airflow may therefore exit the vent without having to pass through the dampening member 12460. In other forms, the recessed surface 12430 may not include ribs, and the dampening member 12452 may lie flat against the recessed surface 12430.

In some forms, each rib 12460 may extend about 0.1 mm to about 25 mm above the face of the recessed surface 12430. In some forms, each rib 12460 may extend about 0.5 mm to about 10 mm above the face of the recessed surface 12430. In some forms, each rib 12460 may extend about 1 mm to about 5 mm above the face of the recessed surface 12430. In some forms, each rib 12460 may extend about 1.5 mm above the face of the recessed surface 12430.

In some forms, a diameter (e.g., an outer diameter) of the dampening member 12452 may be larger than a distance from the center of the vent body 12404 to the vent holes 12436. Thus, the dampening member 12452 may project over the vent openings 12436. This may assist in limiting noise production in use.

In some forms, the cover 12456 may include may have a substantially circular shape (e.g., when viewed from the anterior direction as shown in Fig. 38). A diameter of the cover 12456 may be less than the diameter of the opening 12446 so that the cover 12456 may fit within the opening 12446 in use.

As shown in Fig. 50, some forms of the cover 12456 may not extend to the edge of the opening 12446. A gap 12464 may be formed between the edge of the cover 12456 and the surface of the vent body 12404 forming the opening 12446.

In some forms, the cover 12456 may be constructed from a rigid or semi-rigid material. For example, the cover 12456 may be constructed from the same material as the vent body 12404.

In some forms, the edge of the cover 12456 may be formed with a fillet, a chamfer, and/or may be rounded. This shape of the edge may assist in ensuring that the cover 12456 is spaced apart from the edge of the opening 12444. The shape of the edge of the cover 12456 may also assist in creating smooth fluid flow through the gap 12464.

In some forms, the cover 12456 may have a diameter that is at least as large as the outer diameter of the dampening member 12456 so that the cover 12456 covers the dampening member 12452 when coupled to the vent body 12404. The cover 12456 may limit accidental removal of the dampening member 12452 from the vent body 12404.

In some forms, a first (e.g., anterior) surface 12457 of the cover 12456 may include a curvature. For example, the first surface 12457 may include a negative domed shape when viewed in the posterior direction (e.g., Fig. 38).

As shown in Fig. 49-1, the cover 12456 may include a connecting feature 12468, which may extend from a posterior surface 12458 opposite the anterior surface 12457. The posterior surface 12458 may be substantially planar (e.g., not curved like the anterior surface 12457). The connecting feature 12468 may be arranged in a substantially circular shape that may be similar to the shape of the connecting feature 12440.

In some forms, the connecting feature 12468 of the cover 12456 may form a complete, uninterrupted perimeter. In some forms, the connecting feature 12468 may be discontinuous around the perimeter (e.g., similar to the connecting feature 12440 (see e.g., Fig. 49).

In some forms, a lip 12470 may be formed on the outer perimeter of the connecting feature 12468 of the cover 12456. In some forms, the lip 12470 may extend around the entire perimeter of the connecting feature 12468. In other forms, the lip 12470 may extend around only a portion of the connecting feature 12468.

In some forms, the connecting features 12468 may be spaced apart from an outer perimeter of the cover 12456. In other words, the position of the connecting features 12468 on the posterior surface 12458 may be spaced apart from the edge of the posterior surface 12458 so as not to overlap the edge of the posterior surface 12458.

In certain forms, the connecting feature 12468 may be positioned proximate to the center of the posterior surface 12458 in order to be spaced well away from the edge of the cover 12456.

In some forms, the surface 12457 of the cover 12456 may form a smooth and/or continuous curvature with the anterior face 12418 of the anterior surface 12408. For example, a single curve with one radius of curvature may be drawn over the anterior face 12418 and the surface 12457.

As shown in Fig. 50, the lip 12470 may be sized to connect with the finger(s) 12442 of the vent body 12404. For example, the outer diameter of the connecting feature 12468 of the cover 12456 may be less than the inner diameter of the connecting feature 12440 of the vent body 12404. The connection feature 12468 and the lip 12470 may be able to fit within the inner diameter of the connecting feature 12440. This engagement may occur radially inside of the outermost edge of the cover 12456. Once inserted, the lip 12470 may fit under the fingers 12442 in order to connect the cover 12456 to the vent body 12404. For example, the connection between the fingers 12442 and the lip 12470 forms a snap-fit connection.

In some forms, the connecting features 12440, 12468 may not be visible by a patient or bed partner when the cover 12456 is connected to the vent body 12404. For example, positioning the connecting features 12440, 12468 proximate to a center of the respective support surface (i.e., the anterior surface 12408 and the posterior surface 12458) means that the connecting features 12440, 12468 may be covered by the anterior surface 12457 of the cover 12456.

In some forms, the cover 12456 may be permanently connected to the vent body 12404. In other words, the lip 12470 may not be able to disconnect from the fingers 12442 once the snap-fit connection is initiated. This may make assembly easier for patients because they do not have to disassemble the vent 12400 and potentially lose pieces. The patient may clean the vent 12400 with all of the components attached (e.g., cleaning fluid may drain through the gaps 12444 to limit the cleaning fluid from being trapped in the vent 12400). The patient may dispose of the vent 12400 after a number of uses and replace the entire vent 12400. In other forms, the cover 12456 may be removably connected to the vent body 12404, and may be moved so that the vent body 12404, the dampening member 12452, and/or the cover 12456 may be cleaned and/or replaced individually,

In use, the vent 12400 may include a single airflow path for air exiting the plenum chamber 12200 through the vent 12400. Air may only be able to travel through the vent holes 12436 and through the gap 12464. In other words, the cover 12456 may block air from traveling through a center of the vent 12400 (e.g., through the connecting features 12440, 12468). Instead, there may only be a circumferential airflow path through the vent 12400. Because the gap 12464 may be continuous around the perimeter of the vent 12400 (e.g., as shown in Fig. 46), this may be a single opening.

In some forms, the gap 12464 may be continuous around the perimeter of the cover 12456. In other words, there may be a single opening, and not a plurality of discontinuous openings. This may be possible because the connecting features 12440, 12468 are spaced apart from the edge of the cover 12456, and therefore do not interfere with the gap 12464 (e.g., do not break the gap 12464 into a plurality of smaller gaps).

In the illustrated example, airflow passing through the gap 12464 may pass into the ambient environment. In other words, the vent 12400 may not extend above the anterior face 12418 such that air passing through the gap 12464 is still contained within the vent 12400. Although the surface 12457 of the cover 12456 may be more anterior to the anterior face 12418 (e.g., as a result of the curvature of the cover 12456), the surface 12457 does not retain or direct fluid flow that has passed through the gap 12464. Once the fluid passes anterior to the anterior face 12418, it is no longer within the vent 12400.

Limiting airflow through the center of the vent 12400 (e.g., because of the solid posterior surface 12458 of the cover 12456) may assist in liming noise output of the vent 12400, which may help the patient and/or the patient's bedpartner sleep. For example, the airflow may not be disturbed as a result of the passing over the fingers 12442 or the lip 12470.

Air exiting the vent holes 12436 may be directed into the dampening member 12452 (e.g., as a result of the dampening member 12452 projecting over the vent holes 12452). The noise of the air may be reduced as a result of passing through the dampening member 12452, which may help the patient or a bed partner better sleep. Moisture from exhaled air may collect in the dampening member 12452. As more moisture collects, the at least partially saturated dampening member 12452 may impede additional airflow through the dampening member 12452. The ribs 12460 space the dampening member 12452 apart from the vent holes 12436 so that air may still exit even if the dampening member 12542 is fully saturated. Additionally, the dampening member 12452 may not extend to the wall of the vent body 12404 so that a flow path (e.g., a circumferential path) exists between the dampening member 12452 and the vent body 12452. The airflow may flow alongside the dampening member 12452 and exit through the gap 12464. In some forms, the air may exit through the gap 12464 inclined with respect to an axis perpendicular to the recessed surface 12430. In other forms, the air may exit through the gap 12464 parallel with respect to an axis perpendicular to the recessed surface 12430.

In some forms, the sound power output by the vent 12400 with the single circumferential opening through the gap 12464 may be about 1 dBA to about 50 dBA. In some forms, the sound power output by the vent 12400 with the single circumferential opening through the gap 12464 may be about 5 dBA to about 25 dBA. In some forms, the sound power output by the vent 12400 with the single circumferential opening through the gap 12464 may be about 10 dBA to about 20 dBA. In some forms, the sound power output by the vent 12400 may be about 19.3 dBA.

In some forms, the sound pressure output by the vent 12000 with the single circumferential opening through the gap 12464 may be about 1 dBA to about 50 dBA. In some forms, the sound pressure output by the vent 12000 with the single circumferential opening through the gap 12464 may be about 5 dBA to about 25 dBA. In some forms, the sound pressure output by the vent 12000 with the single circumferential opening through the gap 12464 may be about 10 dBA to about 20 dBA. In some forms, the sound pressure output by the vent 12000 with the single circumferential opening through the gap 12464 may be about 12.7 dBA.

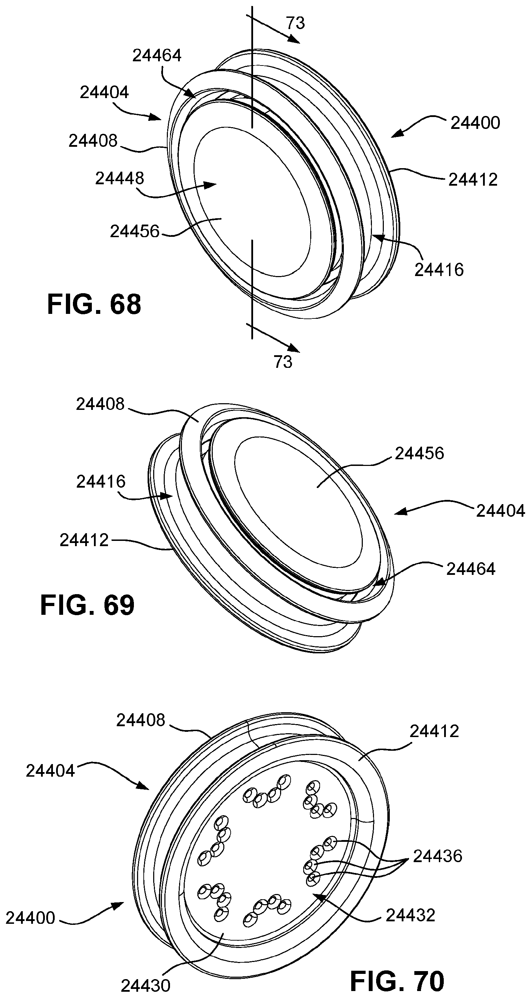

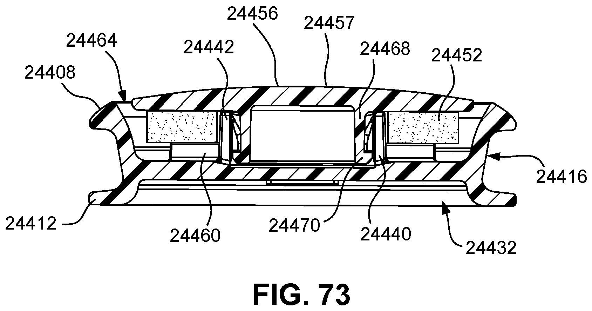

As shown in Figs. 68 to 73, an alternate example of the vent 12000 is illustrated. The vent 24000 may be similar to the vent 12000, and similar elements may be labelled with similar reference numerals plus "12000". Only some similarities and differences between the vent 12000 and the vent 24000 may be disclosed.

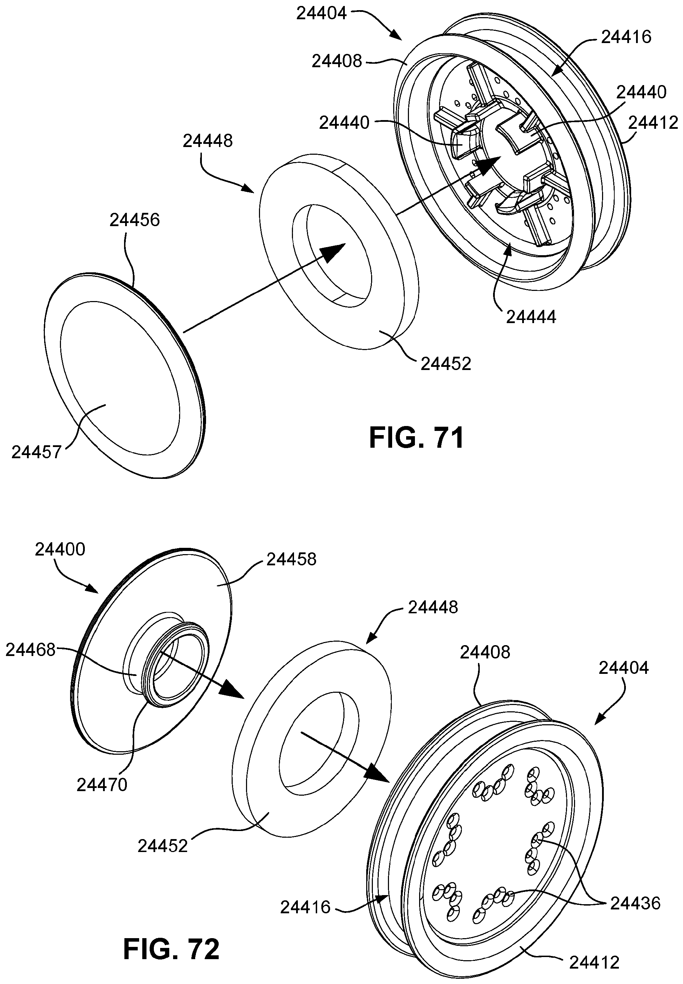

With specific reference to Fig. 72, the connecting feature 24468 may form a complete perimeter. For example, the connecting feature 24468 may have a substantially circular perimeter, although it may include other shapes (e.g., a oval, a rectangle, etc.) without departing from the scope of the disclosure.

Similarly, the lip 24470 may extend completely around the perimeter of the connecting feature 24468. The lip 24470 may form the substantially the same shape as the connecting feature.

In other examples, the lip 24470 may be discontinuous (e.g., similar to the example in Fig. 49-1) even if the connecting feature 24468 forms a complete perimeter.

In some forms, the connecting features 24440 may be discontinuous similar to the connecting features 12440 in Fig. 49. The connecting feature 24440 may similarly include a finger 24442 that may connect (e.g., permanently or removably via a snap-fit) to the lip 24470 when the cover 24456 and the housing 24404 are connected to one another.

Figs. 51 to 55 illustrate an alternate example of a diffuser 14448, which may be similar to the diffuser 12448. Similar features may be labelled with similar reference numbers plus "2000". Only some similarities and differences are described below.

As shown in Figs. 54 and 54-1, the diffuser 14448 may include a dampening member 14452 and a cover 14456. The dampening member 14452 and the cover 14456 may be coupled to the vent body 14404.

In some forms, the dampening member 14452 may be constructed from a textile. The textile may be a breathable material, and allow airflow to pass through with substantially small resistance (e.g., in order to limit breathing disruptions). For example, the dampening member 14452 may be a fleece. In other forms, the dampening member 14452 may be constructed from a foam material, of from any other material that can be used to dampen noise.

In some forms, the dampening member 14448 may be about 0.1 mm to about 25 mm thick (e.g., measured perpendicular to the recessed surface 14430 when fully assembled). In some forms, the dampening member 14448 may be about 0.5 mm to about 10 mm thick. In some forms, the dampening member 14448 may be about 1 mm to about 5 mm thick. In some forms, the dampening member 14448 may be about 2.5 mm thick.

The dampening member 14452 may include a generally ring shape, and may be positionable (e.g., removably positionable) within the opening 14426 of the vent body 14404. For example, the dampening member 14452 may be positioned radially outside of the connecting features 14440. In some forms, the dampening member 14452 may contact an outer surface of the connecting features 14440 (e.g., the dampening member 14452 may contact a radially outer surface of the inclined connecting features 14440). The dampening member 14452 may be snuggly positioned against the opening 12426 against the connecting features 12440 (e.g., in order to limit the dampening member from sliding within the opening 12426.

In some forms, an inner diameter of the dampening member 14452 may be larger than the inner diameter of the dampening member 12452. For example, the connecting features 14440 may be spaced further apart than the connecting features 12440. Therefore, the inner diameter of the dampening member 14452 may be larger in order to fit around the connecting features 14440. In some forms the other diameter of the dampening member 14452 is about the same as the outer diameter of the dampening member 14452, although they may be different sizes.

In some forms, the dampening member 14452 may be spaced apart from the recessed surface 12430 during use by the ribs 14460. The dampening member 14452 may contact the ribs 14460 in order to create a space between the vent openings 14436 and the dampening member 14452. Creating a space between the dampening member 14452 and the vent holes 14436 may reduce impedance of the air exiting or entering the vent holes 14436 in the opening 14426. This may be particularly helpful when humidified air is passing through the vent holes causing the dampening member 14460 to become saturated so that air exiting the plenum chamber 14200 is unable to easily flow through the dampening member 14460. Airflow may therefore exit the vent without having to pass through the dampening member 14460.

In some forms, a diameter (e.g., an outer diameter) of the dampening member 14452 may be larger than a distance from the center of the vent body 14404 to the vent holes 14436. Thus, the dampening member 14452 may project over the vent openings 14436. This may assist in limiting noise production in use.

In some forms, the cover 14456 may include may have a substantially ring shape (e.g., when viewed in the posterior direction as shown in Fig. 38). An outer diameter of the cover 14456 may be less than the diameter of the opening 14446 so that the cover 14456 may fit within the opening 14446 in use. An inner diameter of the cover 14456 may be less than a diameter of the connecting features 14440.

As shown in Fig. 55, some forms of the cover 14456 may not extend to the edge of the opening 14446. A gap 14464 may be formed between the edge of the cover 14456 and the surface of the vent body 14404 forming the opening 14446.

In some forms, the cover 14456 may be constructed from a rigid or semi-rigid material. For example, the cover 14456 may be constructed from the same material as the vent body 14404.

In some forms, the edge of the cover 14456 may be formed with a fillet, a chamfer, and/or may be rounded. This shape of the edge may assist in ensuring that the cover 14456 is spaced apart from the edge of the opening 14444. The shape of the edge of the cover 14456 may also assist in creating smooth fluid flow through the gap 14464.

In some forms, the cover 14456 may have a diameter that is at least as large as the outer diameter of the dampening member 14456 so that the cover 14456 covers the dampening member 14452 when coupled to the vent body 14404. The cover 14456 may limit accidental removal of the dampening member 14452 from the vent body 14404.

In some forms, a first (e.g., anterior) surface 14457 of the cover 14456 may include a curvature. For example, the first surface 14457 may include a partially negative domed shape when viewed in the posterior direction (e.g., Fig. 38). In other words, the curvature of the anterior surface 14457 may be similar to the curvature of the anterior surface 12247, except that the anterior surface 14457 is ring shaped and does not include material at a central point.

As shown in Fig. 54-1, the cover 14456 may include a connecting feature 14468, which may extend from a posterior surface 14458 opposite the anterior surface 14457. The posterior surface 14458 may be substantially planar (e.g., not curved like the anterior surface 14457). The connecting feature 14468 may be arranged in a substantially circular shape that may be similar to the shape of the connecting feature 14440.

In some forms, the connecting feature 14468 of the cover 14456 may form a complete, uninterrupted perimeter. In some forms, the connecting feature 14468 may be discontinuous around the perimeter (e.g., similar to the connecting feature 14440 (see e.g., Fig. 54)).

In some forms, a lip 14470 may be formed on the outer perimeter of the connecting feature 14468 of the cover 14456. In some forms, the lip 14470 may extend around the entire perimeter of the connecting feature 14468. In other forms, the lip 14470 may extend around only a portion of the connecting feature 14468.

As shown in Fig. 55, the lip 14470 may be sized to connect with the finger(s) 14442 of the vent body 14404. For example, the outer diameter of the connecting feature 14468 of the cover 14456 may be less than the inner diameter of the connecting feature 14440 of the vent body 14404. The connecting feature 14468 and the lip 14470 may be able to fit within the inner diameter of the connecting feature 14440. Once inserted, the lip 14470 may fit under the fingers 14442 in order to connect the cover 14456 to the vent body 14404. For example, the connection between the fingers 14442 and the lip 14470 forms a snap-fit connection. The engagement between the connecting features 14440, 14442 may be radially inside of an outermost edge of the cover 14456, so that the outermost edge of the cover 14456 is not in contact with the vent body 14404 (e.g., spaced apart from the anterior surface 14408 to form the gap 14464).

As shown in Fig. 54, some forms of the connecting feature 14440 may include a lower ledge 14472. The lower ledge 14472 may be parallel to and spaced apart from the respective finger 14442. In some forms, the distance between the respective finger 14442 and the lower ledge 14472 may be approximately the thickness of the lip 14472. This may limit movement of the cover 14456 while connected to the vent body 14404 in order to further limit noise output.

In some forms, the cover 14456 may be permanently connected to the vent body 14404. In other words, the lip 14470 may not be able to disconnect from the fingers 14442 once the snap-fit connection is initiated. This may make assembly easier for patients because they do not have to disassemble the vent 14400 and potentially lose pieces. The patient may clean the vent 14400 with all of the components attached (e.g., cleaning fluid may drain through the gaps 14444 to limit the cleaning fluid from being trapped in the vent 14400). The patient may dispose of the vent 14400 after a number of uses and replace the entire vent 14400. In other forms, the cover 14456 may be removably connected to the vent body 14404, and may be moved so that the vent body 14404, the dampening member 14452, and/or the cover 14456 may be cleaned and/or replaced individually,

In use, the vent 14400 may include multiple airflow paths for air exiting the plenum chamber 14200 through the vent 14400. For example, the vent 14400 may include two airflow paths. The first path may be similar to the single path in the vent 14400. For example, air may be able to travel through the vent holes 14436 and through the gap 14464.

A second airflow path may allow air to travel through a center of the vent 14400 (e.g., radially inside of the connecting features 14440, 14468). For example, the airflow may also be able to travel through the gaps 14444. Unlike the cover 12456, the cover 14456 is ring shaped and includes a central opening 14474 through which the airflow may also be able to exit. Thus, the vent 14400 may include a circumferential airflow path through the gap 14464 and a central airflow path through the central opening 14474 of the cover 14456.

In some forms, a center of the central opening 14474 may be concentric with a center of the cover 14456. The circumferential airflow path through the gap 14464 and the central opening 14474 may be concentric with one another.

Air exiting the vent holes 14436 may be directed into the dampening member 14452 (e.g., as a result of the dampening member 14452 projecting over the vent holes 14452). The noise of the air may be reduced as a result of passing through the dampening member 14452, which may help the patient or a bed partner better sleep. Moisture from exhaled air may collect in the dampening member 14452. As more moisture collects, the at least partially saturated dampening member 12452 may impede additional airflow through the dampening member 14452. The ribs 14460 space the dampening member 14452 apart from the vent holes 14436 so that air may still exit even if the dampening member 14542 is fully saturated. Additionally, the dampening member 14452 may not extend to the wall of the vent body 14404 so that a flow path (e.g., a circumferential path and/or central path) exists between the dampening member 14452 and the vent body 14452. The airflow may flow alongside the dampening member 14452 and exit through the gap 14464. In some forms, the air may exit through the gap 14464 inclined with respect to an axis perpendicular to the recessed surface 14430. In other forms, the air may exit through the gap 14464 parallel with respect to an axis perpendicular to the recessed surface 14430.

Figs. 51 to 55 illustrate an alternate example of a diffuser 14448, which may be similar to the diffuser 12448. Similar features may be labelled with similar reference numbers plus "2000". Only some similarities and differences are described below.

As shown in Fig. 56, the diffuser 16448 may include a dampening member 16452 and a cover 16456. The dampening member 16452 and the cover 14456 may be coupled to the vent body 16404. The dampening member 16452 may contact the ribs 16460. The diffuser 16448 may allow for two separate airflow paths like the diffuser 14448. For example, there may be a circumferential path and a central path.

With continued reference to Fig. 56, the cover 16456 may include fingers 16480. The fingers may project radially outward from the outer diameter of the cover 16456. The fingers 16480 may be sized to fit within the connection openings 16478. For example, assembling the diffuser 16448, the cover 16456 may be inserted into the opening 16426 so that the fingers 16480 are aligned with the connection openings 16478. The fingers 16480 may be positioned within or through the connection openings 16478 with a snap fit connection. In some forms, this may form a permanent connection as described with the engagement between the connecting features 14440, 14468. In other forms, the engagement between the fingers 16480 and the connection openings 16478 may be removable.

The fingers 16480 may be the radially outermost portion of the cover 16456. However, there may still be a gap or space between the cover 16456 and the vent body 16404 in the engaged position. This may still allow for the circumferential exhaust of the fluid flow through the vent (e.g., like in vent 12400 and 14400).

Figs. 57 to 59 illustrate an alternate example of a vent 18400, which may be similar to the vent 12400. Similar features may be labelled with similar reference numbers plus "6000". Only some similarities and differences are described below.

As illustrated in Fig. 57, the cover 18456 may be connected (e.g., permanently connected or removably connected) to the vent body 18404. The cover 18456 may have a circular shape (e.g., like the cover 12456) and form a single flow path, although other examples of the cover may be similar to the covers 14456, 16456 and be ring shaped to form multiple flow paths.

In some forms, the cover 18456 may be permanently connected to the vent body 18404 such that they are formed as one piece. Various gaps may be formed along the interface between the cover 18456 and the vent body 18404. The gaps may form the vent holes 18482.

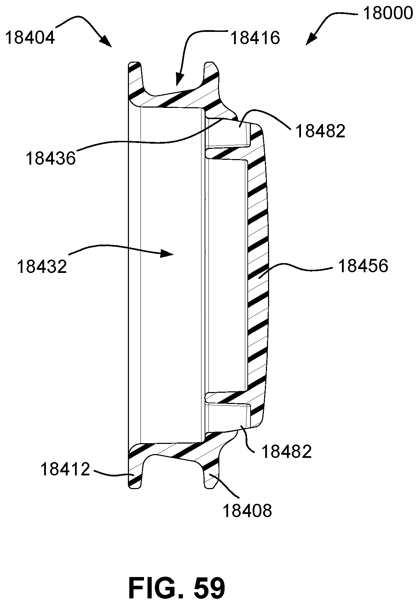

In other forms, the cover 18456 may extend to an edge of the vent body 18404 when connected to the vent body 18404. For example, the cover 18456 may include vent holes 18482 that are formed on an outer surface of the cover 18456. The cover 18456 may include vent holes 18482 connected with the vent holes 18436 of the vent body 18404 along a flow path. A fluid flow path may exist between the vent holes 18436 and the vent holes 18482 in order to create a circumferential flow path. The cover 18456 may not directly connect to the vent body 18404 because it is at least partially spaced apart. A dampening member 18452 (described below with respect to Fig. 59) may be positioned at least partially between the cover 18456 and the vent body 18404 in order to connect the two with a press fit or a frictional fit.

In other forms, the cover 18456 may extend to an edge of the vent body 18404 when connected to the vent body 18404 with a snap-fit. In other words, there may be no gap between the cover 18456 and the edge of the vent body 18404. Instead, the cover 18456 may include vent holes 18482 aligned with the vent holes 18436 of the vent body 18404. A fluid flow path may exist between the vent holes 18436 and the vent holes 18482 in order to create a circumferential flow path.

In some forms, the vent openings 18482 may include a U-shape, although any other shape may be used (e.g., V-shaped, circular, elliptical, etc.).

As shown in Fig. 59, an inner surface of the vent body 18404 may be arranged in a facing relationship with an outer surface of the cover 18456. In certain forms, a dampening member (not illustrated) may be positioned between the inner surface of the vent body 18404 and the outer surface of the cover 18456. The dampening member may be sized so that it substantially fills the space between the inner surface of the vent body 18404 and the outer surface of the cover 18456. For example, the width of the dampening member may be the same as the distance between the inner and outer surface. Alternatively, the dampening member may be larger than the distance between the inner and outer surface but may compress and/or deform in order to fit into the smaller space.

In certain forms, the dampening member may obstruct at least a portion of the vent holes 18482 in the cover 18456. As described with respect to previous examples, this may allow the airflow to pass through the dampening member 18452 before passing through the vent holes 18482 in order to dampen the airflow.

In some forms, the vent holes 18482 may be oriented radially outward. For example, this may be substantially parallel to a direction of a diameter of the vent body 18404 (e.g., an axis intersecting the vent body 18404 through the groove 18416), although the vent holes 18452 may be slightly inclined with respect to the diameter of the vent body 18404. The orientation of the vent holes 18482 may create an airflow path that is approximately 90° from the direction of the airflow into the vent body 18404. In other words, the direction of flow through the vent holes 18436 may be approximately 90° offset from the direction of flow through the vent holes 18482.

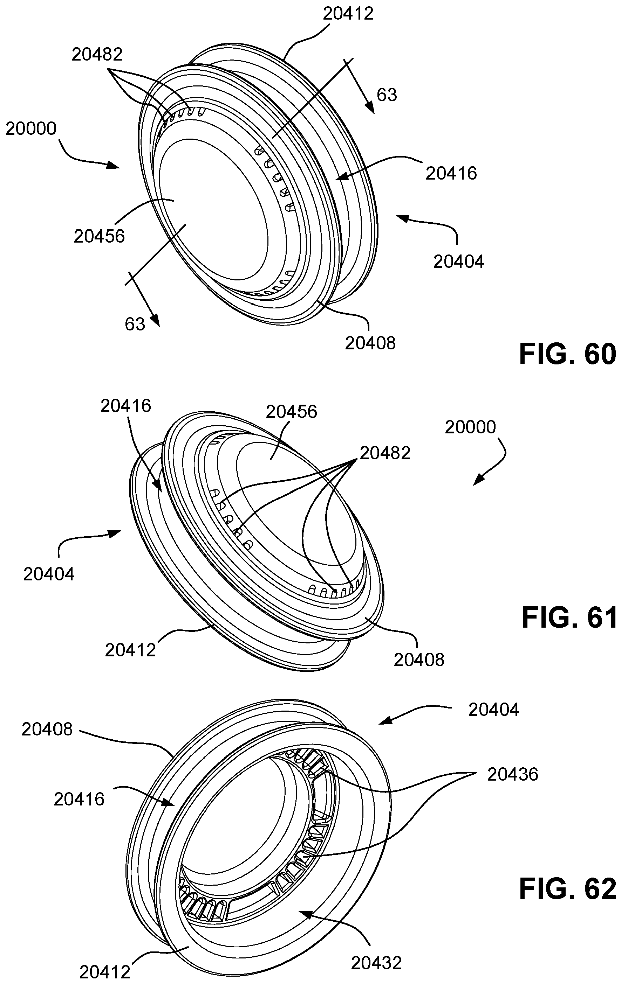

As illustrated in Figs. 60 to 63, a further alternate example of a vent 20400, which may be similar to the vent 18400. Similar features may be labelled with similar reference numbers plus "2000". Only some similarities and differences are described below.

As illustrated in Figs. 60 and 61, the cover 20456 may be connected (e.g., permanently connected or removably connected) to the vent body 20404. The cover 20456 may have a circular shape (e.g., like the cover 18456) and form a single flow path, although other examples of the cover may be similar to the covers 14456, 16456 and be ring shaped to form multiple flow paths.

In some forms, the cover 20456 may be permanently connected to the vent body 20404 such that they are formed as one piece. Various gaps may be formed along the interface between the cover 20456 and the vent body 20404. The gaps may form the vent holes 20482.

In other forms, the cover 20456 may extend to an edge of the vent body 20404 when connected to the vent body 20404. For example, the cover 20456 may include vent holes 20482 that are formed on an outer surface of the cover 20456. The cover 20456 may include vent holes 20482 connected with the vent holes 20436 of the vent body 20404 along a flow path. A fluid flow path may exist between the vent holes 20436 and the vent holes 20482 in order to create a circumferential flow path.

In some forms, the vent openings 20482 may include a U-shape, although any other shape may be used (e.g., V-shaped, circular, elliptical, etc.).

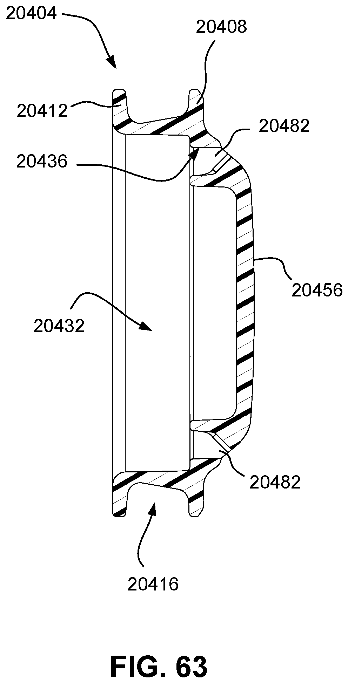

As shown in Fig. 63, an inner surface of the vent body 20404 may be arranged in a facing relationship with an outer surface of the cover 20456. In certain forms, a dampening member (not illustrated) may be positioned between the inner surface of the vent body 20404 and the outer surface of the cover 20456. The dampening member may be sized so that it substantially fills the space between the inner surface of the vent body 20404 and the outer surface of the cover 20456. For example, the width of the dampening member may be the same as the distance between the inner and outer surface. Alternatively, the dampening member may be larger than the distance between the inner and outer surface but may compress and/or deform in order to fit into the smaller space.

In certain forms, the dampening member may obstruct at least a portion of the vent holes 20482 in the cover 20456. As described with respect to previous examples, this may allow the airflow to pass through the dampening member 20452 before passing through the vent holes 20482 in order to dampen the airflow.

In some forms, the vent holes 20482 may be oriented radially outward. For example, this may be inclined relative to a direction of a diameter of the vent body 20404 (e.g., an axis intersecting the vent body 20404 through the groove 20416). The orientation of the vent holes 20482 may create an airflow path that is directed away from the posterior surface 20412. For example, a direction of an axis through the vent holes 20482 may be angled approximately 1° to approximately 89° from the posterior surface 20412. In some forms, a direction of an axis through the vent holes 20482 may be angled approximately 10° to approximately 70° from the posterior surface 20412. In some forms, a direction of an axis through the vent holes 20482 may be angled approximately 30° to approximately 50° from the posterior surface 20412. In some forms, a direction of an axis through the vent holes 20482 may be angled approximately 45° from the posterior surface 20412.

In certain forms, the orientation of the vent holes 20482 may assist in directing the airflow away from the patient's face (e.g., the patient's eyes). This may assist in avoiding irritation associated with airflow blowing into the patient's eyes.

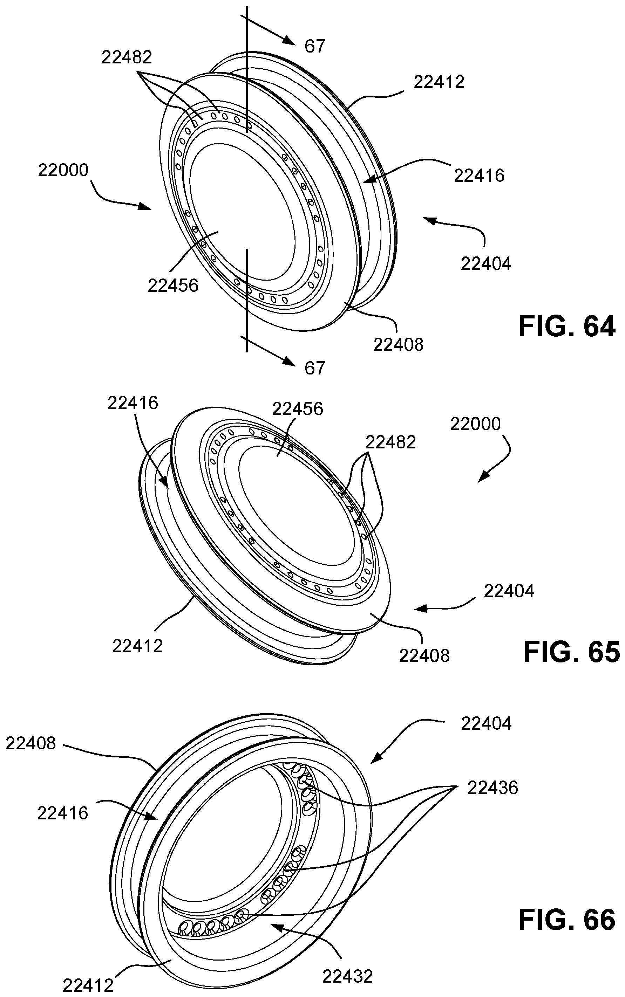

As illustrated in Figs. 64 to 67, a further alternate example of a vent 22400, which may be similar to the vent 18400. Similar features may be labelled with similar reference numbers plus "4000". Only some similarities and differences are described below.

As illustrated in Figs. 64 and 65, the cover 22456 may be connected (e.g., permanently connected or removably connected) to the vent body 22404. The cover 22456 may have a circular shape (e.g., like the cover 18456) and form a single flow path, although other examples of the cover may be similar to the covers 14456, 16456 and be ring shaped to form multiple flow paths.

In some forms, the cover 22456 may be permanently connected to the vent body 22404 such that they are formed as one piece. Various gaps may be formed along the interface between the cover 22456 and the vent body 22404. The gaps may form the vent holes 22482.

In other forms, the cover 22456 may extend to an edge of the vent body 22404 when connected to the vent body 22404. For example, the cover 22456 may include vent holes 22482 that are formed on an outer surface of the cover 22456. The cover 22456 may include vent holes 22482 connected with the vent holes 22436 of the vent body 22404 along a flow path.

In some forms, the vent openings 22482 may include a rounded shape, although any other shape may be used. For example, the vent openings 22482 may be circular shape and/or an elliptical shape. This may be similar to the shape shown in the previous example of Fig. 55 (see e.g., the shape of the opening 14436).

In some forms, the vent openings 22436 may include a similar shape (e.g., a rounded shape) as the vent openings 22482. This may be different than some of the previous examples (see e.g., the opening 20482 in Fig. 62) where the opening had an elongated shape (e.g., a U-shape).

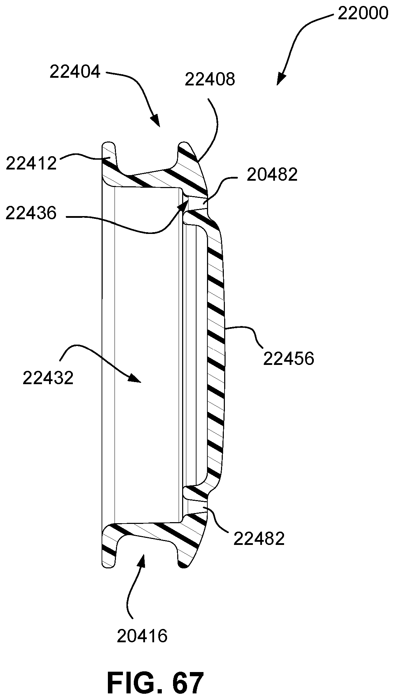

In certain forms, the diameter of each opening 22436, 22482 may be different. As illustrated in Fig. 67, the opening 22436 may have a larger diameter than the opening 22482, so that the flow path narrows as the air is exhausted.

In some forms, the vent holes 22482 may be oriented along an axial direction of the vent body 22404. For example, this may be substantially parallel relative to a direction of an axis intersecting the center of the vent cover 22456 and the center of the vent body 22404 (e.g., an axis intersecting the vent body 22404 through the groove 22416). The orientation of the vent holes 22482 may create an airflow path that is directed away from the posterior surface 22412.

In certain forms, the orientation of the vent holes 20482 may assist in directing the airflow away from the patient's face (e.g., the patient's eyes). This may assist in avoiding irritation associated with airflow blowing into the patient's eyes.

In some forms, the cover 22456 may be similarly shaped relative to the anterior surface 22408. For example, the cover 22456 may not protrude beyond the anterior surface 22408 as much as the previous examples (see e.g., Figs. 59 and 63). This may create a more compact appearance of the vent 22000, which patients may find more comfortable.

### 5.3.5 Decoupling structure(s)

In one form the patient interface 3000 includes at least one decoupling structure 3450, for example, a swivel or a ball and socket.

### 5.3.6 Conduit connection structure

As described above, some forms of the patient interface 9000 may include a conduit connection structure 9500 connected (e.g., removably, permanently) to the frame 9350 (see e.g., Fig. 27).

In some forms, each conduit connection structure 9500 may include a connection port 6600 (described below). The connection port 6600 may be integrally formed with the conduit connection structure 9500 (e.g., constructed from the same material), although in other forms, the connection port 6600 may be removable.

As shown in Figs. 34 and 35, the elliptical shape of the plenum chamber inlet ports 9254 may limit rotation of the conduit connection structures 9500 from rotating relative to the plenum chamber 9200. The engaged position of the conduit connection structures 9500 may be sufficient to provide the necessary force vector for the majority of patients, thus negating some need so adjust the position of the conduit connection structures 9500. However, the conduit connection structures 9500 may be permitted to rotate relative to the plenum chamber 9200 if the plenum chamber inlet ports 9254 are substantially circular in shape.

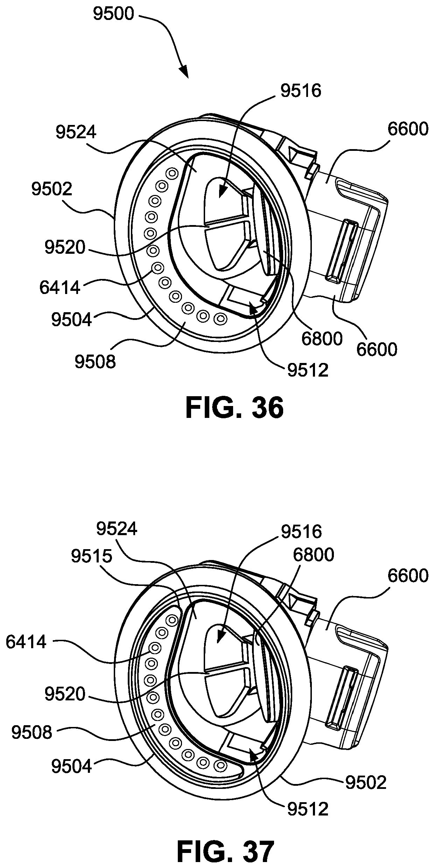

As shown in Figs. 36 and 37, the conduit connection structure 9500 may include an outer rim 9502 and an inner rim 9504, which may have a smaller diameter than the outer rim 9502. The outer rim 9502 may contact the respective annular portion 10050 of the frame 9350, while the inner rim 9504 may fit through the annular portion 10050 and through the plenum chamber inlet ports 9254.

In some forms, the conduit connection structure 9500 may be inserted through the respective annular portion 10050 via a press-fit, friction fit, or snap-fit. The conduit connection structures 9500 and the frame (e.g., either the frame 9350 or the frame 11350) may therefore connect to the plenum chamber inlet port 9254 as a single piece. The portion of the conduit connection structure 9500 that does not extend through the plenum chamber inlet port 9254 may provide a force to retain the frame 9350 within in the groove 9260, and therefore sandwich the frame 9350 against the plenum chamber 9200 (see e.g., Fig. 30).

With continued reference to Figs. 36 and 37, certain forms of the outer rim 9502 and the inner rim 9504 may be substantially coplanar. Here, the inner rim 9504 may not mechanically engage the plenum chamber inlet port 9254 as its perimeter is not entirely exposed. Instead, the outer rim 9502 may be formed as a stepped surface, which may be wider than the plenum chamber inlet opening 9254. The outer rim 9502 may fit entirely within the plenum chamber 9200 so that it is covered in use. The flexible nature of the plenum chamber 9200 may allow the wider conduit connection structure 9500 to fit within the plenum chamber inlet opening 9254, and connect to the plenum chamber 9200 with a snap fit, or similar engagement.

In certain forms, the vent openings 6414 may be disposed on the conduit connection structure 9500. Specifically, the vent openings 6414 may be disposed proximate to the inner rim 9504 in order to provide communication between the ambient and the interior of the plenum chamber 9200 (e.g., in order to permit carbon-dioxide washout).

Within the perimeter of the inner rim 9504, the conduit connection structure 9500 may include a planar surface 9508 and an airflow opening 9512. The vent openings 5414 may be disposed on the planar surface 9508 while pressurized air may flow through the airflow opening 9512.

As shown in Fig. 36, the planar surface 9508 may be flush with the inner rim 9504. The vent openings 6414 disposed on the planar surface 9508 may also be substantially coplanar with the inner rim 9504.

As shown in Fig. 37, the planar surface 9508 may be recessed relative to the inner rim 9504, so that the vent openings 6414 are not substantially coplanar with the inner rim 9504. A dividing wall 9515 may be disposed between the airflow opening 9512 and the planar surface 9508. The dividing wall 9515 may provide a barrier, and obstruct some flow of pressurized air from passing through the vent openings 6414 and directly into the ambient without first being inhaled by the patient. The dividing wall 9515 may also assist in directing exhaled air through the vent openings 6414, and not toward the connection port 6600.

As shown in Figs. 36 and 37, some forms of the conduit connection structure 9500 may include an opening or emergency vent 9516. Each conduit connection structure 9500 connected to the plenum chamber 9200 may include a single emergency vent 9516 with a rib 9520 extending across the width of the emergency vent 9516.

In certain forms, the emergency vent 9516 may be disposed downstream from the connection port 5600 and the upstream from the airflow opening 9512. Thus, the pressurized airflow passes the emergency vent 9516 prior to passing through the airflow opening 9512.

In one form, the connection port 5600 may be approximately 90° from the airflow opening 9512. In other words, the conduit connection structure 9500 may be oriented at approximately a right angle. The conduit connection structure 9500 may include a wall 9524 inclined (e.g., at a 45° angle) with respect to the connection port 6600, and with respect to the airflow opening 9512. The wall 9524 may assist in directing the flow of air from the connection port 6600 to the airflow opening 9512. The emergency vent 9516 may be disposed on the wall 9524. As described in detail below, an anti-asphyxia valve 6800 may be connected to the conduit connection structure 9500 and configured to seal against the emergency vent 9516. The flow of pressurized air through the connection port 6600 may flow directly into the anti-asphyxia valve 6800, and forcing it into a sealing engagement with the emergency vent 9516 (e.g., for the entire time the flow of pressurized air flows through the connection port 6600).

### 5.3.7 Connection port

Connection port 3600 allows for connection to the air circuit 4170.

As shown in Fig. 17, connection ports 6600 may be received in the connection openings 6358 of some forms of the frame 6350. The connection ports 6600 may be permanently received within the connection openings 6358, or may be removable (e.g., to facilitate cleaning and/or replacement).

In some forms, each connection port 6600 may extend substantially along the respective arm 6356. For example, an end of the connection port 6600 may be positioned proximate to the transition between the respective arm 6356 and the central portion 6352 of the frame 6350. Accordingly, each arm 6356 may form a flow path in order to allow pressurized air to flow into the volume of the frame 6350 along the arm 6356 only through the respective connection port 6600.

In some forms, each connection port 6600 may include a slot 6604 for receiving a complementary tab (not shown) on a conduit 6320. The engagement between the slot 6604 and the tab may secure (e.g., removably secure) each conduit 6320 to the respective connection port 6600.

In some forms, each connection port 6600 may include a secondary or emergency vent 6608, which may be aligned with the vent opening 6360 when the connection port 6600 is positioned within the respective arm 6356.

As shown in Fig. 13, the emergency vent 6608 may be inclined with respect to the longitudinal axis of the connection port 6600. In some forms, the emergency vent 6608 may be oriented between approximately 1° to approximately 90° with respect to the longitudinal axis. In some forms, the emergency vent 6608 may be oriented between approximately 5° to approximately 75° with respect to the longitudinal axis. In some forms, the emergency vent 6608 may be oriented between approximately 10° to approximately 60° with respect to the longitudinal axis. In some forms, the emergency vent 6608 may be oriented between approximately 15° to approximately 45° with respect to the longitudinal axis. In some forms, the emergency vent 6608 may be approximately 30° with respect to the longitudinal axis.

Returning to Fig. 17, some forms of the emergency vent 6608 may include a valve connection point 6612, which may receive an anti-asphyxia valve (AAV) 6800. The valve connection point 6612 may be exposed through the vent opening 6360 when each connection port 6600 is coupled to the respective arm 6356.

As shown in Figs. 33, 36, and 37, the connection port 6600 may be connected to the plenum chamber 9200 through a conduit connection structure 9500. In this form, pressurized air flowing through the connection port 6600 may not enter a volume of the frame 9350, and may instead flow directly into the plenum chamber 9200.

### 5.3.8 Forehead support

In one form, the patient interface 3000 includes a forehead support 3700.

### 5.3.9 Anti-asphyxia valve

In one form, the patient interface 3000 includes an anti-asphyxia valve (AAV).

In some forms, the AAV 6800 may be formed from a flexible material (e.g., a silicone membrane).

As shown in Fig. 17, some forms of the AAV 6800 may include a body 6804 and a tab 6808 connected to the body 6804 (e.g., formed as a single piece). The body 6804 may be thinner than the tab 6808, and include greater flexibility.

In some forms, the AAV 6800 may be coupled to the connection port 6600 (e.g., via a press fit, a friction fit, a snap fit, etc.) by inserting the tab 6808 through the valve connection point 6612. In use, the body 6804 may be positioned within the volume of the respective arm 6356 and/or the volume of the connection port 6600. The AAV 6800 may be connected to the connection port 6600 in a cantilevered arrangement, where the body 6804 is the free end and the tab 6808 is the fixed end.

In some forms, the body 6804 of the AAV 6800 may extend substantially perpendicular to the longitudinal axis of the connection port 6600 in a relaxed position. The flow of pressurized breathable gas from the conduits 6320 into the frame 6350 moves the AAV 6800 into a closed position. Specifically, the body 6804 may pivot about its connection point with the tab 6808. The body 6804 may pivot so that it contacts the emergency vent 6608, and may be retained in the closed position as long as the flow of pressurized breathable gas continues. While in contact with the emergency vent 6608, the body 6804 creates a seal so that the flow of pressurized breathable gas cannot leak out of the connection port 6600, and into the ambient.

In some forms, the inclined orientation of the emergency vent 6608 may require the body 6804 to pivot through a lower angle in order to move to the closed position (see e.g., Fig. 13). This may require less time for the body 6804 to seal against the emergency vent 6608, and may also require less time for the body 6804 to unseal from the emergency vent 6608 and return to the open position (e.g., as compared to an emergency vent 6608 with a greater angle).

The body 6804 will pivot back to its relaxed position when the flow of pressurized breathable gas stops. This may allow a patient to continue to breathe if the flow stops while they are asleep. Ambient air may enter and exit the plenum chamber 6200 through the vent opening 6360 and the emergency vent 6608. Examples where the emergency vent 6608 is inclined may allow the patient's airways to more quickly communicate with the ambient, thereby creating minimal disruption with the patient's breathing cycle in the event that the flow of pressurized air into the plenum chamber 6200 stops while the patient remains asleep.

As shown in Figs. 20 to 26, the AAV 6800 may be coupled directly to the central portion 7352 of the frame 7350. For example, the tab 6808 may be positioned within the tab opening 7392 so that the body 6804 extends toward the plenum chamber 7200, and may extend generally across the outlet 7359. As pressurized air flows through the arms 7356, the air may contact the bodies 6804 causing them to pivot toward the central portion 7352. Each body 6804 may be larger than the respective emergency vent opening 7388, so that each body 6804 may cover and seal with the respective emergency vent opening 7388. The ribs 7390 may provide a backstop for each body 6804 so that the bodies 6804 to not pivot through the respective emergency vent opening 7388.

As shown in Figs. 36 and 37, an AAV 6800 may be connected to a conduit connection structure 9500, and may function substantially similarly as the AAVs 6800 connected to a frame and described above.

### 5.3.10 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller, a therapy device controller, a pressure generator 4140, one or more protection circuits, memory, transducers 4270, data communication interface and one or more output devices. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 5.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH2O, or in other forms up to about 30 cmH2O when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000.

In one form, a signal from a transducer 4270 may be filtered, such as by low-pass, high-pass or band-pass filtering.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller.

In one form, the input device 4220 may be constructed and arranged to allow a person to select a value and/or a menu option.

### 5.4.3 RPT device algorithms

In some forms of the present technology, a central controller may be configured to implement one or more algorithms expressed as computer programs stored in a non-transitory computer readable storage medium, such as memory. The algorithms are generally grouped into groups referred to as modules.

In other forms of the present technology, some portion or all of the algorithms may be implemented by a controller of an external device such as the local external device or the remote external device. In such forms, data representing the input signals and/or intermediate algorithm outputs necessary for the portion of the algorithms to be executed at the external device may be communicated to the external device via the local external communication network or the remote external communication network. In such forms, the portion of the algorithms to be executed at the external device may be expressed as computer programs, such as with processor control instructions to be executed by one or more processor(s), stored in a non-transitory computer readable storage medium accessible to the controller of the external device. Such programs configure the controller of the external device to execute the portion of the algorithms.

In such forms, the therapy parameters generated by the external device via the therapy engine module (if such forms part of the portion of the algorithms executed by the external device) may be communicated to the central controller to be passed to the therapy control module.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller. One example of an air circuit 4170 comprising a heated wire circuit is described in United States Patent 8,733,349.

In one form of the present technology, supplementary gas, e.g. oxygen, 4180 is delivered to one or more points in the pneumatic path, such as upstream of the pneumatic block 4020, to the air circuit 4170, and/or to the patient interface 3000 or 3800.

### 3.6 HUMIDIFIER

### 5.6.1 Humidifier overview

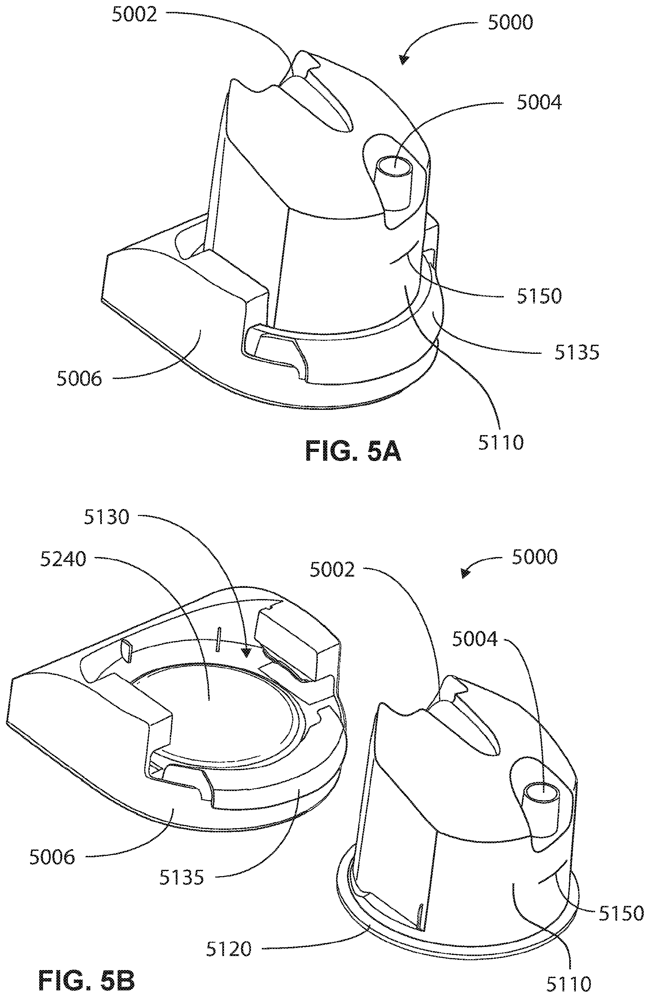

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 5.6.2 Humidifier components

### 5.6.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 5.6.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 5.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 5.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 5.6.2.5 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/171072.

In some forms, the heating element 5240 may be provided in the humidifier base 5006 where heat may be provided to the humidifier reservoir 5110 primarily by conduction as shown in Fig. 5B.

### 5.7 BREATHING WAVEFORMS

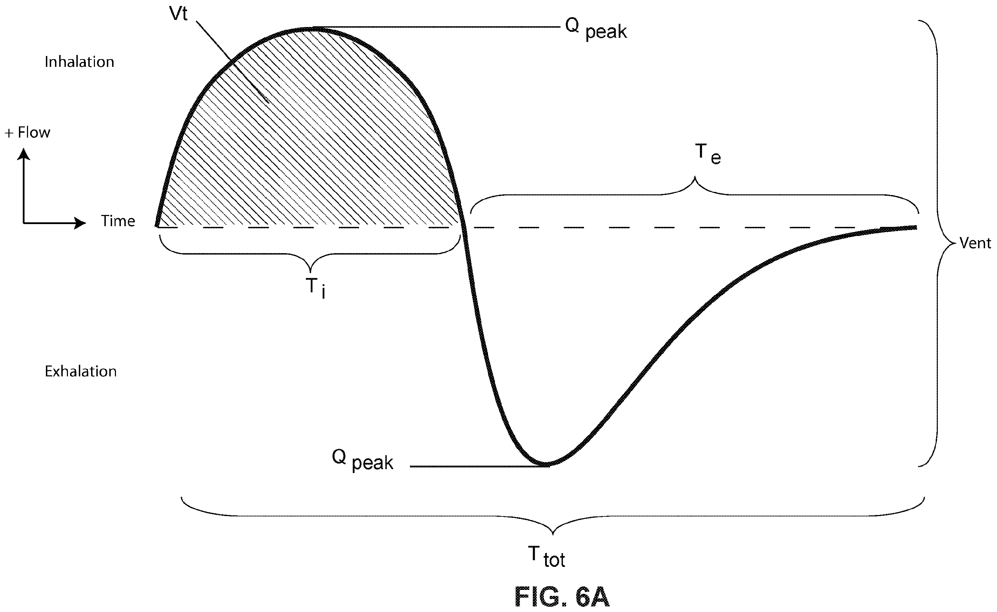

Fig. 6A shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Trot,* is about 4s. The person typically breathes at a rate of about 15 breaths per minute (BPM), with Ventilation *Vent* about 7.5 L/min. A typical duty cycle, the ratio of *Ti* to *Trot,* is about 40%.

### 5.9 GLOSSARY

For the purposes of the present technology disclosure, in certain forms of the present technology, one or more of the following definitions may apply. In other forms of the present technology, alternative definitions may apply.

### 5.9.1 General

*Air:* In certain forms of the present technology, air may be taken to mean atmospheric air, and in other forms of the present technology air may be taken to mean some other combination of breathable gases, e.g. oxygen enriched air.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

Automatic Positive Airway Pressure (APAP) therapy: CPAP therapy in which the treatment pressure is automatically adjustable, e.g. from breath to breath, between minimum and maximum limits, depending on the presence or absence of indications of SDB events.

Continuous Positive Airway Pressure (CPAP) therapy: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol *Q.* 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 5.9.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.9.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.9.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) *Flattened:* Having a rise followed by a relatively flat portion, followed by a fall.
(ii) *M-shaped:* Having two local peaks, one at the leading edge, and one at the trailing edge, and a relatively flat portion between the two peaks.
(iii) *Chair-shaped:* Having a single local peak, the peak being at the leading edge, followed by a relatively flat portion.
(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea:* An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.9.3 Anatomy

### 5.9.3.1 Anatomy of the face

Ala: the external outer wall or "wing" of each nostril (plural: alar)

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 5.9.3.2 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.9.4 Patient interface

Anti-asphyxia valve (AAV): The component or sub-assembly of a mask system that, by opening to atmosphere in a failsafe manner, reduces the risk of excessive CO2 rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.9.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 5.9.5.1 Curvature in one dimension

The curvature of a plane curve at *p* may be described as having a sign (e.g. positive, negative) and a magnitude (e.g. 1/radius of a circle that just touches the curve at *p*)*.*

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 5.9.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from *f*(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.9.5.3 Space curves

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.9.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.10 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Furthermore, "approximately", "substantially", "about", or any similar term as used herein means +/- 5-10% of the recited value.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior technology. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 5.11 REFERENCE SIGNS LIST

| | |
|---|---|
| patient | 1000 |
| bed partner | 1100 |
| patient interface | 3000 |
| seal-forming structure | 3100 |
| plenum chamber | 3200 |
| chord | 3210 |
| superior point | 3220 |
| inferior point | 3230 |
| positioning and stabilizing structure | 3300 |
| vent | 3400 |
| connection port | 3600 |
| forehead support | 3700 |
| RPT device | 4000 |
| external housing | 4010 |
| upper portion | 4012 |
| portion | 4014 |
| panel | 4015 |
| chassis | 4016 |
| handle | 4018 |
| pneumatic block | 4020 |
| air filter | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| air circuit | 4171 |
| electrical components | 4200 |
| single Printed Circuit Board Assembly | 4202 |
| power supply | 4210 |
| input device | 4220 |
| transducer | 4270 |
| humidifier | 5000 |
| humidifier inlet | 5002 |
| humidifier outlet | 5004 |
| humidifier base | 5006 |
| humidifier reservoir | 5110 |
| conductive portion | 5120 |
| humidifier reservoir dock | 5130 |
| locking lever | 5135 |
| water level indicator | 5150 |
| patient interface | 6000 |
| seal - forming structure | 6100 |
| first seal - forming structure | 6101 |
| second seal - forming structure | 6102 |
| entire boundary | 6103 |
| central portion | 6110 |
| lateral portions | 6111 |
| nasal opening | 6112 |
| bridge portion | 6114 |
| ridge | 6120 |
| lip inferior portion | 6130 |
| lip superior portion | 6131 |
| oral hole | 6133 |
| plenum chamber | 6200 |
| oral portion | 6201 |
| nasal portion | 6202 |
| first anterior wall portion | 6240 |
| shell | 6250 |
| central portion | 6251 |
| opening | 6254 |
| groove | 6260 |
| groove | 6266 |
| central portion | 6267 |
| side portion | 6268 |
| transition | 6269 |
| positioning and stabilizing structure | 6300 |
| headgear | 6302 |
| inferior strap | 6304 |
| magnetic member | 6306 |
| conduit | 6320 |
| frame | 6350 |
| central portion | 6352 |
| aperture | 6354 |
| posterior aperture | 6355 |
| arm | 6356 |
| connection opening | 6358 |
| vent opening | 6360 |
| wing | 6364 |
| groove | 6366 |
| central portion | 6367 |
| engagement mechanism | 6368 |
| magnet | 6370 |
| cover | 6372 |
| posterior lip | 6376 |
| anterior surface | 6378 |
| vent | 6400 |
| vent body | 6404 |
| vent arm | 6408 |
| anterior rim | 6410 |
| posterior rim | 6411 |
| groove | 6412 |
| surface | 6413 |
| vent holes | 6414 |
| tabs | 6416 |
| opening | 6418 |
| anterior lip | 6420 |
| posterior lip | 6422 |
| groove | 6424 |
| peripheral surface | 6428 |
| diffuser | 6430 |
| dampening member | 6432 |
| cover | 6436 |
| tabs | 6438 |
| one decoupling structure | 6450 |
| elbow | 6500 |
| respective aperture | 6555 |
| connection port | 6600 |
| slot | 6604 |
| emergency vent | 6608 |
| valve connection point | 6612 |
| forehead support | 6700 |
| anti - asphyxia valve | 6800 |
| body | 6804 |
| tab | 6808 |
| patient interface | 7000 |
| seal - forming structure | 7100 |
| structure | 7102 |
| plenum chamber | 7200 |
| opening | 7254 |
| frame | 7350 |
| central portion | 7352 |
| arm | 7356 |
| connection opening | 7358 |
| outlet | 7359 |
| wall | 7384 |
| wall | 7386 |
| vent openings | 7388 |
| ribs | 7390 |
| tab opening | 7392 |
| sleeve | 8000 |
| central portion | 8002 |
| vent opening | 8004 |
| emergency vent opening | 8008 |
| sleeve arm | 8012 |
| opening | 8016 |
| patient interface | 9000 |
| seal - forming structure | 9100 |
| structures | 9101 |
| structure | 9102 |
| plenum chamber | 9200 |
| oral portion | 9201 |
| nasal portion | 9202 |
| central portion | 9251 |
| plenum chamber inlet port | 9254 |
| groove | 9260 |
| outer portion | 9261 |
| planar surface | 9270 |
| lip | 9290 |
| frame | 9350 |
| central portion | 9360 |
| connection point | 9364 |
| conduit connection structure | 9500 |
| outer rim | 9502 |
| inner rim | 9504 |
| planar surface | 9508 |
| airflow opening | 9512 |
| wall | 9515 |
| emergency vent | 9516 |
| rib | 9520 |
| wall | 9524 |
| annular portion | 10050 |
| annular portion | 11050 |
| protrusion | 11054 |
| frame | 11350 |
| central portion | 11360 |
| patient interface | 12000 |
| seal-forming structure | 12100 |
| first seal - forming structure | 12101 |
| second seal - forming structure | 12102 |
| plenum chamber | 12200 |
| nasal portion | 12201 |
| oral portion | 12202 |
| central portion | 12251 |
| plenum chamber inlet port | 12254 |
| curved side | 12255 |
| groove | 12266 |
| vent | 12400 |
| vent housing | 12404 |
| anterior surface | 12408 |
| posterior surface | 12412 |
| groove | 12416 |
| anterior face | 12418 |
| posterior face | 12420 |
| anterior face | 12422 |
| posterior face | 12424 |
| wall | 12425 |
| opening | 12426 |
| opening | 12428 |
| recessed surface | 12430 |
| additional opening | 12432 |
| vent hole | 12436 |
| connecting feature | 12440 |
| vent opening | 13388 |
| lip | 13392 |
| plenum chamber | 14200 |
| clip | 14304 |
| arm | 14304-1 |
| clip | 14308 |
| center line | 14312 |
| fold line | 14316 |
| vent body | 14404 |
| anterior surface | 14408 |
| posterior surface | 14412 |
| groove | 14416 |
| anterior face | 14418 |
| posterior face | 14420 |
| anterior face | 14422 |
| posterior face | 14424 |
| opening | 14426 |
| opening | 14428 |
| recessed surface | 14430 |
| additional opening | 14432 |
| opening | 14436 |
| connecting feature | 14440 |
| finger | 14442 |
| gap | 14444 |
| opening | 14446 |
| diffuser | 14448 |
| damping member | 14452 |
| cover | 14456 |
| anterior surface | 14457 |
| posterior surface | 14458 |
| rib | 14460 |
| gap | 14464 |
| connecting feature | 14468 |
| lip | 14470 |
| central opening | 14474 |
| vent | 16400 |
| vent body | 16404 |
| anterior surface | 16408 |
| posterior surface | 16412 |
| groove | 16416 |
| opening | 16426 |
| recessed surface | 16430 |
| additional opening | 16432 |
| opening | 16436 |
| diffuser | 16448 |
| damping member | 16452 |
| cover | 16456 |
| rib | 16460 |
| central opening | 16478 |
| opening | 16474 |
| finger | 16480 |
| vent | 18400 |
| vent body | 18404 |
| anterior surface | 18408 |
| posterior surface | 18412 |
| groove | 18416 |
| additional opening | 18432 |
| opening | 18436 |
| cover | 18456 |
| vent holes | 18482 |
| vent | 20400 |
| vent body | 20404 |
| anterior surface | 20408 |
| posterior surface | 20412 |
| groove | 20416 |
| additional opening | 20432 |
| opening | 20436 |
| cover | 20456 |
| vent holes | 20482 |
| vent | 22400 |
| vent body | 22404 |
| anterior surface | 22408 |
| posterior surface | 22412 |
| groove | 22416 |
| additional opening | 22432 |
| opening | 22436 |
| cover | 22456 |
| vent holes | 22482 |
| vent | 24400 |
| vent body | 24404 |
| anterior surface | 24408 |
| posterior surface | 24412 |
| groove | 24416 |
| additional opening | 24432 |
| opening | 24436 |
| cover | 24456 |
| vent holes | 24482 |

## Claims

1. A patient interface (12000) comprising:
a plenum chamber (12200) including a cavity that is pressurisable to a therapeutic pressure of at least 6 cmH₂O above ambient air pressure, said plenum chamber including a left plenum chamber inlet port (12254) sized and structured to receive a flow of air at the therapeutic pressure for breathing by a patient, and a right plenum chamber inlet port (12254) sized and structured to receive a flow of air at the therapeutic pressure for breathing by a patient, the plenum chamber being constructed from a flexible material and being configured to be able to bend, the plenum chamber further including a left groove (12266) at least partially surrounding the left plenum chamber inlet port and a right groove at least partially surrounding the right plenum chamber inlet port;
a seal-forming structure (12100) constructed and arranged to form a seal with a region of the patient's face surrounding an entrance to the patient's airways such that the flow of air at said therapeutic pressure is delivered to the patient's airways, the seal-forming structure constructed and arranged to maintain said therapeutic pressure in the plenum chamber throughout the patient's respiratory cycle in use;
a vent structure (12400) connected to a vent opening (13388) in the plenum chamber, the vent structure configured to allow a continuous flow of gases exhaled by the patient from an interior of the plenum chamber to vent to ambient, said vent structure being sized and shaped to maintain the therapeutic pressure in the plenum chamber in use, the vent structure comprising:
a vent body (12404) formed from a rigid material and directly positioned within the vent opening, the vent body configured to direct bending movement in the plenum chamber away from central axis that intersects the vent opening, the vent body comprising a surface having a plurality of vent holes (12436) configured to allow air to exit the plenum chamber; and
a positioning and stabilizing structure (12300) configured to maintain the seal-forming structure in a therapeutically effective position, the positioning and stabilizing structure comprising:
a first left clip (14304) received in the left plenum chamber inlet port (12254),
a second left clip received in the left groove,
a first right clip (14304) received in the right plenum chamber inlet port (12254), and
a second right clip received in the right groove.

2. The patient interface (12000) of claim 1, wherein the first left clip (14304) and the second left clip are connected to the positioning and stabilizing structure (12300) independently of one another, and wherein the first right clip (14304) and the second right clip are connected to the plenum chamber (12200) independently of one another.

3. The patient interface (12000) of claim 2, wherein the second left clip and the second right clip are permanently connected to the plenum chamber (12200).

4. The patient interface (12000) of claim 1, wherein the first left clip (14304) and the second left clip are permanently connected to one another, and wherein the first right clip (14304) and the second right clip are permanently connected to one another.

5. The patient interface (12000) of any one of claims 1 to 4, wherein the second left clip and the second right clip each include a connection member (12364), preferably wherein the connection member is a magnet.

6. The patient interface (12000) of any one of claims 1 to 5, wherein the positioning and stabilizing structure (12300) includes a conduit headgear configured to convey the flow of air to the plenum chamber (12200).

7. The patient interface (12000) of claim 6, wherein the first left clip (14304) and the first right clip (14304) are connected to the conduit headgear.

8. The patient interface (12000) of any one of claims 6 to 7, wherein the positioning and stabilizing structure (12300) further comprises at least one strap (12304), and wherein the conduit headgear includes a tab configured to removably receive the at least one strap.

9. The patient interface (12000) of any one of claims 1 to 8, wherein a cover (12456) is permanently connected to the vent body (12404) and includes a plurality of openings (12436), and wherein each opening is in communication with one of the respective vent holes (12436) in order to provide a plurality of flow paths for air to exit the plenum chamber (12200).

10. The patient interface (12000) of claim 9, wherein the plurality of openings on the cover (12456) are oriented radially outward.

11. The patient interface (12000) of claim 9, wherein the plurality of openings on the cover (12456) are oriented in substantially the same direction as the plurality of vent holes (12436), preferably wherein diameters of the plurality of vent holes is greater than diameters of the plurality of openings.

12. The patient interface (12000) of any one of claims 9 to 11, wherein the plurality of vent holes (12436) are arranged in clusters, wherein each cluster is arranged in a given pattern and is spaced apart from adjacent clusters, preferably wherein the clusters include four, five, or six vent holes.

13. The patient interface (12000) of any one of claims 9 to 12, wherein the vent body (12404) includes an anterior surface (12418) and a posterior surface (12420) opposite the anterior surface and positioned within the plenum chamber (12200) in use, preferably wherein the plurality of vent holes (12436) are recessed relative to the posterior surface in the direction of the anterior surface.

14. The patient interface (12000) of claim 13, wherein the cover (12456) projects beyond the anterior surface (12418) in a direction away from the posterior surface (12420), and wherein the plurality of openings are disposed between the anterior surface and an outer surface of the cover.

15. The patient interface (12000) of any one of claims 13 to 14, wherein a groove (12416) is disposed between the anterior surface (12418) and the posterior surface (12420), and wherein the groove is configured to receive a portion of the plenum chamber (12200) forming an outer perimeter of the vent opening (13388).

## Patentansprüche

1. Patientenschnittstelle (12000), die aufweist:
eine Plenumkammer (12200) mit einem Hohlraum, der auf einen therapeutischen Druck von mindestens 6 cmH₂O über Umgebungsluftdruck druckbeaufschlagbar ist, wobei die Plenumkammer aufweist: einen linken Plenumkammer-Einlassanschluss (12254), der so bemessen und strukturiert ist, dass er eine Luftströmung mit dem therapeutischen Druck zur Atmung durch einen Patienten empfängt, und einen rechten Plenumkammer-Einlassanschluss (12254), der so bemessen und strukturiert ist, dass er eine Luftströmung mit dem therapeutischen Druck zur Atmung durch einen Patienten empfängt, wobei die Plenumkammer aus einem flexiblen Material aufgebaut und so konfiguriert ist, dass sie sich biegen kann, und die Plenumkammer ferner aufweist: eine linke Nut (12266), die den linken Plenumkammer-Einlassanschluss zumindest teilweise umgibt, und eine rechte Nut, die den rechten Plenumkammer-Einlassanschluss zumindest teilweise umgibt;
eine dichtungsbildende Struktur (12100), die so aufgebaut und angeordnet ist, dass sie eine Dichtung mit einer Region des Gesichts des Patienten bildet, die einen Eingang zu den Luftwegen des Patienten umgibt, so dass die Luftströmung mit dem therapeutischen Druck zu den Luftwegen des Patienten abgegeben wird, wobei die dichtungsbildende Struktur so aufgebaut und angeordnet ist, dass sie den therapeutischen Druck in der Plenumkammer über den gesamten Atemzyklus des Patienten im Gebrauch aufrecht erhält;
eine Entlüftungsstruktur (12400), die mit einer Entlüftungsöffnung (13388) in der Plenumkammer verbunden ist, wobei die Entlüftungsstruktur so konfiguriert ist, dass sie eine kontinuierliche Strömung von Gasen, die vom Patienten ausgeatmet werden, aus einem Innenraum der Plenumkammer ermöglicht, um sie in die Umgebung zu entlüften, wobei die Entlüftungsstruktur so bemessen und geformt ist, dass sie den therapeutischen Druck in der Plenumkammer im Gebrauch aufrecht erhält, und die Entlüftungsstruktur aufweist:
einen Entlüftungskörper (12404), der aus einem steifen Material gebildet und direkt in der Entlüftungsöffnung positioniert ist, wobei der Entlüftungskörper so konfiguriert ist, dass er Biegebewegung in der Plenumkammer weg von der Mittelachse leitet, die die Entlüftungsöffnung schneidet, und der Entlüftungskörper eine Oberfläche mit mehreren Entlüftungslöchern (12436) aufweist, die so konfiguriert sind, dass Luft aus der Plenumkammer austreten kann; und
eine Positionier- und Stabilisierstruktur (12300), die so konfiguriert ist, dass sie die dichtungsbildende Struktur in einer therapeutisch wirksamen Position hält, wobei die Positionier- und Stabilisierstruktur aufweist:
einen ersten linken Clip (14304), der im linken Plenumkammer-Einlassanschluss (12254) aufgenommen ist,
einen zweiten linken Clip, der in der linken Nut aufgenommen ist,
einen ersten rechten Clip (14304), der im rechten Plenumkammer-Einlassanschluss (12254) aufgenommen ist, und
einen zweiten rechten Clip, der in der rechten Nut aufgenommen ist.

2. Patientenschnittstelle (12000) nach Anspruch 1, wobei der erste linke Clip (14304) und der zweite linke Clip mit der Positionier- und Stabilisierstruktur (12300) unabhängig voneinander verbunden sind und wobei der erste rechte Clip (14304) und der zweite rechte Clip mit der Plenumkammer (12200) unabhängig voneinander verbunden sind.

3. Patientenschnittstelle (12000) nach Anspruch 2, wobei der zweite linke Clip und der zweite rechte Clip dauerhaft mit der Plenumkammer (12200) verbunden sind.

4. Patientenschnittstelle (12000) nach Anspruch 1, wobei der erste linke Clip (14304) und der zweite linke Clip dauerhaft miteinander verbunden sind und wobei der erste rechte Clip (14304) und der zweite rechte Clip dauerhaft miteinander verbunden sind.

5. Patientenschnittstelle (12000) nach einem der Ansprüche 1 bis 4, wobei der zweite linke Clip und der zweite rechte Clip jeweils ein Verbindungsteil (12364) aufweisen, wobei vorzugsweise das Verbindungsteil ein Magnet ist.

6. Patientenschnittstelle (12000) nach einem der Ansprüche 1 bis 5, wobei die Positionier- und Stabilisierstruktur (12300) eine Leitungskopfmontur aufweist, die so konfiguriert ist, dass sie die Luftströmung zur Plenumkammer (12200) fördert.

7. Patientenschnittstelle (12000) nach Anspruch 6, wobei der erste linke Clip (14304) und der erste rechte Clip (14304) mit der Leitungskopfmontur verbunden sind.

8. Patientenschnittstelle (12000) nach einem der Ansprüche 6 bis 7, wobei die Positionier- und Stabilisierstruktur (12300) ferner mindestens einen Riemen (12304) aufweist und wobei die Leitungskopfmontur eine Lasche aufweist, die so konfiguriert ist, dass sie den mindestens einen Riemen entfernbar aufnimmt.

9. Patientenschnittstelle (12000) nach einem der Ansprüche 1 bis 8, wobei eine Abdeckung (12456) dauerhaft mit dem Entlüftungskörper (12404) verbunden ist und mehrere Öffnungen (12436) aufweist und wobei jede Öffnung mit einem der jeweiligen Entlüftungslöcher (12436) kommuniziert, um mehrere Strömungswege bereitzustellen, so dass Luft aus der Plenumkammer (12200) austritt.

10. Patientenschnittstelle (12000) nach Anspruch 9, wobei die mehreren Öffnungen an der Abdeckung (12456) radial nach außen orientiert sind.

11. Patientenschnittstelle (12000) nach Anspruch 9, wobei die mehreren Öffnungen an der Abdeckung (12456) im Wesentlichen in der gleichen Richtung wie die mehreren Entlüftungslöcher (12436) orientiert sind, wobei vorzugsweise Durchmesser der mehreren Entlüftungslöcher größer sind als Durchmesser der mehreren Öffnungen.

12. Patientenschnittstelle (12000) nach einem der Ansprüche 9 bis 11, wobei die mehreren Entlüftungslöcher (12436) in Clustern angeordnet sind, wobei jeder Cluster in einem vorgegebenen Muster angeordnet und von benachbarten Clustern beabstandet ist, wobei vorzugsweise die Cluster vier, fünf oder sechs Entlüftungslöcher aufweisen.

13. Patientenschnittstelle (12000) nach einem der Ansprüche 9 bis 12, wobei der Entlüftungskörper (12404) eine anterior gelegene Oberfläche (12418) und eine posterior gelegene Oberfläche (12420) entgegengesetzt zur anterior gelegenen Oberfläche und in der Plenumkammer (12200) im Gebrauch positioniert aufweist, wobei vorzugsweise die mehreren Entlüftungslöcher (12436) relativ zur posterior gelegenen Oberfläche in der Richtung der anterior gelegenen Oberfläche vertieft sind.

14. Patientenschnittstelle (12000) nach Anspruch 13, wobei die Abdeckung (12456) über die anterior gelegene Oberfläche (12418) hinaus in einer von der posterior gelegenen Oberfläche (12420) wegführenden Richtung vorsteht und wobei die mehreren Öffnungen zwischen der anterior gelegenen Oberfläche und einer Außenfläche der Abdeckung angeordnet sind.

15. Patientenschnittstelle (12000) nach einem der Ansprüche 13 bis 14, wobei eine Nut (12416) zwischen der anterior gelegenen Oberfläche (12418) und der posterior gelegenen Oberfläche (12420) angeordnet ist und wobei die Nut so konfiguriert ist, dass sie einen Abschnitt der Plenumkammer (12200) aufnimmt, der einen Außenumfang der Entlüftungsöffnung (13388) bildet.

## Revendications

1. Interface patient (12000) comprenant:
une chambre de distribution (12200) incluant une cavité qui est pressurisable à une pression thérapeutique d'au moins 6 cmH₂O au-dessus de la pression atmosphérique ambiante, ladite chambre de distribution incluant un orifice d'entrée de chambre de distribution gauche (12254) dimensionné et structuré pour recevoir un flux d'air à la pression thérapeutique pour la respiration par un patient, et un orifice d'entrée de chambre de distribution droit (12254) dimensionné et structuré pour recevoir un flux d'air à la pression thérapeutique pour la respiration par un patient, la chambre de distribution étant construite en un matériau flexible et étant configurée pour être capable de fléchir, la chambre de distribution incluant en outre une rainure gauche (12266) entourant au moins partiellement l'orifice d'entrée de chambre de distribution gauche et une rainure droite entourant au moins partiellement l'orifice d'entrée de chambre de distribution droit;
une structure formant joint d'étanchéité (12100) construite et agencée pour former un joint étanchéité avec une zone du visage du patient entourant une entrée des voies respiratoires du patient de sorte que le flux d'air à ladite pression thérapeutique est délivré aux voies respiratoires du patient, la structure formant joint d'étanchéité étant construite et agencée pour maintenir ladite pression thérapeutique dans la chambre de distribution tout au long du cycle respiratoire du patient pendant l'utilisation;
une structure d'évent (12400) reliée à une ouverture d'évent (13388) dans la chambre de distribution, la structure d'évent configurée pour permettre un flux continu de gaz expirés par le patient depuis un intérieur de la chambre de distribution vers l'environnement, ladite structure d'évent étant dimensionnée et façonnée pour maintenir la pression thérapeutique dans la chambre de distribution pendant l'utilisation, la structure d'évent comprenant:
un corps d'évent (12404) formé d'un matériau rigide et positionné directement à l'intérieur de l'ouverture d'évent, le corps d'évent étant configuré pour diriger le mouvement de flexion dans la chambre de distribution à distance de l'axe central qui intersecte l'ouverture d'évent, le corps d'évent comprenant une surface ayant une pluralité de trous d'évent (12436) configurés pour permettre à l'air de sortir de la chambre de distribution; et
une structure de positionnement et de stabilisation (12300) configurée pour maintenir la structure formant joint d'étanchéité dans une position thérapeutiquement efficace, la structure de positionnement et de stabilisation comprenant:
une première agrafe gauche (14304) reçue dans l'orifice d'entrée de chambre de distribution gauche (12254),
une seconde agrafe gauche reçue dans la rainure gauche,
une première agrafe droite (14304) reçue dans l'orifice d'entrée de chambre de distribution droit (12254), et
une seconde agrafe droite reçue dans la rainure droite.

2. Interface patient (12000) selon la revendication 1, dans laquelle la première agrafe gauche (14304) et la seconde agrafe gauche sont connectées à la structure de positionnement et de stabilisation (12300) indépendamment l'une de l'autre, et dans laquelle la première agrafe droite (14304) et la seconde agrafe droite sont connectées à la chambre de distribution (12200) indépendamment l'une de l'autre.

3. Interface patient (12000) selon la revendication 2, dans laquelle la seconde agrafe gauche et la seconde agrafe droite sont connectées de manière permanente à la chambre de distribution (12200).

4. Interface patient (12000) selon la revendication 1, dans laquelle la première agrafe gauche (14304) et la seconde agrafe gauche sont connectées de manière permanente l'une à l'autre, et dans laquelle la première agrafe droite (14304) et la seconde agrafe droite sont connectées de manière permanente l'une à l'autre.

5. Interface patient (12000) selon l'une quelconque des revendications 1 à 4, dans laquelle la seconde agrafe gauche et la seconde agrafe droite incluent chacune un élément de connexion (12364), de préférence dans laquelle l'élément de connexion est un aimant.

6. Interface patient (12000) selon l'une quelconque des revendications 1 à 5, dans laquelle la structure de positionnement et de stabilisation (12300) inclut un harnais à conduit configuré pour acheminer le flux d'air jusqu'à la chambre de distribution (12200).

7. Interface patient (12000) selon la revendication 6, dans laquelle la première agrafe gauche (14304) et la première agrafe droite (14304) sont reliées au harnais à conduit.

8. Interface patient (12000) selon l'une quelconque des revendications 6 à 7, dans laquelle la structure de positionnement et de stabilisation (12300) comprend en outre au moins une sangle (12304), et dans laquelle le harnais à conduit inclut une languette configurée pour recevoir de manière amovible la au moins une sangle.

9. Interface patient (12000) selon l'une quelconque des revendications 1 à 8, dans laquelle un couvercle (12456) est connecté de manière permanente au corps d'évent (12404) et inclut une pluralité d'ouvertures (12436), et dans laquelle chaque ouverture est en communication avec l'un des trous d'évent (12436) respectifs afin de fournir une pluralité de voies d'écoulement pour que l'air sorte de la chambre de distribution (12200).

10. Interface patient (12000) selon la revendication 9, dans laquelle la pluralité d'ouvertures sur le couvercle (12456) sont orientées radialement vers l'extérieur.

11. Interface patient (12000) selon la revendication 9, dans laquelle la pluralité d'ouvertures sur le couvercle (12456) sont orientées sensiblement dans la même direction que la pluralité de trous d'évent (12436), de préférence dans laquelle le diamètre de la pluralité de trous d'évent est supérieur au diamètre de la pluralité d'ouvertures.

12. Interface patient (12000) selon l'une quelconque des revendications 9 à 11, dans laquelle la pluralité de trous d'évent (12436) sont disposées en groupes, dans laquelle chaque groupe est disposé selon un motif donné et est espacé des groupes adjacents, de préférence dans laquelle les groupes incluent quatre, cinq ou six trous d'évent.

13. Interface patient (12000) selon l'une quelconque des revendications 9 à 12, dans laquelle le corps d'évent (12404) inclut une surface antérieure (12418) et une surface postérieure (12420) opposée à la surface antérieure et positionnée à l'intérieur de la chambre de distribution (12200) pendant l'utilisation, de préférence dans laquelle la pluralité de trous d'évent (12436) sont en retrait par rapport à la surface postérieure dans la direction de la surface antérieure.

14. Interface patient (12000) selon la revendication 13, dans laquelle le couvercle (12456) fait saillie au-delà de la surface antérieure (12418) dans une direction à distance de la surface postérieure (12420), et dans laquelle la pluralité d'ouvertures sont disposées entre la surface antérieure et une surface extérieure du couvercle.

15. Interface patient (12000) selon l'une quelconque des revendications 13 à 14, dans laquelle une rainure (12416) est disposée entre la surface antérieure (12418) et la surface postérieure (12420), et dans laquelle la rainure est configurée pour recevoir une partie de la chambre de distribution (12200) formant un périmètre extérieur de l'ouverture d'évent (13388)
